# EUROPEAN PATENT SPECIFICATION

(11) **EP 1 778 284 B1**
(45) Date of publication and mention of the grant of the patent: **25.04.2012**
(21) Application number: 04816811.6
(22) Date of filing: 16.08.2004
(51) Int. Cl.: C07K 14/47, C07K 16/30, G01N 33/574, C07K 16/28

(54) **NUCLEIC ACIDS AND CORRESPONDING PROTEINS ENTITLED 58P1D12 USEFUL IN DETECTION OF CANCER**
NUKLEINSÄUREN UND ENTSPRECHENDE PROTEINE 58P1D12 ZUM NACHWEIS VON KREBS
ACIDES NUCLEIQUES ET PROTEINES CORRESPONDANTES APPELES 58P1D12 UTILES DANS LA DETECTION DU CANCER

(43) Date of publication of application: 02.05.2007
(73) Proprietor: Agensys, Inc., Santa Monica CA 90404 (US)
(72) Inventor: KANNER, Steven, B., Santa Monica, CA 90403 (US); JAKOBOVITS, Aya, Beverly Hills, CA 90210 (US); CHALLITA-EID, Pia, M., Encino, CA 91436 (US); GE, Wangmao, Los Angeles, CA 90034 (US); RAITANO, Arthur, B., Los Angeles, CA 90066 (US)
(74) Representative: Lord, Hilton David
(86) International application number: PCT/US2004/026829
(87) International publication number: WO 2006/022722

(56) References cited:
- JP-A- 2004 187 620
- US-A1- 2003 077 710
- US-A1- 2003 180 837
- US-B1- 6 602 501
- WENG L. ET AL.: "A novel alternative spliced chondrolectin isoform lacking the transmembrane domain is expressed during T cell maturation" THE JOURNAL OF BIOLOGICAL CHEMISTRY, vol. 278, no. 21, 2003, pages 19164-19170, XP002485630

### SEQUENCE LISTING

<110> Agensys, Inc.
   Kanner, Steven B.
   Jakobovits, Aya
   Challit-Eid, Pia M.
   Wangmao, Ge
   Raitano, Arthur B.
<120> Nucleic Acids and corresponding Proteins Entitled 58P1012 Useful in Treatment and Detection of Cancer
<130> 511582002047
<140> Not Yet Assigned
   <141> Filed Herewith
<160> 83
<170> FastSEQ for Windows version 4.0
<210> 1
   <211> 427
   <212> DNA
   <213> Homo sapiens
<400> 1
   cagaattaca aaattctgga accaagtcaa tgagttatta tacttccatg ccactttctt 60
   ttctggtact ctttgaaatc cacagtgtag actggtttgg actagttttt gttcttcctt 120
   tacttttatg cagcatctgg aaacaacagg ttccaaaagc aaccagtatc agtaagagca 180
   ggggtattgt tggtataaca acataaatta gattgggaat tatacctgct tcagtaacaa 240
   ccacattctg atgggtgtct cctggttgat ttgtaagata aggcttttct acaggggctg 300
   ttggattaat ctctggttca tacttgcaaa tataattgtg cttcatgtta cacctgtcat 360
   cattccactg gtaaaggtag ggacccccaa ggccaggatt ggcagttggt tggtgatcat 420
   cacaaca                                                           427
<210> 2
   <211> 2550
   <212> DNA
   <213> Homo sapiens
<220>
   <221> CDS
   <222> (380)...(1201)
<400> 2
   atccaggacc agggcgcacc ggctcagcct ctcacttgtc agaggccggg gaagagaagc 60
   aaagcgcaac ggtgtggtcc aagccggggc ttctgcttcg cctctaggac atacacggga 120
   ccccctaact tcagtccccc aaacgcgcac cctcgaagtc ttgaactcca gccccgcaca 180
   tccacgcgcg gcacaggcgc ggcaggcggc aggtcccggc cgaaggcgat gcgcgcaggg 240
   ggtcgggcag ctgggctcgg gcggcgggag tagggcccgg cagggaggca gggaggctgc 300
   agagtcagag tcgcgggctg cgccctgggc agaggccgcc ctcgctccac gcaacacctg 360
   ctgctgccac cgcgccgcg atg agc cgc gtg gtc tcg ctg ctg ctg ggc gcc  412
                        Met Ser Arg Val Val Ser Leu Leu Leu Gly Ala
                         1               5                  10
   gcg ctg ctc tgc ggc cac gga gcc ttc tgc cgc cgc gtg gtc agc ggc   460
   Ala Leu Leu Cys Gly His Gly Ala Phe Cys Arg Arg Val Val Ser Gly
               15                  20                  25
   caa aag gtg tgt ttt gct gac ttc aag cat ccc tgc tac aaa atg gcc   508
   Gln Lys Val Cys Phe Ala Asp Phe Lys His Pro Cys Tyr Lys Met Ala
           30                  35                  40
   tac ttc cat gaa ctg tcc agc cga gtg agc ttt cag gag gca cgc ctg   556
   Tyr Phe His Glu Leu Ser Ser Arg Val Ser Phe Gln Glu Ala Arg Leu
       45                  50                  55
   gct tgt gag agt gag gga gga gtc ctc ctc agc ctt gag aat gaa gca      604
   Ala Cys Glu Ser Glu Gly Gly Val Leu Leu Ser Leu Glu Asn Glu Ala
    60                 65                  70                  75
   gaa cag aag tta ata gag agc atg ttg caa aac ctg aca aaa ccc ggg      652
   Glu Gln Lys Leu Ile Glu Ser Met Leu Gln Asn Leu Thr Lys Pro Gly
                   80                  85                  90
   aca ggg att tct gat ggt gat ttc tgg ata ggg ctt tgg agg aat gga      700
   Thr Gly Ile Ser Asp Gly Asp Phe Trp Ile Gly Leu Trp Arg Asn Gly
               95                  100                 105
   gat ggg caa aca tct ggt gcc tgc cca gat ctc tac cag tgg tct gat      748
   Asp Gly Gln Thr Ser Gly Ala Cys Pro Asp Leu Tyr Gln Trp Ser Asp
           110                 115                 120
   gga agc aat tcc cag tac cga aac tgg tac aca gat gaa cct tcc tgc      796
   Gly Ser Asn Ser Gln Tyr Arg Asn Trp Tyr Thr Asp Glu Pro Ser Cys
       125                 130                 135
   gga agt gaa aag tgt gtt gtg atg tat cac caa cca act gcc aat cct      844
   Gly Ser Glu Lys Cys Val Val Met Tyr His Gln Pro Thr Ala Asn Pro
   140                 145                 150                 155
   ggc ctt ggg ggt ccc tac ctt tac cag tgg aat gat gac agg tgt aac      892
   Gly Leu Gly Gly Pro Tyr Leu Tyr Gln Trp Asn Asp Asp Arg Cys Asn
                   160                 165                 170
   atg aag cac aat tat att tgc aag tat gaa cca gag att aat cca aca      940
   Met Lys His Asn Tyr Ile Cys Lys Tyr Glu Pro Glu Ile Asn Pro Thr
               175                 180                 185
   gcc cct gta gaa aag cct tat ctt aca aat caa cca gga gac acc cat      988
   Ala Pro Val Glu Lys Pro Tyr Leu Thr Asn Gln Pro Gly Asp Thr His
           190                 195                 200
   cag aat gtg gtt gtt act gaa gca ggt ata att ccc aat cta att tat     1036
   Gln Asn Val Val Val Thr Glu Ala Gly Ile Ile Pro Asn Leu Ile Tyr
       205                 210                 215
   gtt gtt ata cca aca ata ccc ctg ctc tta ctg ata ctg gtt gct ttt     1084
   Val Val Ile Pro Thr Ile Pro Leu Leu Leu Leu Ile Leu Val Ala Phe
   220                 225                 230                 235
   gga acc tgt tgt ttc cag atg ctg cat aaa agt aaa gga aga aca aaa     1132
   Gly Thr Cys Cys Phe Gln Met Leu His Lys Ser Lys Gly Arg Thr Lys
                   240                 245                 250
   act agt cca aac cag tct aca ctg tgg att tca aag agt acc aga aaa     1180
   Thr Ser Pro Asn Gln Ser Thr Leu Trp Ile Ser Lys Ser Thr Arg Lys
               255                 260                 265
   gaa agt ggc atg gaa gta taa taactcattg acttggttcc agaattttgt        1231
   Glu Ser Gly Met Glu Val  *
           270
   aattctggat ctgtataagg aatggcatca gaacaatagc ttggaatggc ttgaaatcac 1291
   aaaggatctg caagatgaac tgtaagctcc cccttgaggc aaatattaaa gtaattttta 1351
   tatgtctatt atttcattta aagaatatgc tgtgctaata atggagtgag acatgcttat 1411
   tttgctaaag gatgcaccca aacttcaaac ttcaagcaaa tgaaatggac aatgcagata 1471
   aagttgttat caacacgtcg ggagtatgtg tgttagaagc aattcctttt atttctttca 1531
   cctttcataa gttgttatct agtcaatgta atgtatattg tattgaaatt tacagtgtgc 1591
   aaaagtattt tacctttgca taagtgtttg ataaaaatga actgttctaa tatttatttt 1651
   tatggcatct catttttcaa tacatgctct tttgattaaa gaaacttatt actgttgtca 1711
   actgaattca cacacacaca aatatagtac catagaaaaa gtttgttttc tcgaaataat 1771
   tcatctttca gcttctctgc ttttggtcaa tgtctaggaa atctcttcag aaataagaag 1831
   ctatttcatt aagtgtgata taaacctcct caaacatttt acttagaggc aaggattgtc 1891
   taatttcaat tgtgcaagac atgtgcctta taattatttt tagcttaaaa ttaaacagat 1951
   tttgtaataa tgtaactttg ttaataggtg cataaacact aatgcagtca atttgaacaa 2011
   aagaagtgac atacacaata taaatcatat gtcttcacac gttgcctata taatgagaag 2071
   cagctctctg agggttctga aatcaatgtg gtccctctct tgcccactaa acaaagatgg 2131
   ttgttcgggg tttgggattg acactggagg cagatagttg caaagttagt ctaaggtttc 2191
   cctagctgta tttagcctct gactatatta gtatacaaag aggtcatgtg gttgagacca 2251
   ggtgaatagt cactatcagt gtggagacaa gcacagcaca cagacatttt aggaaggaaa 2311
   ggaactacga aatcgtgtga aaatgggttg gaacccatca gtgatcgcat attcattgat 2371
   gagggtttgc ttgagataga aaatggtggc tcctttctgt cttatctcct agtttcttca 2431
   atgcttacgc cttgttcttc tcaagagaaa gttgtaactc tctggtcttc atatgtccct 2491
   gtgctccttt taaccaaata aagagttctt gtttctgaag aaaaaaaaaa aaaaaaaaa  2550
<210> 3
   <211> 273
   <212> PRT
   <213> Homo sapiens
<400> 3
   Met Ser Arg Val Val Ser Leu Leu Leu Gly Ala Ala Leu Leu Cys Gly
   1               5                   10                  15
   His Gly Ala Phe Cys Arg Arg Val Val Ser Gly Gln Lys Val Cys Phe
               20                  25                  30
   Ala Asp Phe Lys His Pro Cys Tyr Lys Met Ala Tyr Phe His Glu Leu
           35                  40                  45
   Ser Ser Arg Val Ser Phe Gln Glu Ala Arg Leu Ala Cys Glu Ser Glu
       50                  55                  60
   Gly Gly Val Leu Leu Ser Leu Glu Asn Glu Ala Glu Gln Lys Leu Ile
   65                  70                  75                  80
   Glu Ser Met Leu Gln Asn Leu Thr Lys Pro Gly Thr Gly Ile Ser Asp
                   85                  90                  95
   Gly Asp Phe Trp Ile Gly Leu Trp Arg Asn Gly Asp Gly Gln Thr Ser
               100                 105                 110
   Gly Ala Cys Pro Asp Leu Tyr Gln Trp Ser Asp Gly Ser Asn Ser Gln
           115                 120                 125
   Tyr Arg Asn Trp Tyr Thr Asp Glu Pro Ser Cys Gly Ser Glu Lys Cys
       130                 135                 140
   Val Val Met Tyr His Gln Pro Thr Ala Asn Pro Gly Leu Gly Gly Pro
   145                 150                 155                 160
   Tyr Leu Tyr Gln Trp Asn Asp Asp Arg Cys Asn Met Lys His Asn Tyr
                   165                 170                 175
   Ile Cys Lys Tyr Glu Pro Glu Ile Asn Pro Thr Ala Pro Val Glu Lys
               180                 185                 190
   Pro Tyr Leu Thr Asn Gln Pro Gly Asp Thr His Gln Asn Val Val Val
           195                 200                 205
   Thr Glu Ala Gly Ile Ile Pro Asn Leu Ile Tyr Val Val Ile Pro Thr
       210                 215                 220
   Ile Pro Leu Leu Leu Leu Ile Leu Val Ala Phe Gly Thr Cys Cys Phe
   225                 230                 235                 240
   Gln Met Leu His Lys Ser Lys Gly Arg Thr Lys Thr Ser Pro Asn Gln
                   245                 250                 255
   Ser Thr Leu Trp Ile Ser Lys Ser Thr Arg Lys Glu Ser Gly Met Glu
               260                 265                 270
   Val
<210> 4
   <211> 2418
   <212> DNA
   <213> Homo sapiens
<220>
   <221> CDS
   <222> (388)...(1086)
<400> 4
   ctgtggtgtt tttcccccgc tcctctggct gccttcctga tggatctctg tggtcccagg 60
   caggaatggc ctgcttgggg acccagcgag ctcccaaggc ctttcctgct gcttcctcta 120
   tccctgtgtt ttgcttggct ctctaaattg actcagctcc aggacatcag gaccccaggt 180
   tctctggtct tgggactctg agacttgcac caggaatcct gcccaggctc tcaggccttt 240
   ggactcagac tgagctactt cactggcttt cctggttctc cagcttgaag atggcagatc 300
   gtgggacttc tcagcctcca taattgagtg agccaattcc ctggccaaaa ggtgtgtttt 360
   gctgacttca agcatccctg ctacaaa atg gcc tac ttc cat gaa ctg tcc agc 414
                                 Met Ala Tyr Phe His Glu Leu Ser Ser
                                 1               5
   cga gtg agc ttt cag gag gca cgc ctg gct tgt gag agt gag gga gga    462
   Arg Val Ser Phe Gln Glu Ala Arg Leu Ala Cys Glu Ser Glu Gly Gly
   10                  15                  20                  25
   gtc ctc ctc agc ctt gag aat gaa gca gaa cag aag tta ata gag agc    510
   Val Leu Leu Ser Leu Glu Asn Glu Ala Glu Gln Lys Leu Ile Glu Ser
                   30                  35                  40
   atg ttg caa aac ctg aca aaa ccc ggg aca ggg att tct gat ggt gat    558
   Met Leu Gln Asn Leu Thr Lys Pro Gly Thr Gly Ile Ser Asp Gly Asp
               45                  50                  55
   ttc tgg ata ggg ctt tgg agg aat gga gat ggg caa aca tct ggt gcc    606
   Phe Trp Ile Gly Leu Trp Arg Asn Gly Asp Gly Gln Thr Ser Gly Ala
           60                  65                  70
   tgc cca gat ctc tac cag tgg tct gat gga agc aat tcc cag tac cga    654
   Cys Pro Asp Leu Tyr Gln Trp Ser Asp Gly Ser Asn Ser Gln Tyr Arg
       75                  80                  85
   aac tgg tac aca gat gaa cct tcc tgc gga agt gaa aag tgt gtt gtg    702
   Asn Trp Tyr Thr Asp Glu Pro Ser Cys Gly Ser Glu Lys Cys Val Val
   90                  95                  100                 105
   atg tat cac caa cca act gcc aat cct ggc ctt ggg ggt ccc tac ctt    750
   Met Tyr His Gln Pro Thr Ala Asn Pro Gly Leu Gly Gly Pro Tyr Leu
                   110                 115                 120
   tac cag tgg aat gat gac agg tgt aac atg aag cac aat tat att tgc    798
   Tyr Gln Trp Asn Asp Asp Arg Cys Asn Met Lys His Asn Tyr Ile Cys
               125                 130                 135
   aag tat gaa cca gag att aat cca aca gcc cct gta gaa aag cct tat    846
   Lys Tyr Glu Pro Glu Ile Asn Pro Thr Ala Pro Val Glu Lys Pro Tyr
           140                 145                 150
   ctt aca aat caa cca gga gac acc cat cag aat gtg gtt gtt act gaa    894
   Leu Thr Asn Gln Pro Gly Asp Thr His Gln Asn Val Val Val Thr Glu
       155                 160                 165
   gca ggt ata att ccc aat cta att tat gtt gtt ata cca aca ata ccc    942
   Ala Gly Ile Ile Pro Asn Leu Ile Tyr Val Val Ile Pro Thr Ile Pro
   170                 175                 180                 185
   ctg ctc tta ctg ata ctg gtt gct ttt gga acc tgt tgt ttc cag atg    990
   Leu Leu Leu Leu Ile Leu Val Ala Phe Gly Thr Cys Cys Phe Gln Met
                   190                 195                 200
   ctg cat aaa agt aaa gga aga aca aaa act agt cca aac cag tct aca    1038
   Leu His Lys Ser Lys Gly Arg Thr Lys Thr Ser Pro Asn Gln Ser Thr
               205                 210                 215
   ctg tgg att tca aag agt acc aga aaa gaa agt ggc atg gaa gta taa    1086
   Leu Trp Ile Ser Lys Ser Thr Arg Lys Glu Ser Gly Met Glu Val  *
           220                 225                 230
   taactcattg acttggttcc agaattttgt aattctggat ctgtataagg aatggcatca 1146
   gaacaatagc ttggaatggc ttgaaatcac aaaggatctg caagatgaac tgtaagctcc 1206
   cccttgaggc aaatattaaa gtaattttta tatgtctatt atttcattta aagaatatgc 1266
   tgtgctaata atggagtgag acatgcttat tttgctaaag gatgcaccca aacttcaaac 1326
   ttcaagcaaa tgaaatggac aatgcagata aagttgttat caacacgtcg ggagtatgtg 1386
   tgttagaagc aattcctttt atttctttca cctttcataa gttgttatct agtcaatgta 1446
   atgtatattg tattgaaatt tacagtgtgc aaaagtattt tacctttgca taagtgtttg 1506
   ataaaaatga actgttctaa tatttatttt tatggcatct catttttcaa tacatgctct 1566
   tttgattaaa gaaacttatt actgttgtca actgaattca cacacacaca aatatagtac 1626
   catagaaaaa gtttgttttc tcgaaataat tcatctttca gcttctctgc ttttggtcaa 1686
   tgtctaggaa atctcttcag aaataagaag ctatttcatt aagtgtgata taaacctcct 1746
   caaacatttt acttagaggc aaggattgtc taatttcaat tgtgcaagac atgtgcctta 1806
   taattatttt tagcttaaaa ttaaacagat tttgtaataa tgtaactttg ttaataggtg 1866
   cataaacact aatgcagtca atttgaacaa aagaagtgac atacacaata taaatcatat 1926
   gtcttcacac gttgcctata taatgagaag cagctctctg agggttctga aatcaatgtg 1986
   gtccctctct tgcccactaa acaaagatgg ttgttcgggg tttgggattg acactggagg 2046
   cagatagttg caaagttagt ctaaggtttc cctagctgta tttagcctct gactatatta 2106
   gtatacaaag aggtcatgtg gttgagacca ggtgaatagt cactatcagt gtggagacaa 2166
   gcacagcaca cagacatttt aggaaggaaa ggaactacga aatcgtgtga aaatgggttg 2226
   gaacccatca gtgatcgcat attcattgat gagggtttgc ttgagataga aaatggtggc 2286
   tcctttctgt cttatctcct agtttcttca atgcttacgc cttgttcttc tcaagagaaa 2346
   gttgtaactc tctggtcttc atatgtccct gtgctccttt taaccaaata aagagttctt 2406
   gtttctgaag aa                                                     2418
<210> 5
   <211> 232
   <212> PRT
   <213> Homo sapiens
<400> 5
   Met Ala Tyr Phe His Glu Leu Ser Ser Arg Val Ser Phe Gln Glu Ala
   1               5                   10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala Gly Ile Ile Pro Asn Leu
                   165                 170                 175
   Ile Tyr Val Val Ile Pro Thr Ile Pro Leu Leu Leu Leu Ile Leu Val
               180                 185                 190
   Ala Phe Gly Thr Cys Cys Phe Gln Met Leu His Lys Ser Lys Gly Arg
           195                 200                 205
   Thr Lys Thr Ser Pro Asn Gln Ser Thr Leu Trp Ile Ser Lys Ser Thr
       210                 215                 220
   Arg Lys Glu Ser Gly Met Glu Val
   225                 230
<210> 6
   <211> 2236
   <212> DNA
   <213> Homo sapiens
<220>
   <221> CDS
   <222> (206)...(904)
<400> 6
   gcttgaagat ggcagatcgt gggacttctc agcctccata attgagtgag ccaattccct 60
   gaatacaaca agaagatggc catctgtaga ccaggaggtg gtccctcccc agaaactgga 120
   tgggccagca cgttgattct gagcttctag cctccagaac tgccaaaagg tgtgttttgc 180
   tgacttcaag catccctgct acaaa atg gcc tac ttc cat gaa ctg tcc agc   232
                               Met Ala Tyr Phe His Glu Leu Ser Ser
                               1               5
   cga gtg agc ttt cag gag gca cgc ctg gct tgt gag agt gag gga gga   280
   Arg Val Ser Phe Gln Glu Ala Arg Leu Ala Cys Glu Ser Glu Gly Gly
   10                  15                  20                  25
   gtc ctc ctc agc ctt gag aat gaa gca gaa cag aag tta ata gag agc   328
   Val Leu Leu Ser Leu Glu Asn Glu Ala Glu Gln Lys Leu Ile Glu Ser
                   30                  35                  40
   atg ttg caa aac ctg aca aaa ccc ggg aca ggg att tct gat ggt gat   376
   Met Leu Gln Asn Leu Thr Lys Pro Gly Thr Gly Ile Ser Asp Gly Asp
               45                  50                  55
   ttc tgg ata ggg ctt tgg agg aat gga gat ggg caa aca tct ggt gcc   424
   Phe Trp Ile Gly Leu Trp Arg Asn Gly Asp Gly Gln Thr Ser Gly Ala
           60                  65                  70
   tgc cca gat ctc tac cag tgg tct gat gga agc aat tcc cag tac cga   472
   Cys Pro Asp Leu Tyr Gln Trp Ser Asp Gly Ser Asn Ser Gln Tyr Arg
       75                  80                  85
   aac tgg tac aca gat gaa cct tcc tgc gga agt gaa aag tgt gtt gtg   520
   Asn Trp Tyr Thr Asp Glu Pro Ser Cys Gly Ser Glu Lys Cys Val Val
   90                  95                  100                 105
   atg tat cac caa cca act gcc aat cct ggc ctt ggg ggt ccc tac ctt   568
   Met Tyr His Gln Pro Thr Ala Asn Pro Gly Leu Gly Gly Pro Tyr Leu
                   110                 115                 120
   tac cag tgg aat gat gac agg tgt aac atg aag cac aat tat att tgc   616
   Tyr Gln Trp Asn Asp Asp Arg Cys Asn Met Lys His Asn Tyr Ile Cys
               125                 130                 135
   aag tat gaa cca gag att aat cca aca gcc cct gta gaa aag cct tat   664
   Lys Tyr Glu Pro Glu Ile Asn Pro Thr Ala Pro Val Glu Lys Pro Tyr
           140                 145                 150
   ctt aca aat caa cca gga gac acc cat cag aat gtg gtt gtt act gaa   712
   Leu Thr Asn Gln Pro Gly Asp Thr His Gln Asn Val Val Val Thr Glu
       155                 160                 165
   gca ggt ata att ccc aat cta att tat gtt gtt ata cca aca ata ccc   760
   Ala Gly Ile Ile Pro Asn Leu Ile Tyr Val Val Ile Pro Thr Ile Pro
   170                 175                 180                 185
   ctg ctc tta ctg ata ctg gtt gct ttt gga acc tgt tgt ttc cag atg   808
   Leu Leu Leu Leu Ile Leu Val Ala Phe Gly Thr Cys Cys Phe Gln Met
                   190                 195                 200
   ctg cat aaa agt aaa gga aga aca aaa act agt cca aac cag tct aca   856
   Leu His Lys Ser Lys Gly Arg Thr Lys Thr Ser Pro Asn Gln Ser Thr
               205                 210                 215
   ctg tgg att tca aag agt acc aga aaa gaa agt ggc atg gaa gta taa   904
   Leu Trp Ile Ser Lys Ser Thr Arg Lys Glu Ser Gly Met Glu Val  *
           220                 225                 230
   taactcattg acttggttcc agaattttgt aattctggat ctgtataagg aatggcatca 964
   gaacaatagc ttggaatggc ttgaaatcac aaaggatctg caagatgaac tgtaagctcc 1024
   cccttgaggc aaatattaaa gtaattttta tatgtctatt atttcattta aagaatatgc 1084
   tgtgctaata atggagtgag acatgcttat tttgctaaag gatgcaccca aacttcaaac 1144
   ttcaagcaaa tgaaatggac aatgcagata aagttgttat caacacgtcg ggagtatgtg 1204
   tgttagaagc aattcctttt atttctttca cctttcataa gttgttatct agtcaatgta 1264
   atgtatattg tattgaaatt tacagtgtgc aaaagtattt tacctttgca taagtgtttg 1324
   ataaaaatga actgttctaa tatttatttt tatggcatct catttttcaa tacatgctct 1384
   tttgattaaa gaaacttatt actgttgtca actgaattca cacacacaca aatatagtac 1444
   catagaaaaa gtttgttttc tcgaaataat tcatctttca gcttctctgc ttttggtcaa 1504
   tgtctaggaa atctcttcag aaataagaag ctatttcatt aagtgtgata taaacctcct 1564
   caaacatttt acttagaggc aaggattgtc taatttcaat tgtgcaagac atgtgcctta 1624
   taattatttt tagcttaaaa ttaaacagat tttgtaataa tgtaactttg ttaataggtg 1684
   cataaacact aatgcagtca atttgaacaa aagaagtgac atacacaata taaatcatat 1744
   gtcttcacac gttgcctata taatgagaag cagctctctg agggttctga aatcaatgtg 1804
   gtccctctct tgcccactaa acaaagatgg ttgttcgggg tttgggattg acactggagg 1864
   cagatagttg caaagttagt ctaaggtttc cctagctgta tttagcctct gactatatta 1924
   gtatacaaag aggtcatgtg gttgagacca ggtgaatagt cactatcagt gtggagacaa 1984
   gcacagcaca cagacatttt aggaaggaaa ggaactacga aatcgtgtga aaatgggttg 2044
   gaacccatca gtgatcgcat attcattgat gagggtttgc ttgagataga aaatggtggc 2104
   tcctttctgt cttatctcct agtttcttca atgcttacgc cttgttcttc tcaagagaaa 2164
   gttgtaactc tctggtcttc atatgtccct gtgctccttt taaccaaata aagagttctt 2224
   gtttctgaag aa                                                     2236
<210> 7
   <211> 232
   <212> PRT
   <213> Homo sapiens
<400> 7
   Met Ala Tyr Phe His Glu Leu Ser Ser Arg Val Ser Phe Gln Glu Ala
    1               5                  10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala Gly Ile Ile Pro Asn Leu
                   165                 170                 175
   Ile Tyr Val Val Ile Pro Thr Ile Pro Leu Leu Leu Leu Ile Leu Val
               180                 185                 190
   Ala Phe Gly Thr Cys Cys Phe Gln Met Leu His Lys Ser Lys Gly Arg
           195                 200                 205
   Thr Lys Thr Ser Pro Asn Gln Ser Thr Leu Trp Ile Ser Lys Ser Thr
       210                 215                 220
   Arg Lys Glu Ser Gly Met Glu Val
   225                 230
<210> 8
   <211> 2133
   <212> DNA
   <213> Homo sapiens
<220>
   <221> CDS
   <222> (206)...(916)
<400> 8
   gcttgaagat ggcagatcgt gggacttctc agcctccata attgagtgag ccaattccct 60
   gaatacaaca agaagatggc catctgtaga ccaggaggtg gtccctcccc agaaactgga 120
   tgggccagca cgttgattct gagcttctag cctccagaac tgccaaaagg tgtgttttgc 180
   tgacttcaag catccctgct acaaa atg gcc tac ttc cat gaa ctg tcc agc   232
                           Met Ala Tyr Phe His Glu Leu Ser Ser
                            1               5
   cga gtg agc ttt cag gag gca cgc ctg gct tgt gag agt gag gga gga     280
   Arg Val Ser Phe Gln Glu Ala Arg Leu Ala Cys Glu Ser Glu Gly Gly
    10                 15                  20                  25
   gtc ctc ctc agc ctt gag aat gaa gca gaa cag aag tta ata gag agc     328
   Val Leu Leu Ser Leu Glu Asn Glu Ala Glu Gln Lys Leu Ile Glu Ser
                   30                  35                  40
   atg ttg caa aac ctg aca aaa ccc ggg aca ggg att tct gat ggt gat     376
   Met Leu Gln Asn Leu Thr Lys Pro Gly Thr Gly Ile Ser Asp Gly Asp
               45                  50                  55
   ttc tgg ata ggg ctt tgg agg aat gga gat ggg caa aca tct ggt gcc     424
   Phe Trp Ile Gly Leu Trp Arg Asn Gly Asp Gly Gln Thr Ser Gly Ala
           60                  65                  70
   tgc cca gat ctc tac cag tgg tct gat gga agc aat tcc cag tac cga     472
   Cys Pro Asp Leu Tyr Gln Trp Ser Asp Gly Ser Asn Ser Gln Tyr Arg
       75                  80                  85
   aac tgg tac aca gat gaa cct tcc tgc gga agt gaa aag tgt gtt gtg     520
   Asn Trp Tyr Thr Asp Glu Pro Ser Cys Gly Ser Glu Lys Cys Val Val
    90                 95                 100                 105
   atg tat cac caa cca act gcc aat cct ggc ctt ggg ggt ccc tac ctt     568
   Met Tyr His Gln Pro Thr Ala Asn Pro Gly Leu Gly Gly Pro Tyr Leu
                   110                 115                 120
   tac cag tgg aat gat gac agg tgt aac atg aag cac aat tat att tgc     616
   Tyr Gln Trp Asn Asp Asp Arg Cys Asn Met Lys His Asn Tyr Ile Cys
               125                 130                 135
   aag tat gaa cca gag att aat cca aca gcc cct gta gaa aag cct tat     664
   Lys Tyr Glu Pro Glu Ile Asn Pro Thr Ala Pro Val Glu Lys Pro Tyr
           140                 145                 150
   ctt aca aat caa cca gga gac acc cat cag aat gtg gtt gtt act gaa     712
   Leu Thr Asn Gln Pro Gly Asp Thr His Gln Asn Val Val Val Thr Glu
       155                 160                 165
   gca gta aag gaa gaa caa aaa cta gtc caa acc agt cta cac tgt gga     760
   Ala Val Lys Glu Glu Gln Lys Leu Val Gln Thr Ser Leu His Cys Gly
   170                 175                 180                 185
   ttt caa aga gta cca gaa aag aaa gtg gca tgg aag tat aat aac tca     808
   Phe Gln Arg Val Pro Glu Lys Lys Val Ala Trp Lys Tyr Asn Asn Ser
                   190                 195                 200
   ttg act tgg ttc cag aat ttt gta att ctg gat ctg tat aag gaa tgg     856
   Leu Thr Trp Phe Gln Asn Phe Val Ile Leu Asp Leu Tyr Lys Glu Trp
               205                 210                 215
   cat cag aac aat agc ttg gaa tgg ctt gaa atc aca aag gat ctg caa     904
   His Gln Asn Asn Ser Leu Glu Trp Leu Glu Ile Thr Lys Asp Leu Gln
           220                 225                 230
   gat gaa ctg taa gctccccctt gaggcaaata ttaaagtaat ttttatatgt         956
   Asp Glu Leu  *
       235
   ctattatttc atttaaagaa tatgctgtgc taataatgga gtgagacatg cttattttgc 1016
   taaaggatgc acccaaactt caaacttcaa gcaaatgaaa tggacaatgc agataaagtt 1076
   gttatcaaca cgtcgggagt atgtgtgtta gaagcaattc cttttatttc tttcaccttt 1136
   cataagttgt tatctagtca atgtaatgta tattgtattg aaatttacag tgtgcaaaag 1196
   tattttacct ttgcataagt gtttgataaa aatgaactgt tctaatattt atttttatgg 1256
   catctcattt ttcaatacat gctcttttga ttaaagaaac ttattactgt tgtcaactga 1316
   attcacacac acacaaatat agtaccatag aaaaagtttg ttttctcgaa ataattcatc 1376
   tttcagcttc tctgcttttg gtcaatgtct aggaaatctc ttcagaaata agaagctatt 1436
   tcattaagtg tgatataaac ctcctcaaac attttactta gaggcaagga ttgtctaatt 1496
   tcaattgtgc aagacatgtg ccttataatt atttttagct taaaattaaa cagattttgt 1556
   aataatgtaa ctttgttaat aggtgcataa acactaatgc agtcaatttg aacaaaagaa 1616
   gtgacataca caatataaat catatgtctt cacacgttgc ctatataatg agaagcagct 1676
   ctctgagggt tctgaaatca atgtggtccc tctcttgccc actaaacaaa gatggttgtt 1736
   cggggtttgg gattgacact ggaggcagat agttgcaaag ttagtctaag gtttccctag 1796
   ctgtatttag cctctgacta tattagtata caaagaggtc atgtggttga gaccaggtga 1856
   atagtcacta tcagtgtgga gacaagcaca gcacacagac attttaggaa ggaaaggaac 1916
   tacgaaatcg tgtgaaaatg ggttggaacc catcagtgat cgcatattca ttgatgaggg 1976
   tttgcttgag atagaaaatg gtggctcctt tctgtcttat ctcctagttt cttcaatgct 2036
   tacgccttgt tcttctcaag agaaagttgt aactctctgg tcttcatatg tccctgtgct 2096
   ccttttaacc aaataaagag ttcttgtttc tgaagaa 2133
<210> 9
   <211> 236
   <212> PRT
   <213> Homo sapiens
<400> 9
   Met Ala Tyr Phe His Glu Leu Ser Ser Arg Val Ser Phe Gln Glu Ala
   1 5 10 15
   Arg Leu Ala Cys Glu Ser Glu Gly Gly Val Leu Leu Ser Leu Glu Asn
   20 25 30
   Glu Ala Glu Gln Lys Leu Ile Glu Ser Met Leu Gln Asn Leu Thr Lys
   35 40 45
   Pro Gly Thr Gly Ile Ser Asp Gly Asp Phe Trp Ile Gly Leu Trp Arg
   50 55 60
   Asn Gly Asp Gly Gln Thr Ser Gly Ala Cys Pro Asp Leu Tyr Gln Trp
   65 70 75 80
   Ser Asp Gly Ser Asn Ser Gln Tyr Arg Asn Trp Tyr Thr Asp Glu Pro
   85 90 95
   Ser Cys Gly Ser Glu Lys Cys Val Val Met Tyr His Gln Pro Thr Ala
   100 105 110
   Asn Pro Gly Leu Gly Gly Pro Tyr Leu Tyr Gln Trp Asn Asp Asp Arg
   115 120 125
   Cys Asn Met Lys His Asn Tyr Ile Cys Lys Tyr Glu Pro Glu Ile Asn
   130 135 140
   Pro Thr Ala Pro Val Glu Lys Pro Tyr Leu Thr Asn Gln Pro Gly Asp
   145 150 155 160
   Thr His Gln Asn Val Val Val Thr Glu Ala Val Lys Glu Glu Gln Lys
   165 170 175
   Leu Val Gln Thr Ser Leu His Cys Gly Phe Gln Arg Val Pro Glu Lys
   180 185 190
   Lys Val Ala Trp Lys Tyr Asn Asn Ser Leu Thr Trp Phe Gln Asn Phe
   195 200 205
   Val Ile Leu Asp Leu Tyr Lys Glu Trp His Gln Asn Asn Ser Leu Glu
   210 215 220
   Trp Leu Glu Ile Thr Lys Asp Leu Gln Asp Glu Leu
   225 230 235
<210> 10
   <211> 2033
   <212> DNA
   <213> Homo sapiens
<220>
   <221> CDS
   <222> (106)...(816)
<400> 10
   gcttgaagat ggcagatcgt gggacttctc agcctccata attgagtgag ccaattccct 60
   ggccaaaagg tgtgttttgc tgacttcaag catccctgct acaaa atg gcc tac ttc 117
                                                     Met Ala Tyr Phe
                                                     1
   cat gaa ctg tcc agc cga gtg agc ttt cag gag gca cgc ctg gct tgt    165
   His Glu Leu Ser Ser Arg Val Ser Phe Gln Glu Ala Arg Leu Ala Cys
    5                  10                  15                  20
   gag agt gag gga gga gtc ctc ctc agc ctt gag aat gaa gca gaa cag    213
   Glu Ser Glu Gly Gly Val Leu Leu Ser Leu Glu Asn Glu Ala Glu Gln
                   25                  30                  35
   aag tta ata gag agc atg ttg caa aac ctg aca aaa ccc ggg aca ggg    261
   Lys Leu Ile Glu Ser Met Leu Gln Asn Leu Thr Lys Pro Gly Thr Gly
               40                  45                  50
   att tct gat ggt gat ttc tgg ata ggg ctt tgg agg aat gga gat ggg    309
   Ile Ser Asp Gly Asp Phe Trp Ile Gly Leu Trp Arg Asn Gly Asp Gly
           55                  60                  65
   caa aca tct ggt gcc tgc cca gat ctc tac cag tgg tct gat gga agc    357
   Gln Thr Ser Gly Ala Cys Pro Asp Leu Tyr Gln Trp Ser Asp Gly Ser
       70                  75                  80
   aat tcc cag tac cga aac tgg tac aca gat gaa cct tcc tgc gga agt    405
   Asn Ser Gln Tyr Arg Asn Trp Tyr Thr Asp Glu Pro Ser Cys Gly Ser
   85                  90                  95                  100
   gaa aag tgt gtt gtg atg tat cac caa cca act gcc aat cct ggc ctt    453
   Glu Lys Cys Val Val Met Tyr His Gln Pro Thr Ala Asn Pro Gly Leu
                   105                 110                 115
   ggg ggt ccc tac ctt tac cag tgg aat gat gac agg tgt aac atg aag    501
   Gly Gly Pro Tyr Leu Tyr Gln Trp Asn Asp Asp Arg Cys Asn Met Lys
               120                 125                 130
   cac aat tat att tgc aag tat gaa cca gag att aat cca aca gcc cct    549
   His Asn Tyr Ile Cys Lys Tyr Glu Pro Glu Ile Asn Pro Thr Ala Pro
           135                 140                 145
   gta gaa aag cct tat ctt aca aat caa cca gga gac acc cat cag aat    597
   Val Glu Lys Pro Tyr Leu Thr Asn Gln Pro Gly Asp Thr His Gln Asn
       150                 155                 160
   gtg gtt gtt act gaa gca gta aag gaa gaa caa aaa cta gtc caa acc    645
   Val Val Val Thr Glu Ala Val Lys Glu Glu Gln Lys Leu Val Gln Thr
   165                 170                 175                 180
   agt cta cac tgt gga ttt caa aga gta cca gaa aag aaa gtg gca tgg    693
   Ser Leu His Cys Gly Phe Gln Arg Val Pro Glu Lys Lys Val Ala Trp
                   185                 190                 195
   aag tat aat aac tca ttg act tgg ttc cag aat ttt gta att ctg gat    741
   Lys Tyr Asn Asn Ser Leu Thr Trp Phe Gln Asn Phe Val Ile Leu Asp
               200                 205                 210
   ctg tat aag gaa tgg cat cag aac aat agc ttg gaa tgg ctt gaa atc    789
   Leu Tyr Lys Glu Trp His Gln Asn Asn Ser Leu Glu Trp Leu Glu Ile
           215                 220                 225
   aca aag gat ctg caa gat gaa ctg taa gctccccctt gaggcaaata          836
   Thr Lys Asp Leu Gln Asp Glu Leu  *
       230                 235
   ttaaagtaat ttttatatgt ctattatttc atttaaagaa tatgctgtgc taataatgga 896
   gtgagacatg cttattttgc taaaggatgc acccaaactt caaacttcaa gcaaatgaaa 956
   tggacaatgc agataaagtt gttatcaaca cgtcgggagt atgtgtgtta gaagcaattc 1016
   cttttatttc tttcaccttt cataagttgt tatctagtca atgtaatgta tattgtattg 1076
   aaatttacag tgtgcaaaag tattttacct ttgcataagt gtttgataaa aatgaactgt 1136
   tctaatattt atttttatgg catctcattt ttcaatacat gctcttttga ttaaagaaac 1196
   ttattactgt tgtcaactga attcacacac acacaaatat agtaccatag aaaaagtttg 1256
   ttttctcgaa ataattcatc tttcagcttc tctgcttttg gtcaatgtct aggaaatctc 1316
   ttcagaaata agaagctatt tcattaagtg tgatataaac ctcctcaaac attttactta 1376
   gaggcaagga ttgtctaatt tcaattgtgc aagacatgtg ccttataatt atttttagct 1436
   taaaattaaa cagattttgt aataatgtaa ctttgttaat aggtgcataa acactaatgc 1496
   agtcaatttg aacaaaagaa gtgacataca caatataaat catatgtctt cacacgttgc 1556
   ctatataatg agaagcagct ctctgagggt tctgaaatca atgtggtccc tctcttgccc 1616
   actaaacaaa gatggttgtt cggggtttgg gattgacact ggaggcagat agttgcaaag 1676
   ttagtctaag gtttccctag ctgtatttag cctctgacta tattagtata caaagaggtc 1736
   atgtggttga gaccaggtga atagtcacta tcagtgtgga gacaagcaca gcacacagac 1796
   attttaggaa ggaaaggaac tacgaaatcg tgtgaaaatg ggttggaacc catcagtgat 1856
   cgcatattca ttgatgaggg tttgcttgag atagaaaatg gtggctcctt tctgtcttat 1916
   ctcctagttt cttcaatgct tacgccttgt tcttctcaag agaaagttgt aactctctgg 1976
   tcttcatatg tccctgtgct ccttttaacc aaataaagag ttcttgtttc tgaagaa    2033
<210> 11
   <211> 236
   <212> PRT
   <213> Homo sapiens
<400> 11
   Met Ala Tyr Phe His Glu Leu Ser Ser Arg Val Ser Phe Gln Glu Ala
   1               5                   10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala Val Lys Glu Glu Gln Lys
                   165                 170                 175
   Leu Val Gln Thr Ser Leu His Cys Gly Phe Gln Arg Val Pro Glu Lys
               180                 185                 190
   Lys Val Ala Trp Lys Tyr Asn Asn Ser Leu Thr Trp Phe Gln Asn Phe
           195                 200                 205
   Val Ile Leu Asp Leu Tyr Lys Glu Trp His Gln Asn Asn Ser Leu Glu
       210                 215                 220
   Trp Leu Glu Ile Thr Lys Asp Leu Gln Asp Glu Leu
   225                 230                 235
<210> 12
   <211> 273
   <212> PRT
   <213> Homo sapiens
<400> 12
   Met Ser Arg Val Val Ser Leu Leu Leu Gly Ala Ala Leu Leu Cys Gly
   1 5 10 15
   His Gly Ala Phe Cys Arg Arg Val Val Ser Gly Gln Lys Val Cys Phe
   20 25 30
   Ala Asp Phe Lys His Pro Cys Tyr Lys Met Ala Tyr Phe His Glu Leu
   35 40 45
   Ser Ser Arg Val Ser Phe Gln Glu Ala Arg Leu Ala Cys Glu Ser Glu
   50 55 60
   Gly Gly Val Leu Leu Ser Leu Glu Asn Glu Ala Glu Gln Lys Leu Ile
   65 70 75 80
   Glu Ser Met Leu Gln Asn Leu Thr Lys Pro Gly Thr Gly Ile Ser Asp
   85 90 95
   Gly Asp Phe Trp Ile Gly Leu Trp Arg Asn Gly Asp Gly Gln Thr Ser
   100 105 110
   Gly Ala Cys Pro Asp Leu Tyr Gln Trp Ser Asp Gly Ser Asn Ser Gln
   115 120 125
   Tyr Arg Asn Trp Tyr Thr Asp Glu Pro Ser Cys Gly Ser Glu Lys Cys
   130 135 140
   Val Val Met Tyr His Gln Pro Thr Ala Asn Pro Gly Leu Gly Gly Pro
   145 150 155 160
   Tyr Leu Tyr Gln Trp Asn Asp Asp Arg Cys Asn Met Lys His Asn Tyr
   165 170 175
   Ile Cys Lys Tyr Glu Pro Glu Ile Asn Pro Thr Ala Pro Val Glu Lys
   180 185 190
   Pro Tyr Leu Thr Asn Gln Pro Gly Asp Thr His Gln Asn Val Val Val
   195 200 205
   Thr Glu Ala Gly Ile Ile Pro Asn Leu Ile Tyr Val Val Ile Pro Thr
   210 215 220
   Ile Pro Leu Leu Leu Leu Ile Leu Val Ala Phe Gly Thr Cys Cys Phe
   225 230 235 240
   Gln Met Leu His Lys Ser Lys Gly Arg Thr Lys Thr Ser Pro Asn Gln
   245 250 255
   Ser Thr Leu Trp Ile Ser Lys Ser Thr Arg Lys Glu Ser Gly Met Glu
   260 265 270
   Val
<210> 13
   <211> 232
   <212> PRT
   <213> Homo sapiens
<400> 13 
   Met Ala Tyr Phe His Glu Leu Ser Ser Arg Val Ser Phe Gln Glu Ala
   1 5 10 15
   Arg Leu Ala Cys Glu Ser Glu Gly Gly Val Leu Leu Ser Leu Glu Asn
   20 25 30
   Glu Ala Glu Gln Lys Leu Ile Glu Ser Met Leu Gln Asn Leu Thr Lys
   35 40 45
   Pro Gly Thr Gly Ile Ser Asp Gly Asp Phe Trp Ile Gly Leu Trp Arg
   50 55 60
   Asn Gly Asp Gly Gln Thr Ser Gly Ala Cys Pro Asp Leu Tyr Gln Trp
   65 70 75 80
   Ser Asp Gly Ser Asn Ser Gln Tyr Arg Asn Trp Tyr Thr Asp Glu Pro
   85 90 95
   Ser Cys Gly Ser Glu Lys Cys Val Val Met Tyr His Gln Pro Thr Ala
   100 105 110
   Asn Pro Gly Leu Gly Gly Pro Tyr Leu Tyr Gln Trp Asn Asp Asp Arg
   115 120 125
   Cys Asn Met Lys His Asn Tyr Ile Cys Lys Tyr Glu Pro Glu Ile Asn
   130 135 140
   Pro Thr Ala Pro Val Glu Lys Pro Tyr Leu Thr Asn Gln Pro Gly Asp
   145 150 155 160
   Thr His Gln Asn Val Val Val Thr Glu Ala Gly Ile Ile Pro Asn Leu
   165 170 175
   Ile Tyr Val Val Ile Pro Thr Ile Pro Leu Leu Leu Leu Ile Leu Val
   180 185 190
   Ala Phe Gly Thr Cys Cys Phe Gln Met Leu His Lys Ser Lys Gly Arg
   195 200 205
   Thr Lys Thr Ser Pro Asn Gln Ser Thr Leu Trp Ile Ser Lys Ser Thr
   210 215 220
   Arg Lys Glu Ser Gly Met Glu Val
   225 230_
<210> 14
   <211> 236
   <212> PRT
   <213> Homo sapiens
<400> 14
   Met Ala Tyr Phe His Glu Leu Ser Ser Arg Val Ser Phe Gln Glu Ala
   1 5 10 15
   Arg Leu Ala Cys Glu Ser Glu Gly Gly Val Leu Leu Ser Leu Glu Asn
   20 25 30
   Glu Ala Glu Gln Lys Leu Ile Glu Ser Met Leu Gln Asn Leu Thr Lys
   35 40 45
   Pro Gly Thr Gly Ile Ser Asp Gly Asp Phe Trp Ile Gly Leu Trp Arg
   50 55 60
   Asn Gly Asp Gly Gln Thr Ser Gly Ala Cys Pro Asp Leu Tyr Gln Trp
   65 70 75 80
   Ser Asp Gly Ser Asn Ser Gln Tyr Arg Asn Trp Tyr Thr Asp Glu Pro
   85 90 95
   Ser Cys Gly Ser Glu Lys Cys Val Val Met Tyr His Gln Pro Thr Ala
   100 105 110
   Asn Pro Gly Leu Gly Gly Pro Tyr Leu Tyr Gln Trp Asn Asp Asp Arg
   115 120 125
   Cys Asn Met Lys His Asn Tyr Ile Cys Lys Tyr Glu Pro Glu Ile Asn
   130 135 140
   Pro Thr Ala Pro Val Glu Lys Pro Tyr Leu Thr Asn Gln Pro Gly Asp
   145 150 155 160
   Thr His Gln Asn Val Val Val Thr Glu Ala Val Lys Glu Glu Gln Lys
   165 170 175
   Leu Val Gln Thr Ser Leu His Cys Gly Phe Gln Arg Val Pro Glu Lys
   180 185 190
   Lys Val Ala Trp Lys Tyr Asn Asn Ser Leu Thr Trp Phe Gln Asn Phe
   195 200 205
   Val Ile Leu Asp Leu Tyr Lys Glu Trp His Gln Asn Asn Ser Leu Glu
   210 215 220
   Trp Leu Glu Ile Thr Lys Asp Leu Gln Asp Glu Leu
   225 230 235
<210> 15
   <211> 273
   <212> PRT
   <213> Homo sapiens
<400> 15
   Met Ser Arg Val Val Ser Leu Leu Leu Gly Ala Ala Leu Leu Cys Gly
   1 5 10 15
   His Gly Ala Phe Cys Arg Arg Val Val Ser Gly Gln Lys Val Cys Phe
   20 25 30
   Ala Asp Phe Lys His Pro Cys Tyr Lys Met Ala Tyr Phe His Glu Leu
   35 40 45
   Ser Ser Arg Val Ser Phe Gln Glu Ala Arg Leu Ala Cys Glu Ser Glu
   50 55 60
   Gly Gly Val Leu Leu Ser Leu Glu Asn Glu Ala Glu Gln Lys Leu Ile
   65 70 75 80
   Glu Ser Met Leu Gln Asn Leu Thr Lys Pro Gly Thr Gly Ile Ser Asp
   85 90 95
   Gly Asp Phe Trp Ile Gly Leu Trp Arg Asn Gly Asp Gly Gln Thr Ser
   100 105 110
   Gly Ala Cys Pro Asp Leu Tyr Gln Trp Ser Asp Gly Ser Asn Ser Gln
   115 120 125
   Tyr Arg Asn Trp Tyr Thr Asp Glu Pro Ser Cys Gly Ser Glu Lys Cys
   130 135 140
   Val Val Met Tyr His Gln Pro Thr Ala Asn Pro Gly Leu Gly Gly Pro
   145 150 155 160
   Tyr Leu Tyr Gln Trp Asn Asp Asp Arg Cys Asn Met Lys His Asn Tyr
   165 170 175
   Ile Cys Lys Tyr Glu Pro Glu Ile Asn Pro Thr Ala Pro Val Glu Lys
   180 185 190
   Pro Tyr Leu Thr Asn Gln Pro Gly Asp Thr His Gln Asn Val Val Val
   195 200 205
   Thr Glu Ala Gly Ile Ile Pro Asn Leu Ile Tyr Val Val Ile Pro Thr
   210 215 220
   Ile Pro Leu Leu Leu Leu Ile Leu Val Ala Phe Gly Thr Cys Cys Phe
   225 230 235 240
   Gln Met Leu His Lys Ser Lys Gly Arg Thr Lys Thr Ser Pro Asn Gln
   245 250 255
   Ser Thr Leu Trp Ile Ser Lys Ser Thr Arg Lys Glu Ser Gly Met Glu
   260 265 270
   Val
<210> 16
   <211> 232
   <212> PRT
   <213> Homo sapiens
<400> 16
   Met Ala Tyr Phe His Glu Leu Ser Ser Arg Val Ser Phe Gln Glu Ala
    1               5                  10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala Gly Ile Ile Pro Asn Leu
                   165                 170                 175
   Ile Tyr Val Val Ile Pro Thr Ile Pro Leu Leu Leu Leu Ile Leu Val
               180                 185                 190
   Ala Phe Gly Thr Cys Cys Phe Gln Met Leu His Lys Ser Lys Gly Arg
           195                 200                 205
   Thr Lys Thr Ser Pro Asn Gln Ser Thr Leu Trp Ile Ser Lys Ser Thr
       210                 215                 220
   Arg Lys Glu Ser Gly Met Glu Val
   225                 230
<210> 17
   <211> 236
   <212> PRT
   <213> Homo sapiens
<400> 17
   Met Ala Tyr Phe His Glu Leu Ser Ser Arg Val Ser Phe Gln Glu Ala
    1               5                  10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
               130                      135                           140
   Pro Thr Ala Pro Val Glu Lys Pro Tyr Leu Thr Asn Gln Pro Gly Asp
   145                 150                     155                 160
   Thr His Gln Asn Val Val Val Thr Glu Ala Val Lys Glu Glu Gln Lys
                   165                     170                 175
   Leu Val Gln Thr Ser Leu His Cys Gly Phe Gln Arg Val Pro Glu Lys
               180                     185                 190
   Lys Val Ala Trp Lys Tyr Asn Asn Ser Leu Thr Trp Phe Gln Asn Phe
           195                     200                 205
   Val Ile Leu Asp Leu Tyr Lys Glu Trp His Gln Asn Asn Ser Leu Glu
       210                     215                 220
   Trp Leu Glu Ile Thr Lys Asp Leu Gln Asp Glu Leu
   225                 230                     235
<210> 18
   <211> 14
   <212> PRT
   <213> Tetanus toxoid
<400> 18 <210> 19
   <211> 21
   <212> PRT
   <213> Plasmodium falciparum
<400> 19
<210> 20
   <211> 16
   <212> PRT
   <213> Streptococcus
<400> 20
   Gly Ala Val Asp Ser Ile Leu Gly Gly Val Ala Thr Tyr Gly Ala Ala
   1               5                   10                  15
<210> 21
   <211> 13
   <212> PRT
   <213> Artificial Sequence
<220>
   <223> Pan-DR binding epitope
<221> VARIANT
   <222> 3
   <223> Xaa = cyclohexyalanine, phenylalanine, or tyrosine
<221> VARIANT
   <222> 1, 13
   <223> Xaa = d-alanine or 1-alanine
<400> 21
   Xaa Lys Xaa Val Ala Ala Trp Thr Leu Lys Ala Ala Xaa
   1               5                   10
<210> 22
   <211> 14
   <212> DNA
   <213> Artificial Sequence
<220>
   <223> primer
<400> 22
   ttttgatcaa gctt 14
<210> 23
   <211> 42
   <212> DNA
   <213> Artificial Sequence
<220>
   <223> primer
<400> 23
   ctaatacgac tcactatagg gctcgagcgg ccgcccgggc ag 42
<210> 24
   <211> 12
   <212> DNA
   <213> Artificial Sequence
<220>
   <223> primer
<400> 24
   gatcctgccc gg 12
<210> 25
   <211> 40
   <212> DNA
   <213> Artificial Sequence
<220>
   <223> primer
<400> 25
   gtaatacgac tcactatagg gcagcgtggt cgcggccgag 40
<210> 26
   <211> 10
   <212> DNA
   <213> Artificial Sequence
<220>
   <223> primer
<400> 26
   gatcctcggc 10
<210> 27
   <211> 22
   <212> DNA
   <213> Artificial Sequence
<220>
   <223> primer
<400> 27
   ctaatacgac tcactatagg gc 22
<210> 28
   <211> 22
   <212> DNA
   <213> Artificial Sequence
<220>
   <223> primer
<400> 28
   tcgagcggcc gcccgggcag ga 22
<210> 29
   <211> 20
   <212> DNA
   <213> Artificial Sequence
<220>
   <223> primer
<400> 29
   agcgtggtcg cggccgagga 20
<210> 30
   <211> 25
   <212> DNA
   <213> Artificial Sequence
<220>
   <223> primer
<400> 30
   atatcgccgc gctcgtcgtc gacaa 25
<210> 31
   <211> 26
   <212> DNA
   <213> Artificial Sequence
<220>
   <223> primer
<400> 31
   agccacacgc agctcattgt agaagg 26
<210> 32
   <211> 25
   <212> DNA
   <213> Artificial Sequence
<220>
   <223> primer
<400> 32
   cctgcttcag taacaaccac attct 25
<210> 33
   <211> 24
   <212> DNA
   <213> Artificial Sequence
<220>
   <223> primer
<400> 33
   ctttaccagt ggaatgatga cagg 24
<210> 34
   <211> 24
   <212> DNA
   <213> Artificial Sequence
<220>
   <223> FLAG Tag
<400> 34
   gattacaagg atgacgacga taag 24
<210> 35
   <211> 24
   <212> DNA
   <213> Homo sapiens
<400> 35
   aagggacgaa gacgaacacu uctt 24
<210> 36
   <211> 23
   <212> DNA
   <213> Homo sapiens
<400> 36
   aactgaagac ctgaagacaa taa 23
<210> 37
   <211> 4
   <212> PRT
   <213> Homo sapiens
<400> 37
   Asn Leu Thr Lys
   1
<210> 38
   <211> 4
   <212> PRT
   <213> Homo sapiens
<400> 38
   Asn Pro Thr Ala
   1
<210> 39
   <211> 9
   <212> PRT
   <213> Homo sapiens
<400> 39
   Ser Ser Arg Val Ser Phe Gln Glu Ala
   1 5
<210> 40
   <211> 9
   <212> PRT
   <213> Homo sapiens
<400> 40
   Gly Val Leu Leu Ser Leu Glu Asn Glu
   1 5
<210> 41
   <211> 9.
   <212> PRT
   <213> Homo sapiens
<400> 41
   Gly Thr Gly Ile Ser Asp Gly Asp Phe
   1 5
<210> 42
   <211> 9
   <212> PRT
   <213> Homo sapiens
<400> 42
   Asp Gly Gln Thr Ser Gly Ala Cys Pro
   1 5
<210> 43
   <211> 9
   <212> PRT
   <213> Homo sapiens
<400> 43
   Asp Gly Ser Asn Ser Gln Tyr Arg Asn
   1 5
<210> 44
   <211> 9
   <212> PRT
   <213> Homo sapiens
<400> 44
   Pro Ser Cys Gly Ser Glu Lys Cys Val
   1 5
<210> 45
   <211> 9
   <212> PRT
   <213> Homo sapiens
<400> 45
   Met Leu His Lys Ser Lys Gly Arg Thr
   1 5
<210> 46
   <211> 9
   <212> PRT
   <213> Homo sapiens
<400> 46
   Arg Thr Lys Thr Ser Pro Asn Gln Ser
   1 5
<210> 47
   <211> 9
   <212> PRT
   <213> Homo sapiens
<400> 47
   Thr Arg Lys Glu Ser Gly Met Glu Val
   1 5
<210> 48
   <211> 9
   <212> PRT
   <213> Homo sapiens
<400> 48
   Ile Ser Lys Ser Thr Arg Lys Glu Ser
   1 5
<210> 49
   <211> 9
   <212> PRT
   <213> Homo sapiens
<400> 49
   Cys Pro Asp Leu Tyr Gln Trp Ser Asp
   1 5
<210> 50
   <211> 9
   <212> PRT
   <213> Homo sapiens
<400> 50
   Ser Asn Ser Gln Tyr Arg Asn Trp Tyr
   1 5
<210> 51
   <211> 9
   <212> PRT
   <213> Homo sapiens
<400> 51
   Tyr Arg Asn Trp Tyr Thr Asp Glu Pro
   1 5
<210> 52
   <211> 9
   <212> PRT
   <213> Homo sapiens
<400> 52
   Met Lys His Asn Tyr Ile Cys Lys Tyr
   1 5
<210> 53
   <211> 9
   <212> PRT
   <213> Homo sapiens
<400> 53
   Val Glu Lys Pro Tyr Leu Thr Asn Gln
   1 5
<210> 54
   <211> 9
   <212> PRT
   <213> Homo sapiens
<400> 54
   Pro Asn Leu Ile Tyr Val Val Ile Pro
   1 5
<210> 55
   <211> 23
   <212> PRT
   <213> Homo sapiens
<400> 55
   Ser Met Asx Asn Asp Asn Gly Ser Thr Val Cys Phe Ala Asp Phe Lys
   1 5 10 15
   His Pro Cys Tyr Lys Met Ala
   20
<210> 56
   <211> 273
   <212> PRT
   <213> Homo sapiens
<400> 56
   Met Ser Arg Val Val Ser Leu Leu Leu Gly Ala Ala Leu Leu Cys Gly
   1 5 10 15
   His Gly Ala Phe Cys Arg Arg Val Val Ser Gly Gln Lys Val Cys Phe
   20 25 30
   Ala Asp Phe Lys His Pro Cys Tyr Lys Met Ala Tyr Phe His Glu Leu
   35 40 45
   Ser Ser Arg Val Ser Phe Gln Glu Ala Arg Leu Ala Cys Glu Ser Glu
   50 55 60
   Gly Gly Val Leu Leu Ser Leu Glu Asn Glu Ala Glu Gln Lys Leu Ile
   65 70 75 80
   Glu Ser Met Leu Gln Asn Leu Thr Lys Pro Gly Thr Gly Ile Ser Asp
   85 90 95
   Gly Asp Phe Trp Ile Gly Leu Trp Arg Asn Gly Asp Gly Gln Thr Ser
   100 105 110
   Gly Ala Cys Pro Asp Leu Tyr Gln Trp Ser Asp Gly Ser Asn Ser Gln
   115 120 125
   Tyr Arg Asn Trp Tyr Thr Asp Glu Pro Ser Cys Gly Ser Glu Lys Cys
   130 135 140
   Val Val Met Tyr His Gln Pro Thr Ala Asn Pro Gly Leu Gly Gly Pro
   145 150 155 160
   Tyr Leu Tyr Gln Trp Asn Asp Asp Arg Cys Asn Met Lys His Asn Tyr
   165 170 175
   Ile Cys Lys Tyr Glu Pro Glu Ile Asn Pro Thr Ala Pro Val Glu Lys
   180 185 190
   Pro Tyr Leu Thr Asn Gln Pro Gly Asp Thr His Gln Asn Val Val Val
   195 200 205
   Thr Glu Ala Gly Ile Ile Pro Asn Leu Ile Tyr Val Val Ile Pro Thr
   210 215 220
   Ile Pro Leu Leu Leu Leu Ile Leu Val Ala Phe Gly Thr Cys Cys Phe
   225 230 235 240
   Gln Met Leu His Lys Ser Lys Gly Arg Thr Lys Thr Ser Pro Asn Gln
   245 250 255
   Ser Thr Leu Trp Ile Ser Lys Ser Thr Arg Lys Glu Ser Gly Met Glu
   260 265 270
   Val
<210> 57
   <211> 74
   <212> PRT
   <213> Homo sapiens
<400> 57
   Gln Asn Val Val Val Thr Glu Ala Val Lys Glu Glu Gln Lys Leu Val
   1 5 10 15
   Gln Thr Ser Leu His Cys Gly Phe Gln Arg Val Pro Glu Lys Lys Val
   20 25 30
   Ala Trp Lys Tyr Asn Asn Ser Leu Thr Trp Phe Gln Asn Phe Val Ile
   35 40 45
   Leu Asp Leu Tyr Lys Glu Trp His Gln Asn Asn Ser Leu Glu Trp Leu
   50 55 60
   Glu Ile Thr Lys Asp Leu Gln Asp Glu Leu
   65 70
<210> 58
   <211> 75
   <212> PRT
   <213> Homo sapiens
<400> 58
   His Gln Asn Val Val Val Thr Glu Ala Val Lys Glu Glu Gln Lys Leu
   1 5 10 15
   Val Gln Thr Ser Leu His Cys Gly Phe Gln Arg Val Pro Glu Lys Lys
   20 25 30
   Val Ala Trp Lys Tyr Asn Asn Ser Leu Thr Trp Phe Gln Asn Phe Val
   35 40 45
   Ile Leu Asp Leu Tyr Lys Glu Trp His Gln Asn Asn Ser Leu Glu Trp
   50 55 60
   Leu Glu Ile Thr Lys Asp Leu Gln Asp Glu Leu
   65 70 75
<210> 59
   <211> 80
   <212> PRT
   <213> Homo sapiens
<400> 59
   Gln Pro Gly Asp Thr His Gln Asn Val Val Val Thr Glu Ala Val Lys
   1 5 10 15
   Glu Glu Gln Lys Leu Val Gln Thr Ser Leu His Cys Gly Phe Gln Arg
   20 25 30
   Val Pro Glu Lys Lys Val Ala Trp Lys Tyr Asn Asn Ser Leu Thr Trp
   35 40 45
   Phe Gln Asn Phe Val Ile Leu Asp Leu Tyr Lys Glu Trp His Gln Asn
   50 55 60
   Asn Ser Leu Glu Trp Leu Glu Ile Thr Lys Asp Leu Gln Asp Glu Leu
   65 70 75 80
<210> 60
   <211> 2418
   <212> DNA
   <213> Homo sapiens
<400> 60
   ctgtggtgtt tttcccccgc tcctctggct gccttcctga tggatctctg tggtcccagg 60
   caggaatggc ctgcttgggg acccagcgag ctcccaaggc ctttcctgct gcttcctcta 120
   tccctgtgtt ttgcttggct ctctaaattg actcagctcc aggacatcag gaccccaggt 180
   tctctggtct tgggactctg agacttgcac caggaatcct gcccaggctc tcaggccttt 240
   ggactcagac tgagctactt cactggcttt cctggttctc cagcttgaag atggcagatc 300
   gtgggacttc tcagcctcca taattgagtg agccaattcc ctggccaaaa ggtgtgtttt 360
   gctgacttca agcatccctg ctacaaaatg gcctacttcc atgaactgtc cagccgagtg 420
   agctttcagg aggcacgcct ggcttgtgag agtgagggag gagtcctcct cagccttgag 480
   aatgaagcag aacagaagtt aatagagagc atgttgcaaa acctgacaaa acccgggaca 540
   gggatttctg atggtgattt ctggataggg ctttggagga atggagatgg gcaaacatct 600
   ggtgcctgcc cagatctcta ccagtggtct gatggaagca attcccagta ccgaaactgg 660
   tacacagatg aaccttcctg cggaagtgaa aagtgtgttg tgatgtatca ccaaccaact 720
   gccaatcctg gccttggggg tccctacctt taccagtgga atgatgacag gtgtaacatg 780
   aagcacaatt atatttgcaa gtatgaacca gagattaatc caacagcccc tgtagaaaag 840
   ccttatctta caaatcaacc aggagacacc catcagaatg tggttgttac tgaagcaggt 900
   ataattccca atctaattta tgttgttata ccaacaatac ccctgctctt actgatactg 960
   gttgcttttg gaacctgttg tttccagatg ctgcataaaa gtaaaggaag aacaaaaact 1020
   agtccaaacc agtctacact gtggatttca aagagtacca gaaaagaaag tggcatggaa 1080
   gtataataac tcattgactt ggttccagaa ttttgtaatt ctggatctgt ataaggaatg 1140
   gcatcagaac aatagcttgg aatggcttga aatcacaaag gatctgcaag atgaactgta 1200
   agctccccct tgaggcaaat attaaagtaa tttttatatg tctattattt catttaaaga 1260
   atatgctgtg ctaataatgg agtgagacat gcttattttg ctaaaggatg cacccaaact 1320
   tcaaacttca agcaaatgaa atggacaatg cagataaagt tgttatcaac acgtcgggag 1380
   tatgtgtgtt agaagcaatt ccttttattt ctttcacctt tcataagttg ttatctagtc 1440
   aatgtaatgt atattgtatt gaaatttaca gtgtgcaaaa gtattttacc tttgcataag 1500
   tgtttgataa aaatgaactg ttctaatatt tatttttatg gcatctcatt tttcaataca 1560
   tgctcttttg attaaagaaa cttattactg ttgtcaactg aattcacaca cacacaaata 1620
   tagtaccata gaaaaagttt gttttctcga aataattcat ctttcagctt ctctgctttt 1680
   ggtcaatgtc taggaaatct cttcagaaat aagaagctat ttcattaagt gtgatataaa 1740
   cctcctcaaa cattttactt agaggcaagg attgtctaat ttcaattgtg caagacatgt 1800
   gccttataat tatttttagc ttaaaattaa acagattttg taataatgta actttgttaa 1860
   taggtgcata aacactaatg cagtcaattt gaacaaaaga agtgacatac acaatataaa 1920
   tcatatgtct tcacacgttg cctatataat gagaagcagc tctctgaggg ttctgaaatc 1980
   aatgtggtcc ctctcttgcc cactaaacaa agatggttgt tcggggtttg ggattgacac 2040
   tggaggcaga tagttgcaaa gttagtctaa ggtttcccta gctgtattta gcctctgact 2100
   atattagtat acaaagaggt catgtggttg agaccaggtg aatagtcact atcagtgtgg 2160
   agacaagcac agcacacaga cattttagga aggaaaggaa ctacgaaatc gtgtgaaaat 2220
   gggttggaac ccatcagtga tcgcatattc attgatgagg gtttgcttga gatagaaaat 2280
   ggtggctcct ttctgtctta tctcctagtt tcttcaatgc ttacgccttg ttcttctcaa 2340
   gagaaagttg taactctctg gtcttcatat gtccctgtgc tccttttaac caaataaaga 2400
   gttcttgttt ctgaagaa                                               2418
<210> 61
   <211> 2076
   <212> DNA
   <213> Homo sapiens
<400> 61
   ggccaaaagg tgtgttttgc tgacttcaag catccctgct acaaaatggc ctacttccat 60
   gaactgtcca gccgagtgag ctttcaggag gcacgcctgg cttgtgagag tgagggagga 120
   gtcctcctca gccttgagaa tgaagcagaa cagaagttaa tagagagcat gttgcaaaac 180
   ctgacaaaac ccgggacagg gatttctgat ggtgatttct ggatagggct ttggaggaat 240
   ggagatgggc aaacatctgg tgcctgccca gatctctacc agtggtctga tggaagcaat 300
   tcccagtacc gaaactggta cacagatgaa ccttcctgcg gaagtgaaaa gtgtgttgtg 360
   atgtatcacc aaccaactgc caatcctggc cttgggggtc cctaccttta ccagtggaat 420
   gatgacaggt gtaacatgaa gcacaattat atttgcaagt atgaaccaga gattaatcca 480
   acagcccctg tagaaaagcc ttatcttaca aatcaaccag gagacaccca tcagaatgtg 540
   gttgttactg aagcaggtat aattcccaat ctaatttatg ttgttatacc aacaataccc 600
   ctgctcttac tgatactggt tgcttttgga acctgttgtt tccagatgct gcataaaagt 660
   aaaggaagaa caaaaactag tccaaaccag tctacactgt ggatttcaaa gagtaccaga 720
   aaagaaagtg gcatggaagt ataataactc attgacttgg ttccagaatt ttgtaattct 780
   ggatctgtat aaggaatggc atcagaacaa tagcttggaa tggcttgaaa tcacaaagga 840
   tctgcaagat gaactgtaag ctcccccttg aggcaaatat taaagtaatt tttatatgtc 900
   tattatttca tttaaagaat atgctgtgct aataatggag tgagacatgc ttattttgct 960
   aaaggatgca cccaaacttc aaacttcaag caaatgaaat ggacaatgca gataaagttg 1020
   ttatcaacac gtcgggagta tgtgtgttag aagcaattcc ttttatttct ttcacctttc 1080
   ataagttgtt atctagtcaa tgtaatgtat attgtattga aatttacagt gtgcaaaagt 1140
   attttacctt tgcataagtg tttgataaaa atgaactgtt ctaatattta tttttatggc 1200
   atctcatttt tcaatacatg ctcttttgat taaagaaact tattactgtt gtcaactgaa 1260
   ttcacacaca cacaaatata gtaccataga aaaagtttgt tttctcgaaa taattcatct 1320
   ttcagcttct ctgcttttgg tcaatgtcta ggaaatctct tcagaaataa gaagctattt 1380
   cattaagtgt gatataaacc tcctcaaaca ttttacttag aggcaaggat tgtctaattt 1440
   caattgtgca agacatgtgc cttataatta tttttagctt aaaattaaac agattttgta 1500
   ataatgtaac tttgttaata ggtgcataaa cactaatgca gtcaatttga acaaaagaag 1560
   tgacatacac aatataaatc atatgtcttc acacgttgcc tatataatga gaagcagctc 1620
   tctgagggtt ctgaaatcaa tgtggtccct ctcttgccca ctaaacaaag atggttgttc 1680
   ggggtttggg attgacactg gaggcagata gttgcaaagt tagtctaagg tttccctagc 1740
   tgtatttagc ctctgactat attagtatac aaagaggtca tgtggttgag accaggtgaa 1800
   tagtcactat cagtgtggag acaagcacag cacacagaca ttttaggaag gaaaggaact 1860
   acgaaatcgt gtgaaaatgg gttggaaccc atcagtgatc gcatattcat tgatgagggt 1920
   ttgcttgaga tagaaaatgg tggctccttt ctgtcttatc tcctagtttc ttcaatgctt 1980
   acgccttgtt cttctcaaga gaaagttgta actctctggt cttcatatgt ccctgtgctc 2040
   cttttaacca aataaagagt tcttgtttct gaagaa 2076
<210> 62
   <211> 2418
   <212> DNA
   <213> Homo sapiens
<400> 62
   ctgtggtgtt tttcccccgc tcctctggct gccttcctga tggatctctg tggtcccagg 60
   caggaatggc ctgcttgggg acccagcgag ctcccaaggc ctttcctgct gcttcctcta 120
   tccctgtgtt ttgcttggct ctctaaattg actcagctcc aggacatcag gaccccaggt 180
   tctctggtct tgggactctg agacttgcac caggaatcct gcccaggctc tcaggccttt 240
   ggactcagac tgagctactt cactggcttt cctggttctc cagcttgaag atggcagatc 300
   gtgggacttc tcagcctcca taattgagtg agccaattcc ctggccaaaa ggtgtgtttt 360
   gctgacttca agcatccctg ctacaaaatg gcctacttcc atgaactgtc cagccgagtg 420
   agctttcagg aggcacgcct ggcttgtgag agtgagggag gagtcctcct cagccttgag 480
   aatgaagcag aacagaagtt aatagagagc atgttgcaaa acctgacaaa acccgggaca 540
   gggatttctg atggtgattt ctggataggg ctttggagga atggagatgg gcaaacatct 600
   ggtgcctgcc cagatctcta ccagtggtct gatggaagca attcccagta ccgaaactgg 660
   tacacagatg aaccttcctg cggaagtgaa aagtgtgttg tgatgtatca ccaaccaact 720
   gccaatcctg gccttggggg tccctacctt taccagtgga atgatgacag gtgtaacatg 780
   aagcacaatt atatttgcaa gtatgaacca gagattaatc caacagcccc tgtagaaaag 840
   ccttatctta caaatcaacc aggagacacc catcagaatg tggttgttac tgaagcaggt 900
   ataattccca atctaattta tgttgttata ccaacaatac ccctgctctt actgatactg 960
   gttgcttttg gaacctgttg tttccagatg ctgcataaaa gtaaaggaag aacaaaaact 1020
   agtccaaacc agtctacact gtggatttca aagagtacca gaaaagaaag tggcatggaa 1080
   gtataataac tcattgactt ggttccagaa ttttgtaatt ctggatctgt ataaggaatg 1140
   gcatcagaac aatagcttgg aatggcttga aatcacaaag gatctgcaag atgaactgta 1200
   agctccccct tgaggcaaat attaaagtaa tttttatatg tctattattt catttaaaga 1260
   atatgctgtg ctaataatgg agtgagacat gcttattttg ctaaaggatg cacccaaact 1320
   tcaaacttca agcaaatgaa atggacaatg cagataaagt tgttatcaac acgtcgggag 1380
   tatgtgtgtt agaagcaatt ccttttattt ctttcacctt tcataagttg ttatctagtc 1440
   aatgtaatgt atattgtatt gaaatttaca gtgtgcaaaa gtattttacc tttgcataag 1500
   tgtttgataa aaatgaactg ttctaatatt tatttttatg gcatctcatt tttcaataca 1560
   tgctcttttg attaaagaaa cttattactg ttgtcaactg aattcacaca cacacaaata 1620
   tagtaccata gaaaaagttt gttttctcga aataattcat ctttcagctt ctctgctttt 1680
   ggtcaatgtc taggaaatct cttcagaaat aagaagctat ttcattaagt gtgatataaa 1740
   cctcctcaaa cattttactt agaggcaagg attgtctaat ttcaattgtg caagacatgt 1800
   gccttataat tatttttagc ttaaaattaa acagattttg taataatgta actttgttaa 1860
   taggtgcata aacactaatg cagtcaattt gaacaaaaga agtgacatac acaatataaa 1920
   tcatatgtct tcacacgttg cctatataat gagaagcagc tctctgaggg ttctgaaatc 1980
   aatgtggtcc ctctcttgcc cactaaacaa agatggttgt tcggggtttg ggattgacac 2040
   tggaggcaga tagttgcaaa gttagtctaa ggtttcccta gctgtattta gcctctgact 2100
   atattagtat acaaagaggt catgtggttg agaccaggtg aatagtcact atcagtgtgg 2160
   agacaagcac agcacacaga cattttagga aggaaaggaa ctacgaaatc gtgtgaaaat 2220
   gggttggaac ccatcagtga tcgcatattc attgatgagg gtttgcttga gatagaaaat 2280
   ggtggctcct ttctgtctta tctcctagtt tcttcaatgc ttacgccttg ttcttctcaa 2340
   gagaaagttg taactctctg gtcttcatat gtccctgtgc tccttttaac caaataaaga 2400
   gttcttgttt ctgaagaa                                               2418
<210> 63
   <211> 232
   <212> PRT
   <213> Homo sapiens
<400> 63
   Met Ala Tyr Phe His Glu Leu Ser Ser Arg Val Ser Phe Gln Glu Ala
    1               5                  10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala Gly Ile Ile Pro Asn Leu
                   165                 170                 175
   Ile Tyr Val Val Ile Pro Thr Ile Pro Leu Leu Leu Leu Ile Leu Val
               180                 185                 190
   Ala Phe Gly Thr Cys Cys Phe Gln Met Leu His Lys Ser Lys Gly Arg
           195                 200                 205
   Thr Lys Thr Ser Pro Asn Gln Ser Thr Leu Trp Ile Ser Lys Ser Thr
       210                 215                 220
   Arg Lys Glu Ser Gly Met Glu Val
   225                 230
<210> 64
   <211> 232
   <212> PRT
   <213> Homo sapiens
<400> 64
   Met Ala Tyr Phe His Glu Leu Ser Ser Arg Val Ser Phe Gln Glu Ala
   1               5                   10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala Gly Ile Ile Pro Asn Leu
                   165                 170                 175
   Ile Tyr Val Val Ile Pro Thr Ile Pro Leu Leu Leu Leu Ile Leu Val
               180                 185                 190
   Ala Phe Gly Thr Cys Cys Phe Gln Met Leu His Lys Ser Lys Gly Arg
           195                 200                 205
   Thr Lys Thr Ser Pro Asn Gln Ser Thr Leu Trp Ile Ser Lys Ser Thr
       210                 215                 220
   Arg Lys Glu Ser Gly Met Glu Val
   225                 230
<210> 65
   <211> 232
   <212> PRT
   <213> Homo sapiens
<400> 65 
   Met Ala Tyr Phe His Glu Leu Ser Ser Arg Val Ser Phe Gln Glu Ala
   1               5                   10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala Gly Ile Ile Pro Asn Leu
                   165                 170                 175
   Ile Tyr Val Val Ile Pro Thr Ile Pro Leu Leu Leu Leu Ile Leu Val
               180                 185                 190
   Ala Phe Gly Thr Cys Cys Phe Gln Met Leu His Lys Ser Lys Gly Arg
           195                 200                 205
   Thr Lys Thr Ser Pro Asn Gln Ser Thr Leu Trp Ile Ser Lys Ser Thr
       210                 215                 220
   Arg Lys Glu Ser Gly Met Glu Val
   225                 230_
<210> 66
   <211> 2236
   <212> DNA
   <213> Homo sapiens
<400> 66
   gcttgaagat ggcagatcgt gggacttctc agcctccata attgagtgag ccaattccct 60
   gaatacaaca agaagatggc catctgtaga ccaggaggtg gtccctcccc agaaactgga 120
   tgggccagca cgttgattct gagcttctag cctccagaac tgccaaaagg tgtgttttgc 180
   tgacttcaag catccctgct acaaaatggc ctacttccat gaactgtcca gccgagtgag 240
   ctttcaggag gcacgcctgg cttgtgagag tgagggagga gtcctcctca gccttgagaa 300
   tgaagcagaa cagaagttaa tagagagcat gttgcaaaac ctgacaaaac ccgggacagg 360
   gatttctgat ggtgatttct ggatagggct ttggaggaat ggagatgggc aaacatctgg 420
   tgcctgccca gatctctacc agtggtctga tggaagcaat tcccagtacc gaaactggta 480
   cacagatgaa ccttcctgcg gaagtgaaaa gtgtgttgtg atgtatcacc aaccaactgc 540
   caatcctggc cttgggggtc cctaccttta ccagtggaat gatgacaggt gtaacatgaa 600
   gcacaattat atttgcaagt atgaaccaga gattaatcca acagcccctg tagaaaagcc 660
   ttatcttaca aatcaaccag gagacaccca tcagaatgtg gttgttactg aagcaggtat 720
   aattcccaat ctaatttatg ttgttatacc aacaataccc ctgctcttac tgatactggt 780
   tgcttttgga acctgttgtt tccagatgct gcataaaagt aaaggaagaa caaaaactag 840
   tccaaaccag tctacactgt ggatttcaaa gagtaccaga aaagaaagtg gcatggaagt 900
   ataataactc attgacttgg ttccagaatt ttgtaattct ggatctgtat aaggaatggc 960
   atcagaacaa tagcttggaa tggcttgaaa tcacaaagga tctgcaagat gaactgtaag 1020
   ctcccccttg aggcaaatat taaagtaatt tttatatgtc tattatttca tttaaagaat 1080
   atgctgtgct aataatggag tgagacatgc ttattttgct aaaggatgca cccaaacttc 1140
   aaacttcaag caaatgaaat ggacaatgca gataaagttg ttatcaacac gtcgggagta 1200
   tgtgtgttag aagcaattcc ttttatttct ttcacctttc ataagttgtt atctagtcaa 1260
   tgtaatgtat attgtattga aatttacagt gtgcaaaagt attttacctt tgcataagtg 1320
   tttgataaaa atgaactgtt ctaatattta tttttatggc atctcatttt tcaatacatg 1380
   ctcttttgat taaagaaact tattactgtt gtcaactgaa ttcacacaca cacaaatata 1440
   gtaccataga aaaagtttgt tttctcgaaa taattcatct ttcagcttct ctgcttttgg 1500
   tcaatgtcta ggaaatctct tcagaaataa gaagctattt cattaagtgt gatataaacc 1560
   tcctcaaaca ttttacttag aggcaaggat tgtctaattt caattgtgca agacatgtgc 1620
   cttataatta tttttagctt aaaattaaac agattttgta ataatgtaac tttgttaata 1680
   ggtgcataaa cactaatgca gtcaatttga acaaaagaag tgacatacac aatataaatc 1740
   atatgtcttc acacgttgcc tatataatga gaagcagctc tctgagggtt ctgaaatcaa 1800
   tgtggtccct ctcttgccca ctaaacaaag atggttgttc ggggtttggg attgacactg 1860
   gaggcagata gttgcaaagt tagtctaagg tttccctagc tgtatttagc ctctgactat 1920
   attagtatac aaagaggtca tgtggttgag accaggtgaa tagtcactat cagtgtggag 1980
   acaagcacag cacacagaca ttttaggaag gaaaggaact acgaaatcgt gtgaaaatgg 2040
   gttggaaccc atcagtgatc gcatattcat tgatgagggt ttgcttgaga tagaaaatgg 2100
   tggctccttt ctgtcttatc tcctagtttc ttcaatgctt acgccttgtt cttctcaaga 2160
   gaaagttgta actctctggt cttcatatgt ccctgtgctc cttttaacca aataaagagt 2220
   tcttgtttct gaagaa                                                  2236
<210> 67
   <211> 2076
   <212> DNA
   <213> Homo sapiens
<400> 67
   ggccaaaagg tgtgttttgc tgacttcaag catccctgct acaaaatggc ctacttccat 60
   gaactgtcca gccgagtgag ctttcaggag gcacgcctgg cttgtgagag tgagggagga 120
   gtcctcctca gccttgagaa tgaagcagaa cagaagttaa tagagagcat gttgcaaaac 180
   ctgacaaaac ccgggacagg gatttctgat ggtgatttct ggatagggct ttggaggaat 240
   ggagatgggc aaacatctgg tgcctgccca gatctctacc agtggtctga tggaagcaat 300
   tcccagtacc gaaactggta cacagatgaa ccttcctgcg gaagtgaaaa gtgtgttgtg 360
   atgtatcacc aaccaactgc caatcctggc cttgggggtc cctaccttta ccagtggaat 420
   gatgacaggt gtaacatgaa gcacaattat atttgcaagt atgaaccaga gattaatcca 480
   acagcccctg tagaaaagcc ttatcttaca aatcaaccag gagacaccca tcagaatgtg 540
   gttgttactg aagcaggtat aattcccaat ctaatttatg ttgttatacc aacaataccc 600
   ctgctcttac tgatactggt tgcttttgga acctgttgtt tccagatgct gcataaaagt 660
   aaaggaagaa caaaaactag tccaaaccag tctacactgt ggatttcaaa gagtaccaga 720
   aaagaaagtg gcatggaagt ataataactc attgacttgg ttccagaatt ttgtaattct 780
   ggatctgtat aaggaatggc atcagaacaa tagcttggaa tggcttgaaa tcacaaagga 840
   tctgcaagat gaactgtaag ctcccccttg aggcaaatat taaagtaatt tttatatgtc 900
   tattatttca tttaaagaat atgctgtgct aataatggag tgagacatgc ttattttgct 960
   aaaggatgca cccaaacttc aaacttcaag caaatgaaat ggacaatgca gataaagttg 1020
   ttatcaacac gtcgggagta tgtgtgttag aagcaattcc ttttatttct ttcacctttc 1080
   ataagttgtt atctagtcaa tgtaatgtat attgtattga aatttacagt gtgcaaaagt 1140
   attttacctt tgcataagtg tttgataaaa atgaactgtt ctaatattta tttttatggc 1200
   atctcatttt tcaatacatg ctcttttgat taaagaaact tattactgtt gtcaactgaa 1260
   ttcacacaca cacaaatata gtaccataga aaaagtttgt tttctcgaaa taattcatct 1320
   ttcagcttct ctgcttttgg tcaatgtcta ggaaatctct tcagaaataa gaagctattt 1380
   cattaagtgt gatataaacc tcctcaaaca ttttacttag aggcaaggat tgtctaattt 1440
   caattgtgca agacatgtgc cttataatta tttttagctt aaaattaaac agattttgta 1500
   ataatgtaac tttgttaata ggtgcataaa cactaatgca gtcaatttga acaaaagaag 1560
   tgacatacac aatataaatc atatgtcttc acacgttgcc tatataatga gaagcagctc 1620
   tctgagggtt ctgaaatcaa tgtggtccct ctcttgccca ctaaacaaag atggttgttc 1680
   ggggtttggg attgacactg gaggcagata gttgcaaagt tagtctaagg tttccctagc 1740
   tgtatttagc ctctgactat attagtatac aaagaggtca tgtggttgag accaggtgaa 1800
   tagtcactat cagtgtggag acaagcacag cacacagaca ttttaggaag gaaaggaact 1860
   acgaaatcgt gtgaaaatgg gttggaaccc atcagtgatc gcatattcat tgatgagggt 1920
   ttgcttgaga tagaaaatgg tggctccttt ctgtcttatc tcctagtttc ttcaatgctt 1980
   acgccttgtt cttctcaaga gaaagttgta actctctggt cttcatatgt ccctgtgctc 2040
   cttttaacca aataaagagt tcttgtttct gaagaa 2076
<210> 68
   <211> 2236
   <212> DNA
   <213> Homo sapiens
<400> 68
   gcttgaagat ggcagatcgt gggacttctc agcctccata attgagtgag ccaattccct 60
   gaatacaaca agaagatggc catctgtaga ccaggaggtg gtccctcccc agaaactgga 120
   tgggccagca cgttgattct gagcttctag cctccagaac tgccaaaagg tgtgttttgc 180
   tgacttcaag catccctgct acaaaatggc ctacttccat gaactgtcca gccgagtgag 240
   ctttcaggag gcacgcctgg cttgtgagag tgagggagga gtcctcctca gccttgagaa 300
   tgaagcagaa cagaagttaa tagagagcat gttgcaaaac ctgacaaaac ccgggacagg 360
   gatttctgat ggtgatttct ggatagggct ttggaggaat ggagatgggc aaacatctgg 420
   tgcctgccca gatctctacc agtggtctga tggaagcaat tcccagtacc gaaactggta 480
   cacagatgaa ccttcctgcg gaagtgaaaa gtgtgttgtg atgtatcacc aaccaactgc 540
   caatcctggc cttgggggtc cctaccttta ccagtggaat gatgacaggt gtaacatgaa 600
   gcacaattat atttgcaagt atgaaccaga gattaatcca acagcccctg tagaaaagcc 660
   ttatcttaca aatcaaccag gagacaccca tcagaatgtg gttgttactg aagcaggtat 720
   aattcccaat ctaatttatg ttgttatacc aacaataccc ctgctcttac tgatactggt 780
   tgcttttgga acctgttgtt tccagatgct gcataaaagt aaaggaagaa caaaaactag 840
   tccaaaccag tctacactgt ggatttcaaa gagtaccaga aaagaaagtg gcatggaagt 900
   ataataactc attgacttgg ttccagaatt ttgtaattct ggatctgtat aaggaatggc 960
   atcagaacaa tagcttggaa tggcttgaaa tcacaaagga tctgcaagat gaactgtaag 1020
   ctcccccttg aggcaaatat taaagtaatt tttatatgtc tattatttca tttaaagaat 1080
   atgctgtgct aataatggag tgagacatgc ttattttgct aaaggatgca cccaaacttc 1140
   aaacttcaag caaatgaaat ggacaatgca gataaagttg ttatcaacac gtcgggagta 1200
   tgtgtgttag aagcaattcc ttttatttct ttcacctttc ataagttgtt atctagtcaa 1260
   tgtaatgtat attgtattga aatttacagt gtgcaaaagt attttacctt tgcataagtg 1320
   tttgataaaa atgaactgtt ctaatattta tttttatggc atctcatttt tcaatacatg 1380
   ctcttttgat taaagaaact tattactgtt gtcaactgaa ttcacacaca cacaaatata 1440
   gtaccataga aaaagtttgt tttctcgaaa taattcatct ttcagcttct ctgcttttgg 1500
   tcaatgtcta ggaaatctct tcagaaataa gaagctattt cattaagtgt gatataaacc 1560
   tcctcaaaca ttttacttag aggcaaggat tgtctaattt caattgtgca agacatgtgc 1620
   cttataatta tttttagctt aaaattaaac agattttgta ataatgtaac tttgttaata 1680
   ggtgcataaa cactaatgca gtcaatttga acaaaagaag tgacatacac aatataaatc 1740
   atatgtcttc acacgttgcc tatataatga gaagcagctc tctgagggtt ctgaaatcaa 1800
   tgtggtccct ctcttgccca ctaaacaaag atggttgttc ggggtttggg attgacactg 1860
   gaggcagata gttgcaaagt tagtctaagg tttccctagc tgtatttagc ctctgactat 1920
   attagtatac aaagaggtca tgtggttgag accaggtgaa tagtcactat cagtgtggag 1980
   acaagcacag cacacagaca ttttaggaag gaaaggaact acgaaatcgt gtgaaaatgg 2040
   gttggaaccc atcagtgatc gcatattcat tgatgagggt ttgcttgaga tagaaaatgg 2100
   tggctccttt ctgtcttatc tcctagtttc ttcaatgctt acgccttgtt cttctcaaga 2160
   gaaagttgta actctctggt cttcatatgt ccctgtgctc cttttaacca aataaagagt 2220
   tcttgtttct gaagaa                                                 2236
<210> 69
   <211> 232
   <212> PRT
   <213> Homo sapiens
<400> 69
   Met Ala Tyr Phe His Glu Leu Ser Ser Arg Val Ser Phe Gln Glu Ala
   1               5                   10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala Gly Ile Ile Pro Asn Leu
                   165                 170                 175
   Ile Tyr Val Val Ile Pro Thr Ile Pro Leu Leu Leu Leu Ile Leu Val
               180                 185                 190
   Ala Phe Gly Thr Cys Cys Phe Gln Met Leu His Lys Ser Lys Gly Arg
           195                 200                 205
   Thr Lys Thr Ser Pro Asn Gln Ser Thr Leu Trp Ile Ser Lys Ser Thr
       210                 215                 220
   Arg Lys Glu Ser Gly Met Glu Val
   225                 230
<210> 70
   <211> 232
   <212> PRT
   <213> Homo sapiens
<400> 70
<Z10> 71
   <211> 232
   <212> PRT
   <213> Homo sapiens
<400> 71
<210> 72
   <211> 2133
   <212> DNA
   <213> Homo sapiens
<400> 72
   gcttgaagat ggcagatcgt gggacttctc agcctccata attgagtgag ccaattccct 60
   gaatacaaca agaagatggc catctgtaga ccaggaggtg gtccctcccc agaaactgga 120
   tgggccagca cgttgattct gagcttctag cctccagaac tgccaaaagg tgtgttttgc 180
   tgacttcaag catccctgct acaaaatggc ctacttccat gaactgtcca gccgagtgag 240
   ctttcaggag gcacgcctgg cttgtgagag tgagggagga gtcctcctca gccttgagaa 300
   tgaagcagaa cagaagttaa tagagagcat gttgcaaaac ctgacaaaac ccgggacagg 360
   gatttctgat ggtgatttct ggatagggct ttggaggaat ggagatgggc aaacatctgg 420
   tgcctgccca gatctctacc agtggtctga tggaagcaat tcccagtacc gaaactggta 480
   cacagatgaa ccttcctgcg gaagtgaaaa gtgtgttgtg atgtatcacc aaccaactgc 540
   caatcctggc cttgggggtc cctaccttta ccagtggaat gatgacaggt gtaacatgaa 600
   gcacaattat atttgcaagt atgaaccaga gattaatcca acagcccctg tagaaaagcc 660
   ttatcttaca aatcaaccag gagacaccca tcagaatgtg gttgttactg aagcagtaaa 720
   ggaagaacaa aaactagtcc aaaccagtct acactgtgga tttcaaagag taccagaaaa 780
   gaaagtggca tggaagtata ataactcatt gacttggttc cagaattttg taattctgga 840
   tctgtataag gaatggcatc agaacaatag cttggaatgg cttgaaatca caaaggatct 900
   gcaagatgaa ctgtaagctc ccccttgagg caaatattaa agtaattttt atatgtctat 960
   tatttcattt aaagaatatg ctgtgctaat aatggagtga gacatgctta ttttgctaaa 1020
   ggatgcaccc aaacttcaaa cttcaagcaa atgaaatgga caatgcagat aaagttgtta 1080
   tcaacacgtc gggagtatgt gtgttagaag caattccttt tatttctttc acctttcata 1140
   agttgttatc tagtcaatgt aatgtatatt gtattgaaat ttacagtgtg caaaagtatt 1200
   ttacctttgc ataagtgttt gataaaaatg aactgttcta atatttattt ttatggcatc 1260
   tcatttttca atacatgctc ttttgattaa agaaacttat tactgttgtc aactgaattc 1320
   acacacacac aaatatagta ccatagaaaa agtttgtttt ctcgaaataa ttcatctttc 1380
   agcttctctg cttttggtca atgtctagga aatctcttca gaaataagaa gctatttcat 1440
   taagtgtgat ataaacctcc tcaaacattt tacttagagg caaggattgt ctaatttcaa 1500
   ttgtgcaaga catgtgcctt ataattattt ttagcttaaa attaaacaga ttttgtaata 1560
   atgtaacttt gttaataggt gcataaacac taatgcagtc aatttgaaca aaagaagtga 1620
   catacacaat ataaatcata tgtcttcaca cgttgcctat ataatgagaa gcagctctct 1680
   gagggttctg aaatcaatgt ggtccctctc ttgcccacta aacaaagatg gttgttcggg 1740
   gtttgggatt gacactggag gcagatagtt gcaaagttag tctaaggttt ccctagctgt 1800
   atttagcctc tgactatatt agtatacaaa gaggtcatgt ggttgagacc aggtgaatag 1860
   tcactatcag tgtggagaca agcacagcac acagacattt taggaaggaa aggaactacg 1920
   aaatcgtgtg aaaatgggtt ggaacccatc agtgatcgca tattcattga tgagggtttg 1980
   cttgagatag aaaatggtgg ctcctttctg tcttatctcc tagtttcttc aatgcttacg 2040
   ccttgttctt ctcaagagaa agttgtaact ctctggtctt catatgtccc tgtgctcctt 2100
   ttaaccaaat aaagagttct tgtttctgaa gaa 2133
<210> 73
   <211> 2076
   <212> DNA
   <213> Homo sapiens
<400> 73
   ggccaaaagg tgtgttttgc tgacttcaag catccctgct acaaaatggc ctacttccat 60
   gaactgtcca gccgagtgag ctttcaggag gcacgcctgg cttgtgagag tgagggagga 120
   gtcctcctca gccttgagaa tgaagcagaa cagaagttaa tagagagcat gttgcaaaac 180
   ctgacaaaac ccgggacagg gatttctgat ggtgatttct ggatagggct ttggaggaat 240
   ggagatgggc aaacatctgg tgcctgccca gatctctacc agtggtctga tggaagcaat 300
   tcccagtacc gaaactggta cacagatgaa ccttcctgcg gaagtgaaaa gtgtgttgtg 360
   atgtatcacc aaccaactgc caatcctggc cttgggggtc cctaccttta ccagtggaat 420
   gatgacaggt gtaacatgaa gcacaattat atttgcaagt atgaaccaga gattaatcca 480
   acagcccctg tagaaaagcc ttatcttaca aatcaaccag gagacaccca tcagaatgtg 540
   gttgttactg aagcaggtat aattcccaat ctaatttatg ttgttatacc aacaataccc 600
   ctgctcttac tgatactggt tgcttttgga acctgttgtt tccagatgct gcataaaagt 660
   aaaggaagaa caaaaactag tccaaaccag tctacactgt ggatttcaaa gagtaccaga 720
   aaagaaagtg gcatggaagt ataataactc attgacttgg ttccagaatt ttgtaattct 780
   ggatctgtat aaggaatggc atcagaacaa tagcttggaa tggcttgaaa tcacaaagga 840
   tctgcaagat gaactgtaag ctcccccttg aggcaaatat taaagtaatt tttatatgtc 900
   tattatttca tttaaagaat atgctgtgct aataatggag tgagacatgc ttattttgct 960
   aaaggatgca cccaaacttc aaacttcaag caaatgaaat ggacaatgca gataaagttg 1020
   ttatcaacac gtcgggagta tgtgtgttag aagcaattcc ttttatttct ttcacctttc 1080
   ataagttgtt atctagtcaa tgtaatgtat attgtattga aatttacagt gtgcaaaagt 1140
   attttacctt tgcataagtg tttgataaaa atgaactgtt ctaatattta tttttatggc 1200
   atctcatttt tcaatacatg ctcttttgat taaagaaact tattactgtt gtcaactgaa 1260
   ttcacacaca cacaaatata gtaccataga aaaagtttgt tttctcgaaa taattcatct 1320
   ttcagcttct ctgcttttgg tcaatgtcta ggaaatctct tcagaaataa gaagctattt 1380
   cattaagtgt gatataaacc tcctcaaaca ttttacttag aggcaaggat tgtctaattt 1440
   caattgtgca agacatgtgc cttataatta tttttagctt aaaattaaac agattttgta 1500
   ataatgtaac tttgttaata ggtgcataaa cactaatgca gtcaatttga acaaaagaag 1560
   tgacatacac aatataaatc atatgtcttc acacgttgcc tatataatga gaagcagctc 1620
   tctgagggtt ctgaaatcaa tgtggtccct ctcttgccca ctaaacaaag atggttgttc 1680
   ggggtttggg attgacactg gaggcagata gttgcaaagt tagtctaagg tttccctagc 1740
   tgtatttagc ctctgactat attagtatac aaagaggtca tgtggttgag accaggtgaa 1800
   tagtcactat cagtgtggag acaagcacag cacacagaca ttttaggaag gaaaggaact 1860
   acgaaatcgt gtgaaaatgg gttggaaccc atcagtgatc gcatattcat tgatgagggt 1920
   ttgcttgaga tagaaaatgg tggctccttt ctgtcttatc tcctagtttc ttcaatgctt 1980
   acgccttgtt cttctcaaga gaaagttgta actctctggt cttcatatgt ccctgtgctc 2040
   cttttaacca aataaagagt tcttgtttct gaagaa                          2076
<210>- 74
   <211> 2133
   <212> DNA
   <213> Homo sapiens
<400> 74
<210> 75
   <211> 236
   <212> PRT
   <213> Homo sapiens
<400> 75
   Met Ala Tyr Phe His Glu Leu Ser Ser Arg Val Ser Phe Gln Glu Ala
   1               5                   10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala Val Lys Glu Glu Gln Lys
                   165                 170                 175
   Leu Val Gln Thr Ser Leu His Cys Gly Phe Gln Arg Val Pro Glu Lys
               180                 185                 190
   Lys Val Ala Trp Lys Tyr Asn Asn Ser Leu Thr Trp Phe Gln Asn Phe
           195                 200                 205
   Val Ile Leu Asp Leu Tyr Lys Glu Trp His Gln Asn Asn Ser Leu Glu
       210                 215                 220
   Trp Leu Glu Ile Thr Lys Asp Leu Gln Asp Glu Leu
   225                 230                 235
<210> 76
   <211> 232
   <212> PRT
   <213> Homo sapiens
<400> 76
   Met Ala Tyr Phe His Glu Leu Ser Ser Arg Val Ser Phe Gln Glu Ala
   1               5                   10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala Gly Ile Ile Pro Asn Leu
                   165                 170                 175
   Ile Tyr Val Val Ile Pro Thr Ile Pro Leu Leu Leu Leu Ile Leu Val
               180                 185                 190
   Ala Phe Gly Thr Cys Cys Phe Gln Met Leu His Lys Ser Lys Gly Arg
           195                 200                 205
   Thr Lys Thr Ser Pro Asn Gln Ser Thr Leu Trp Ile Ser Lys Ser Thr
       210                 215                 220
   Arg Lys Glu Ser Gly Met Glu Val
   225                 230
<210> 77
   <211> 236
   <212> PRT
   <213> Homo sapiens
<400> 77
   Met Ala Tyr Phe His Glu Leu Ser Ser Arg Val Ser Phe Gln Glu Ala
   1 5 10 15
   Arg Leu Ala Cys Glu Ser Glu Gly Gly Val Leu Leu Ser Leu Glu Asn
   20 25 30
   Glu Ala Glu Gln Lys Leu Ile Glu Ser Met Leu Gln Asn Leu Thr Lys
   35 40 45
   Pro Gly Thr Gly Ile Ser Asp Gly Asp Phe Trp Ile Gly Leu Trp Arg
   50 55 60
   Asn Gly Asp Gly Gln Thr Ser Gly Ala Cys Pro Asp Leu Tyr Gln Trp
   65 70 75 80
   Ser Asp Gly Ser Asn Ser Gln Tyr Arg Asn Trp Tyr Thr Asp Glu Pro
   85 90 95
   Ser Cys Gly Ser Glu Lys Cys Val Val Met Tyr His Gln Pro Thr Ala
   100 105 110
   Asn Pro Gly Leu Gly Gly Pro Tyr Leu Tyr Gln Trp Asn Asp Asp Arg
   115 120 125
   Cys Asn Met Lys His Asn Tyr Ile Cys Lys Tyr Glu Pro Glu Ile Asn
   130 135 140
   Pro Thr Ala Pro Val Glu Lys Pro Tyr Leu Thr Asn Gln Pro Gly Asp
   145 150 155 160
   Thr His Gln Asn Val Val Val Thr Glu Ala Val Lys Glu Glu Gln Lys
   165 170 175
   Leu Val Gln Thr Ser Leu His Cys Gly Phe Gln Arg Val Pro Glu Lys
   180 185 190
   Lys Val Ala Trp Lys Tyr Asn Asn Ser Leu Thr Trp Phe Gln Asn Phe
   195 200 205
   Val Ile Leu Asp Leu Tyr Lys Glu Trp His Gln Asn Asn Ser Leu Glu
   210 215 220
   Trp Leu Glu Ile Thr Lys Asp Leu Gln Asp Glu Leu
   225 230 235
<210> 78
   <211> 2033
   <212> DNA
   <213> Homo sapiens
<400> 78
   gcttgaagat ggcagatcgt gggacttctc agcctccata attgagtgag ccaattccct 60
   ggccaaaagg tgtgttttgc tgacttcaag catccctgct acaaaatggc ctacttccat 120
   gaactgtcca gccgagtgag ctttcaggag gcacgcctgg cttgtgagag tgagggagga 180
   gtcctcctca gccttgagaa tgaagcagaa cagaagttaa tagagagcat gttgcaaaac 240
   ctgacaaaac ccgggacagg gatttctgat ggtgatttct ggatagggct ttggaggaat 300
   ggagatgggc aaacatctgg tgcctgccca gatctctacc agtggtctga tggaagcaat 360
   tcccagtacc gaaactggta cacagatgaa ccttcctgcg gaagtgaaaa gtgtgttgtg 420
   atgtatcacc aaccaactgc caatcctggc cttgggggtc cctaccttta ccagtggaat 480
   gatgacaggt gtaacatgaa gcacaattat atttgcaagt atgaaccaga gattaatcca 540
   acagcccctg tagaaaagcc ttatcttaca aatcaaccag gagacaccca tcagaatgtg 600
   gttgttactg aagcagtaaa ggaagaacaa aaactagtcc aaaccagtct acactgtgga 660
   tttcaaagag taccagaaaa gaaagtggca tggaagtata ataactcatt gacttggttc 720
   cagaattttg taattctgga tctgtataag gaatggcatc agaacaatag cttggaatgg 780
   cttgaaatca caaaggatct gcaagatgaa ctgtaagctc ccccttgagg caaatattaa 840
   agtaattttt atatgtctat tatttcattt aaagaatatg ctgtgctaat aatggagtga 900
   gacatgctta ttttgctaaa ggatgcaccc aaacttcaaa cttcaagcaa atgaaatgga 960
   caatgcagat aaagttgtta tcaacacgtc gggagtatgt gtgttagaag caattccttt 1020
   tatttctttc acctttcata agttgttatc tagtcaatgt aatgtatatt gtattgaaat 1080
   ttacagtgtg caaaagtatt ttacctttgc ataagtgttt gataaaaatg aactgttcta 1140
   atatttattt ttatggcatc tcatttttca atacatgctc ttttgattaa agaaacttat 1200
   tactgttgtc aactgaattc acacacacac aaatatagta ccatagaaaa agtttgtttt 1260
   ctcgaaataa ttcatctttc agcttctctg cttttggtca atgtctagga aatctcttca 1320
   gaaataagaa gctatttcat taagtgtgat ataaacctcc tcaaacattt tacttagagg 1380
   caaggattgt ctaatttcaa ttgtgcaaga catgtgcctt ataattattt ttagcttaaa 1440
   attaaacaga ttttgtaata atgtaacttt gttaataggt gcataaacac taatgcagtc 1500
   aatttgaaca aaagaagtga catacacaat ataaatcata tgtcttcaca cgttgcctat 1560
   ataatgagaa gcagctctct gagggttctg aaatcaatgt ggtccctctc ttgcccacta 1620
   aacaaagatg gttgttcggg gtttgggatt gacactggag gcagatagtt gcaaagttag 1680
   tctaaggttt ccctagctgt atttagcctc tgactatatt agtatacaaa gaggtcatgt 1740
   ggttgagacc aggtgaatag tcactatcag tgtggagaca agcacagcac acagacattt 1800
   taggaaggaa aggaactacg aaatcgtgtg aaaatgggtt ggaacccatc agtgatcgca 1860
   tattcattga tgagggtttg cttgagatag aaaatggtgg ctcctttctg tcttatctcc 1920
   tagtttcttc aatgcttacg ccttgttctt ctcaagagaa agttgtaact ctctggtctt 1980
   catatgtccc tgtgctcctt ttaaccaaat aaagagttct tgtttctgaa gaa        2033
<210> 79
   <211> 2076
   <212> DNA
   <213> Homo sapiens
<400> 79
   ggccaaaagg tgtgttttgc tgacttcaag catccctgct acaaaatggc ctacttccat 60
   gaactgtcca gccgagtgag ctttcaggag gcacgcctgg cttgtgagag tgagggagga 120
   gtcctcctca gccttgagaa tgaagcagaa cagaagttaa tagagagcat gttgcaaaac 180
   ctgacaaaac ccgggacagg gatttctgat ggtgatttct ggatagggct ttggaggaat 240
   ggagatgggc aaacatctgg tgcctgccca gatctctacc agtggtctga tggaagcaat 300
   tcccagtacc gaaactggta cacagatgaa ccttcctgcg gaagtgaaaa gtgtgttgtg 360
   atgtatcacc aaccaactgc caatcctggc cttgggggtc cctaccttta ccagtggaat 420
   gatgacaggt gtaacatgaa gcacaattat atttgcaagt atgaaccaga gattaatcca 480
   acagcccctg tagaaaagcc ttatcttaca aatcaaccag gagacaccca tcagaatgtg 540
   gttgttactg aagcaggtat aattcccaat ctaatttatg ttgttatacc aacaataccc 600
   ctgctcttac tgatactggt tgcttttgga acctgttgtt tccagatgct gcataaaagt 660
   aaaggaagaa caaaaactag tccaaaccag tctacactgt ggatttcaaa gagtaccaga 720
   aaagaaagtg gcatggaagt ataataactc attgacttgg ttccagaatt ttgtaattct 780
   ggatctgtat aaggaatggc atcagaacaa tagcttggaa tggcttgaaa tcacaaagga 840
   tctgcaagat gaactgtaag ctcccccttg aggcaaatat taaagtaatt tttatatgtc 900
   tattatttca tttaaagaat atgctgtgct aataatggag tgagacatgc ttattttgct 960
   aaaggatgca cccaaacttc aaacttcaag caaatgaaat ggacaatgca gataaagttg 1020
   ttatcaacac gtcgggagta tgtgtgttag aagcaattcc ttttatttct ttcacctttc 1080
   ataagttgtt atctagtcaa tgtaatgtat attgtattga aatttacagt gtgcaaaagt 1140
   attttacctt tgcataagtg tttgataaaa atgaactgtt ctaatattta tttttatggc 1200
   atctcatttt tcaatacatg ctcttttgat taaagaaact tattactgtt gtcaactgaa 1260
   ttcacacaca cacaaatata gtaccataga aaaagtttgt tttctcgaaa taattcatct 1320
   ttcagcttct ctgcttttgg tcaatgtcta ggaaatctct tcagaaataa gaagctattt 1380
   cattaagtgt gatataaacc tcctcaaaca ttttacttag aggcaaggat tgtctaattt 1440
   caattgtgca agacatgtgc cttataatta tttttagctt aaaattaaac agattttgta 1500
   ataatgtaac tttgttaata ggtgcataaa cactaatgca gtcaatttga acaaaagaag 1560
   tgacatacac aatataaatc atatgtcttc acacgttgcc tatataatga gaagcagctc 1620
   tctgagggtt ctgaaatcaa tgtggtccct ctcttgccca ctaaacaaag atggttgttc 1680
   ggggtttggg attgacactg gaggcagata gttgcaaagt tagtctaagg tttccctagc 1740
   tgtatttagc ctctgactat attagtatac aaagaggtca tgtggttgag accaggtgaa 1800
   tagtcactat cagtgtggag acaagcacag cacacagaca ttttaggaag gaaaggaact 1860
   acgaaatcgt gtgaaaatgg gttggaaccc atcagtgatc gcatattcat tgatgagggt 1920
   ttgcttgaga tagaaaatgg tggctccttt ctgtcttatc tcctagtttc ttcaatgctt 1980
   acgccttgtt cttctcaaga gaaagttgta actctctggt cttcatatgt ccctgtgctc 2040
   cttttaacca aataaagagt tcttgtttct gaagaa                          2076
<210> 80
   <211> 2033
   <212> DNA
   <213> Homo sapiens
<400> 80
   gcttgaagat ggcagatcgt gggacttctc agcctccata attgagtgag ccaattccct 60
   ggccaaaagg tgtgttttgc tgacttcaag catccctgct acaaaatggc ctacttccat 120
   gaactgtcca gccgagtgag ctttcaggag gcacgcctgg cttgtgagag tgagggagga 180
   gtcctcctca gccttgagaa tgaagcagaa cagaagttaa tagagagcat gttgcaaaac 240
   ctgacaaaac ccgggacagg gatttctgat ggtgatttct ggatagggct ttggaggaat 300
   ggagatgggc aaacatctgg tgcctgccca gatctctacc agtggtctga tggaagcaat 360
   tcccagtacc gaaactggta cacagatgaa ccttcctgcg gaagtgaaaa gtgtgttgtg 420
   atgtatcacc aaccaactgc caatcctggc cttgggggtc cctaccttta ccagtggaat 480
   gatgacaggt gtaacatgaa gcacaattat atttgcaagt atgaaccaga gattaatcca 540
   acagcccctg tagaaaagcc ttatcttaca aatcaaccag gagacaccca tcagaatgtg 600
   gttgttactg aagcagtaaa ggaagaacaa aaactagtcc aaaccagtct acactgtgga 660
   tttcaaagag taccagaaaa gaaagtggca tggaagtata ataactcatt gacttggttc 720
   cagaattttg taattctgga tctgtataag gaatggcatc agaacaatag cttggaatgg 780
   cttgaaatca caaaggatct gcaagatgaa ctgtaagctc ccccttgagg caaatattaa 840
   agtaattttt atatgtctat tatttcattt aaagaatatg ctgtgctaat aatggagtga 900
   gacatgctta ttttgctaaa ggatgcaccc aaacttcaaa cttcaagcaa atgaaatgga 960
   caatgcagat aaagttgtta tcaacacgtc gggagtatgt gtgttagaag caattccttt 1020
   tatttctttc acctttcata agttgttatc tagtcaatgt aatgtatatt gtattgaaat 1080
   ttacagtgtg caaaagtatt ttacctttgc ataagtgttt gataaaaatg aactgttcta 1140
   atatttattt ttatggcatc tcatttttca atacatgctc ttttgattaa agaaacttat 1200
   tactgttgtc aactgaattc acacacacac aaatatagta ccatagaaaa agtttgtttt 1260
   ctcgaaataa ttcatctttc agcttctctg cttttggtca atgtctagga aatctcttca 1320
   gaaataagaa gctatttcat taagtgtgat ataaacctcc tcaaacattt tacttagagg 1380
   caaggattgt ctaatttcaa ttgtgcaaga catgtgcctt ataattattt ttagcttaaa 1440
   attaaacaga ttttgtaata atgtaacttt gttaataggt gcataaacac taatgcagtc 1500
   aatttgaaca aaagaagtga catacacaat ataaatcata tgtcttcaca cgttgcctat 1560
   ataatgagaa gcagctctct gagggttctg aaatcaatgt ggtccctctc ttgcccacta 1620
   aacaaagatg gttgttcggg gtttgggatt gacactggag gcagatagtt gcaaagttag 1680
   tctaaggttt ccctagctgt atttagcctc tgactatatt agtatacaaa gaggtcatgt 1740
   ggttgagacc aggtgaatag tcactatcag tgtggagaca agcacagcac acagacattt 1800
   taggaaggaa aggaactacg aaatcgtgtg aaaatgggtt ggaacccatc agtgatcgca 1860
   tattcattga tgagggtttg cttgagatag aaaatggtgg ctcctttctg tcttatctcc 1920
   tagtttcttc aatgcttacg ccttgttctt ctcaagagaa agttgtaact ctctggtctt 1980
   catatgtccc tgtgctcctt ttaaccaaat aaagagttct tgtttctgaa gaa        2033
<210> 81
   <211> 236
   <212> PRT
   <213> Homo sapiens
<400> 81
   Met Ala Tyr Phe His Glu Leu Ser Ser Arg Val Ser Phe Gln Glu Ala
    1               5                  10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala Val Lys Glu Glu Gln Lys
                   165                 170                 175
   Leu Val Gln Thr Ser Leu His Cys Gly Phe Gln Arg Val Pro Glu Lys
               180                 185                 190
   Lys Val Ala Trp Lys Tyr Asn Asn Ser Leu Thr Trp Phe Gln Asn Phe
           195                 200                 205
   Val Ile Leu Asp Leu Tyr Lys Glu Trp His Gln Asn Asn Ser Leu Glu
       210                 215                 220
   Trp Leu Glu Ile Thr Lys Asp Leu Gln Asp Glu Leu
   225                 230                 235
<210> 82
   <211> 170
   <212> PRT
   <213> Homo sapiens
<400> 82
   Met Ala Tyr Phe His Glu Leu Ser Ser Arg Val Ser Phe Gln Glu Ala
    1               5                  10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala
                   165                 170
<210> 83
   <211> 236
   <212> PRT
   <213> Homo sapiens
<400> 83
   Met Ala Tyr Phe His Glu Leu Ser Ser Arg Val Ser Phe Gln Glu Ala
   1               5                   10                  15
   Arg Leu Ala Cys Glu Ser Glu Gly Gly Val Leu Leu Ser Leu Glu Asn
               20                  25                  30
   Glu Ala Glu Gln Lys Leu Ile Glu Ser Met Leu Gln Asn Leu Thr Lys
           35                  40                  45
   Pro Gly Thr Gly Ile Ser Asp Gly Asp Phe Trp Ile Gly Leu Trp Arg
       50                  55                  60
   Asn Gly Asp Gly Gln Thr Ser Gly Ala Cys Pro Asp Leu Tyr Gln Trp
   65                  70                  75                  80
   Ser Asp Gly Ser Asn Ser Gln Tyr Arg Asn Trp Tyr Thr Asp Glu Pro
                   85                  90                  95
   Ser Cys Gly Ser Glu Lys Cys Val Val Met Tyr His Gln Pro Thr Ala
               100                 105                 110
   Asn Pro Gly Leu Gly Gly Pro Tyr Leu Tyr Gln Trp Asn Asp Asp Arg
           115                 120                 125
   Cys Asn Met Lys His Asn Tyr Ile Cys Lys Tyr Glu Pro Glu Ile Asn
       130                 135                 140
   Pro Thr Ala Pro Val Glu Lys Pro Tyr Leu Thr Asn Gln Pro Gly Asp
   145                 150                 155                 160
   Thr His Gln Asn Val Val Val Thr Glu Ala Val Lys Glu Glu Gln Lys
                   165                 170                 175
   Leu Val Gln Thr Ser Leu His Cys Gly Phe Gln Arg Val Pro Glu Lys
               180                 185                 190
   Lys Val Ala Trp Lys Tyr Asn Asn Ser Leu Thr Trp Phe Gln Asn Phe
           195                 200                 205
   Val Ile Leu Asp Leu Tyr Lys Glu Trp His Gln Asn Asn Ser Leu Glu
       210                 215                 220
   Trp Leu Glu Ile Thr Lys Asp Leu Gln Asp Glu Leu
   225                 230                 235

## Description

### Statement of Rights to Inventions Made Under Federally Sponsored Research

Not applicable.

### Reference to Sequence Listing Submitted on Compact Disc

Seq ID 1-83

### Reference to Compact Disc Appendix

### Field of the Invention

The invention described herein relates to methods useful in the management of cancers that express the gene termed 58P1D12.

### Background of the Invention

Cancer is the second leading cause of human death next to coronary disease. Worldwide, millions of people die from cancer every year. In the United States alone, as reported by the American Cancer Society, cancer causes the death of well over a half-million people annually, with over 1.2 million new cases diagnosed per year. While deaths from heart disease have been declining significantly, those resulting from cancer generally are on the rise. In the early part of the next century, cancer is predicted to become the leading cause of death.

Worldwide, several cancers stand out as the leading killers. In particular, carcinomas of the lung, prostate, breast colon, pancreas, and ovary represent the primary causes of cancer death. These and virtually all other carcinomas share a common lethal feature. With very few exceptions, metastatic disease from a carcinoma is fatal. Moreover, even for those cancer patients who initially survive their primary cancers, common experience has shown that their lives are dramatically altered. Many cancer patients experience strong anxieties driven by the awareness of the potential for recurrence or treatment failure. Many cancer patients experience physical debilitations following treatment Furthermore, many cancer patients experience a recurrence.

Worldwide, prostate cancer is the fourth most prevalent cancer in men. In North America and Northern Europe, it is by far the most common cancer in males and is the second leading cause of cancer death in men. In the United States alone, well over 30,000 men die annually of this disease - second only to lung cancer. Despite the magnitude of these figures, there is still no effective treatment for metastatic prostate cancer. Surgical prostatectomy, radiation therapy, hormone ablation therapy, surgical castration and chemotherapy continue to be the main treatment modalities. Unfortunately, these treatments are ineffective for many and are often associated with undesirable consequences.

On the diagnostic front, the lack of a prostate tumor marker that can accurately detect early-stage, localized tumors remains a significant limitation in the diagnosis and management of this disease. Although the serum prostate specific antigen (PSA) assay has been a very useful tool, however its specificity and general utility is widely regarded as lacking in several important respects.

Progress in identifying additional specific markers for prostate cancer has been improved by the generation of prostate cancer xenografts that can recapitulate different stages of the disease in mice. The LAPC (Los Angeles Prostate Cancer) xenografts are prostate cancer xenografts that have survived passage in severe combined immune deficient (SCID) mice and have exhibited the capacity to mimic the transition from androgen dependence to androgen independence (Klein et al., 1997, Nat. Med. 3:402). More recently identified prostate cancer markers include PCTA-1 (Su et al.,1996, Proc. Natl. Acad. Sci. USA 93: 7252), prostate-specific membrane (PSM) antigen (Pinto et al., Clin Cancer Res 1996 Sep 2 (9): 1445-51). STEAP (Hubert, et al., Proc Natl Acad Sci U S A.1999 Dec 7; 96(25): 14523-8) and prostate stem cell antigen (PSCA) (Reiter et al., 1998, Proc. Natl. Acad. Sci. USA 95:1735).

While previously identified markers such as PSA, PSM, PCTA and PSCA have facilitated efforts to diagnose and treat prostate cancer, there is need for the identification of additional markers and therapeutic targets for prostate and related cancers in order to further improve diagnosis and therapy.

Renal cell carcinoma (RCC) accounts for approximately 3 percent of adult malignancies. Once adenomas reach a diameter of 2 to 3 cm, malignant potential exists. In the adult, the two principal malignant renal tumors are renal cell adenocarcinoma and transitional cell carcinoma of the renal pelvis or urethras. The incidence of renal cell adenocarcinoma is estimated at more than 29,000 cases in the United States, and more than 11,600 patients died of this disease in 1998. Transitional cell carcinoma is less frequent, with an incidence of approximately 500 cases per year in the United States.

Surgery has been the primary therapy for renal cell adenocarcinoma for many decades. Until recently, metastatic disease has been refractory to any systemic therapy. With recent developments in systemic therapies, particularly immunotherapies, metastatic renal cell carcinoma may be approached aggressively in appropriate patients with a possibility of durable responses. Nevertheless, there is a remaining need for effective therapies for these patients.

Of all new cases of cancer in the United States, bladder cancer represents approximately 5 percent in men (fifth most common neoplasm) and 3 percent in women (eighth most common neoplasm). The incidence is increasing slowly, concurrent with an increasing older population. In 1998, there was an estimated 54,500 cases, including 39,500 in men and 15,000 in women. The age-adjusted incidence in the United States is 32 per 100,000 for men and eight per 100,000 in women. The historic male/female ratio of 3:1 may be decreasing related to smoking patterns in women. There were an estimated 11,000 deaths from bladder cancer in 1998 (7,800 In men and 3,900 in women). Bladder cancer incidence and mortality strongly increase with age and will be an increasing problem as the population becomes more elderly.

Most bladder cancers recur in the bladder. Bladder cancer is managed with a combination of transurethral resection of the bladder (TUR) and Intravesical chemotherapy or immunotherapy. The multifocal and recurrent nature of bladder cancer points out the limitations of TUR. Most muscle-invasive cancers are not cured by TUR alone. Radical cystectomy and urinary diversion is the most effective means to eliminate the cancer but carry an undeniable impact on urinary and sexual function. There continues to be a significant need for treatment modalities that are beneficial for bladder cancer patients.

An estimated 130,200 cases of colorectal cancer occurred in 2000 in the United States, including 93,800 cases of colon cancer and 36,400 of rectal cancer. Colorectal cancers are the third most common cancers in men and women. Incidence rates declined significantly during 1992-1996 (-2.1 % per year). Research suggests that these declines have been due to increased screening and polyp removal, preventing progression of polyps to invasive cancers. There were an estimated 56,300 deaths (47,700 from colon cancer, 8,600 from rectal cancer) in 2000, accounting for about 11 % of all U.S. cancer deaths.

At present, surgery is the most common form of therapy for colorectal cancer, and for cancers that have not spread, it is frequently curative. Chemotherapy, or chemotherapy plus radiation, is given before or after surgery to most patients whose cancer has deeply perforated the bowel wall or has spread to the lymph nodes. A permanent colostomy (creation of an abdominal opening for elimination of body wastes) is occasionally needed for colon cancer and is infrequently required for rectal cancer. There continues to be a need for effective diagnostic and treatment modalities for colorectal cancer.

There were an estimated 164,100 new cases of lung and bronchial cancer in 2000, accounting for 14% of all U.S. cancer diagnoses. The incidence rate of lung and bronchial cancer is declining significantly in men, from a high of 86.5 per 100,000 in 1984 to 70.0 in 1996. In the 1990s, the rate of increase among women began to slow. In 1996, the incidence rate in women was 42.3 per 100,000.

Lung and bronchial cancer caused an estimated 156,900 deaths in 2000, accounting for 28% of all cancer deaths. During 1992-1996, mortality from lung cancer declined significantly among men (-1.7% per year) while rates for women were still significantly increasing (0.9% per year). Since 1987, more women have died each year of lung cancer than breast cancer, which, for over 40 years, was the major cause of cancer death In women. Decreasing lung cancer incidence and mortality rates most likely resulted from decreased smoking rates over the previous 30 years; however, decreasing smoking patterns among women lag behind those of men. Of concern, although the declines in adult tobacco use have slowed, tobacco use in youth is increasing again.

Treatment options for lung and bronchial cancer are determined by the type and stage of the cancer and include surgery, radiation therapy, and chemotherapy. For many localized cancers, surgery is usually the treatment of choice. Because the disease has usually spread by the time it is discovered, radiation therapy and chemotherapy are often needed in combination with surgery. Chemotherapy alone or combined with radiation is the treatment of choice for small cell lung cancer; on this regimen, a large percentage of patients experience remission, which in some cases is long lasting. There is however, an ongoing need for effective treatment and diagnostic approaches for lung and bronchial cancers.

An estimated 182,800 new invasive cases of breast cancer were expected to occur among women in the United States during 2000. Additionally, about 1,400 new cases of breast cancer were expected to be diagnosed in men in 2000. After increasing about 4% per year in the 1980s, breast cancer incidence rates in women have leveled off in the 1990s to about 110.6 cases per 100,000.

In the U.S. alone, there were an estimated 41,200 deaths (40,800 women, 400 men) in 2000 due to breast cancer. Breast cancer ranks second among cancer deaths in women. According to the most recent data, mortality rates declined significantly during 1992-1996 with the largest decreases in younger women, both white and black. These decreases were probably the result of earlier detection and improved treatment.

Taking into account the medical circumstances and the patient's preferences, treatment of breast cancer may involve lumpectomy (local removal of the tumor) and removal of the lymph nodes under the arm; mastectomy (surgical removal of the breast) and removal of the lymph nodes under the arm; radiation therapy; chemotherapy; or hormone therapy. Often, two or more methods are used in combination. Numerous studies have shown that, for early stage disease, long-term survival rates after lumpectomy plus radiotherapy are similar to survival rates after modified radical mastectomy. Significant advances in reconstruction techniques provide several options for breast reconstruction after mastectomy. Recently, such reconstruction has been done at the same time as the mastectomy.

Local excision of ductal carcinoma in situ (DCIS) with adequate amounts of surrounding normal breast tissue may prevent the local recurrence of the DCIS. Radiation to the breast and/or tamoxifen may reduce the chance of DCIS occurring in the remaining breast tissue. This is important because DCIS, if left untreated, may develop into invasive breast cancer. Nevertheless, there are serious side effects or sequelae to these treatments. There is, therefore, a need for efficacious breast cancer treatments.

There were an estimated 23,100 new cases of ovarian cancer in the United States in 2000. It accounts for 4% of all cancers among women and ranks second among gynecologic cancers. During 1992-1996, ovarian cancer incidence rates were significantly declining. Consequent to ovarian cancer, there were an estimated 14,000 deaths in 2000. Ovarian cancer causes more deaths than any other cancer of the female reproductive system.

Surgery, radiation therapy, and chemotherapy are treatment options for ovarian cancer. Surgery usually includes the removal of one or both ovaries, the falloplan tubes (salpingo-cophorectomy), and the uterus (hysterectomy). In some very early tumors, only the involved ovary will be removed, especially in young women who wish to have children. In advanced disease, an attempt is made to remove all intra-abdominal disease to enhance the effect of chemotherapy. There continues to be an important need for effective treatment options for ovarian cancer.

There were an estimated 28,300 new cases of pancreatic cancer in the United States in 2000. Over the past 20 years, rates of pancreatic cancer have declined in men. Rates among women have remained approximately constant but may be beginning to decline. Pancreatic cancer caused an estimated 28,200 deaths in 2000 In the United States. Over the past 20 years, there has been a slight but significant decrease in mortality rates among men (about -0.9% per year) while rates have Increased slightly among women.

Surgery, radiation therapy, and chemotherapy are treatment options for pancreatic cancer. These treatment options can extend survival and/or relieve symptoms in many patients but are not likely to produce a cure for most There is a significant need for additional therapeutic and diagnostic options for pancreatic cancer.

### Summary of the Invention

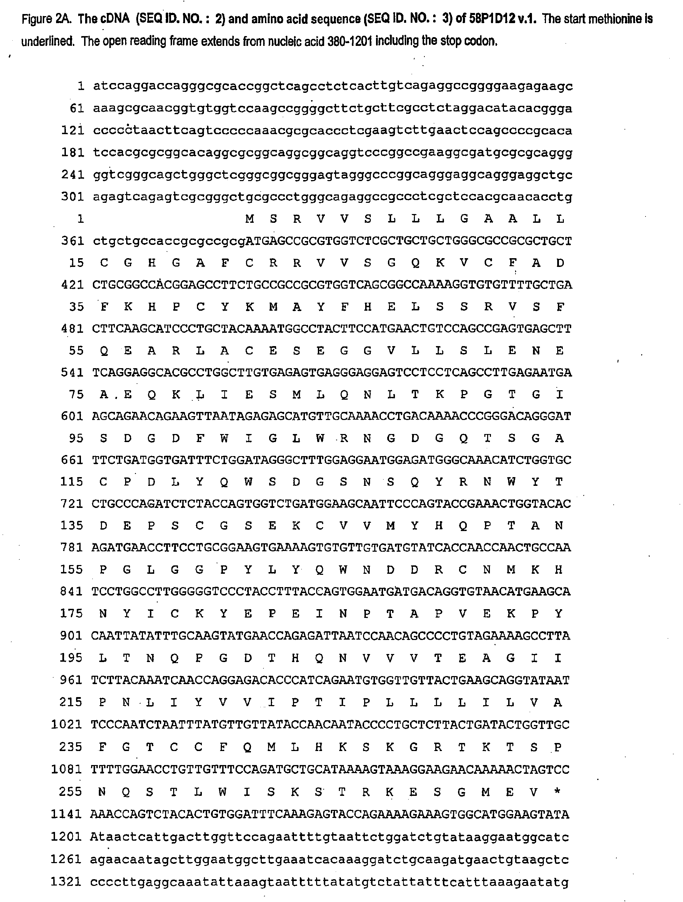

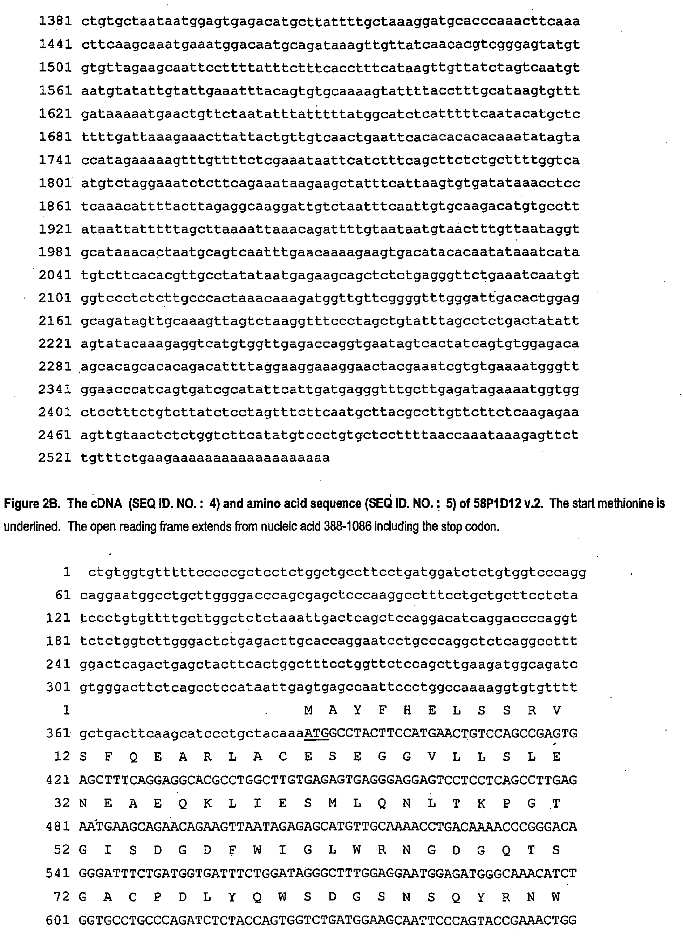

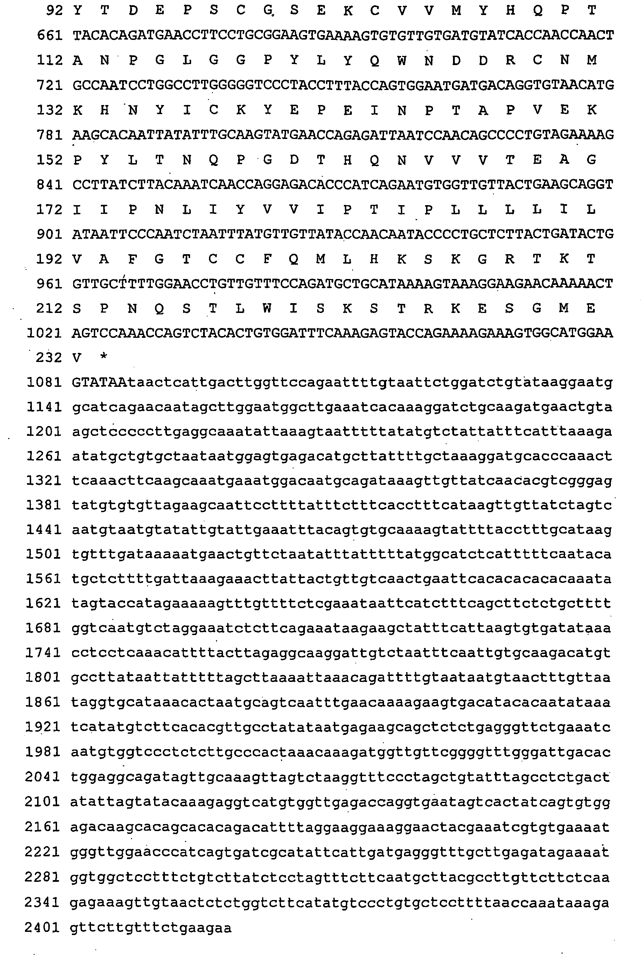

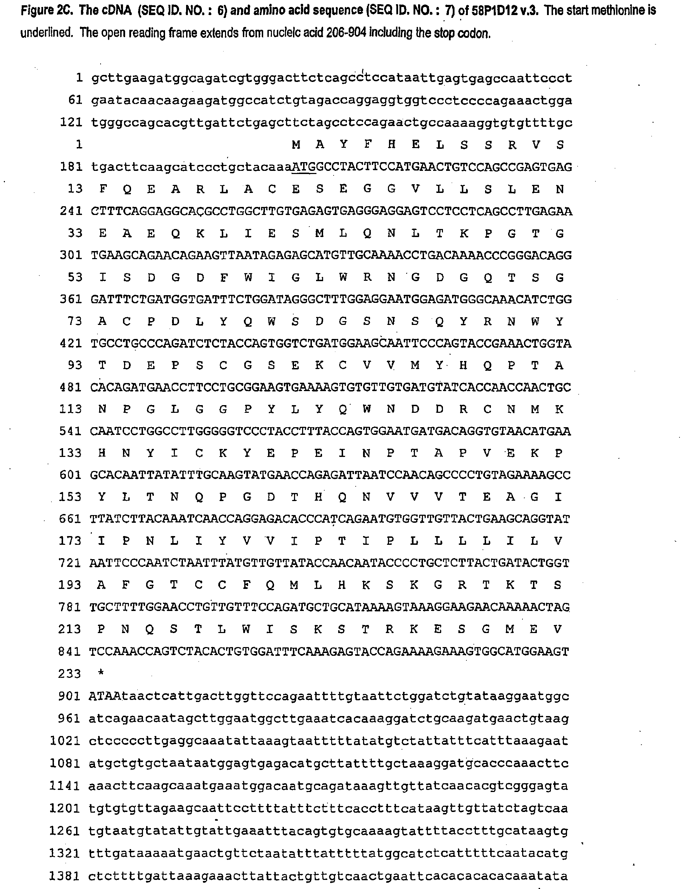

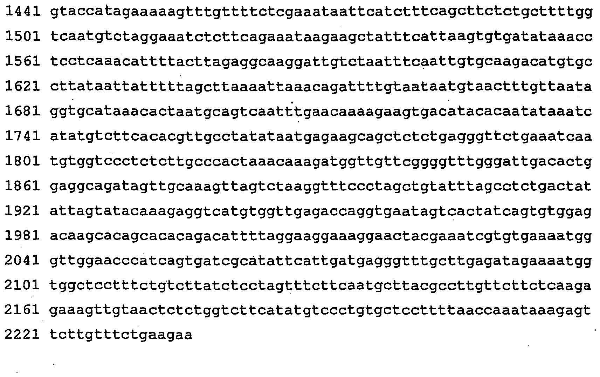

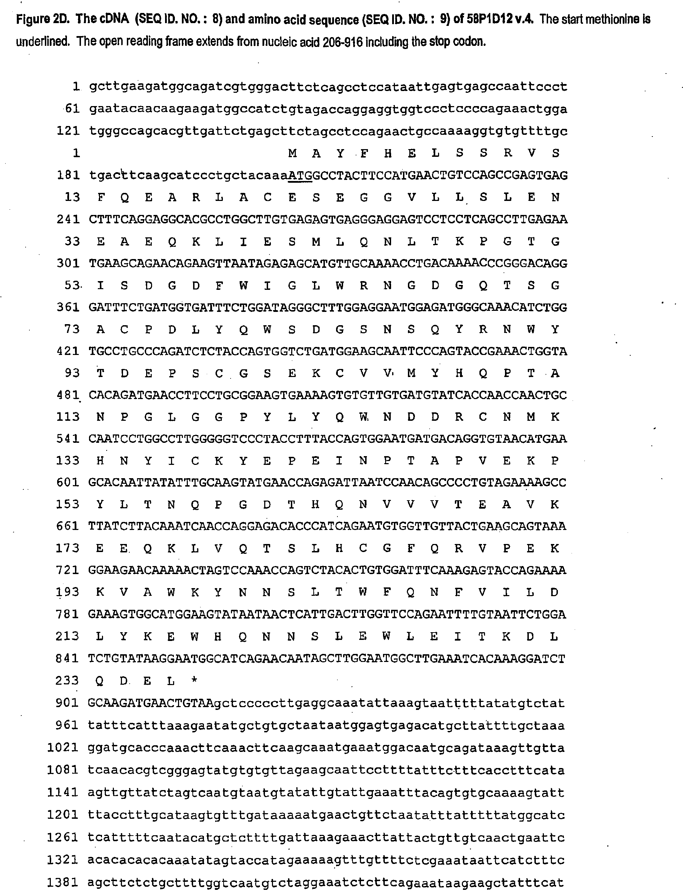

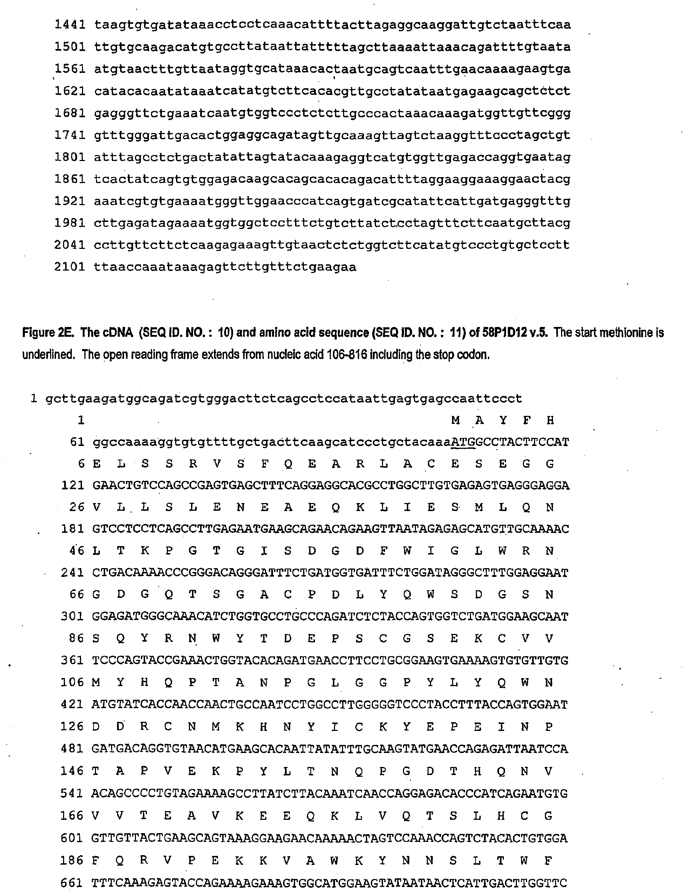

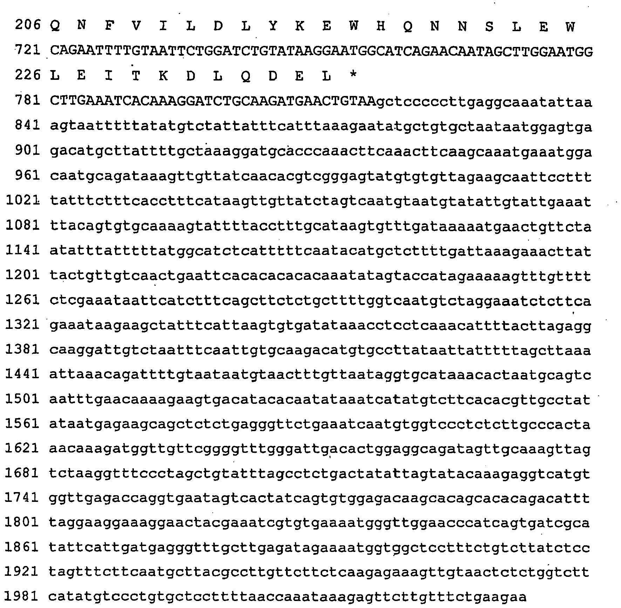

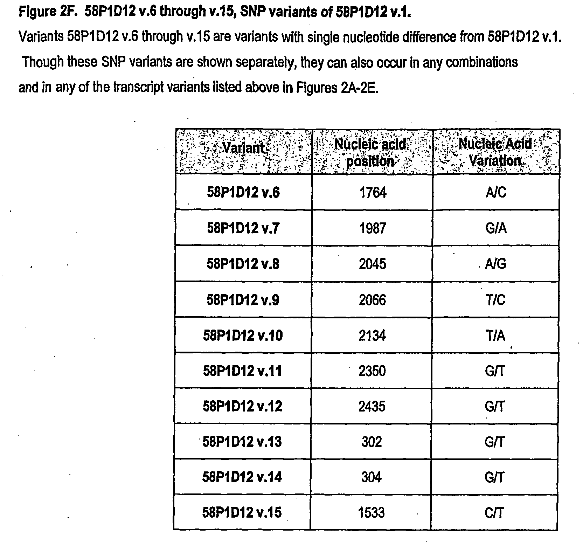

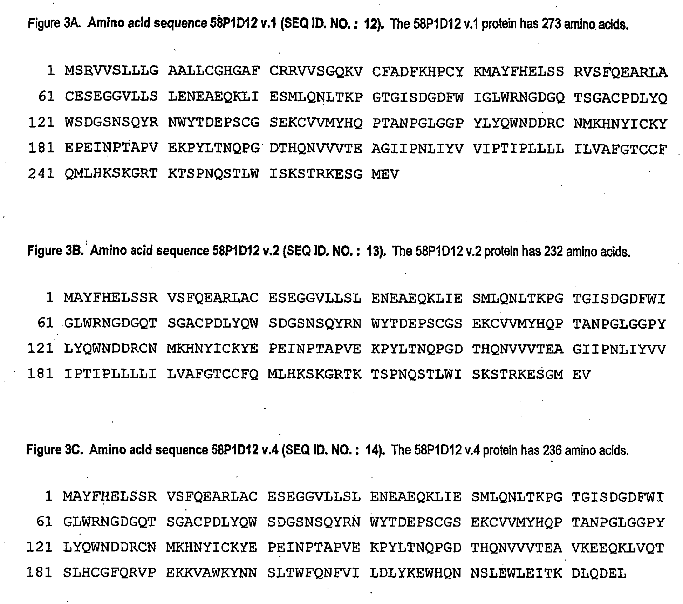

The present invention relates to methods for detecting expression levels of a gene, designated 58P1D12, that has now been found to be over-expressed in the cancer(s) listed in Table I. Northern blot expression analysis of 58P1D12 gene expression in normal tissues shows a restricted expression pattern In adult tissues. The nucleotide (Figure 2) and amino acid (Figure 2, and Figure 3) sequences of 58P1D12 are shown. The tissue-related profile of 58P1D12 in normal adult tissues, combined with the over-expression observed in the tissues listed in Table I, shows that 58P1D12 is aberrantly over-expressed in at least some cancers, and thus serves as a useful diagnostic, prophylactic, prognostic, and/or therapeutic target for cancers of the tissue(s) such as those listed in Table I.

The invention provides methods for detecting the presence and status of 58P1 D12 polynucleotides and proteins in various biological samples, as well as methods for identifying cells that express 58P1D12. A typical embodiment of this Invention provides methods for monitoring 58P1D12 gene products in a tissue or hematology sample having or suspected of having some form of growth dysregulation such as cancer.

### Brief Description of the Figures

Figure 1. The 58P1D12 SSH sequence of 427 nucleotides.

Figure 2A) The cDNA and amino acid sequence of 58P1D12 variant 1 (also called "58P1D12 v.1" or 58P1D12 variant 1") is shown in Figure 2A. The start methionine is underlined. The open reading frame extends from nucleic acid 380-1201 including the stop codon.

Figure 2B) The cDNA and amino acid sequence of 58P1D12 variant 2 (also called "58P1D12 v.2") is shown in Figure 2B. The codon for the start methionine is underlined. The open reading frame extends from nucleic acid 388-1086 including the stop codon.

Figure 2C) The cDNA and amino acid sequence of 58P1D12 variant 3 (also called "58P1D12 v.3") is shown in Figure 2C. The codon for the start methionine is underlined. The open reading frame extends from nucleic acid 206-904 including the stop codon.

Figure 2D) The cDNA and amino acid sequence of 58P1D12 variant 4 (also called "58P1D12 v.4") is shown in Figure 2D. The codon for the start methionine is underlined. The open reading frame extends from nucleic add 206-916 including the stop codon.

Figure 2E) The cDNA and amino acid sequence of 58P1D12 variant 5 (also called "58P1D12 v.5") is shown in Figure 2E. The codon for the start methionine is underlined. The open reading frame extends from nucleic acid 106-816 including the stop codon.

Variants 58P1D12 v.6 through v.15 are variants with single nucleotide difference from 58P1D12 v.1. Though these SNP variants are shown separately, they can also occur in any combinations and in any of the transcript variants listed above In Figures 2A-2F.

Figure 3A) The amino acid sequence of 58P1D12 v.1 is shown in Figure 3A; it has 273 amino acids.

Figure 3B) The amino acid sequence of 58P1D12 v.2 is shown in Figure 3B; it has 232 amino acids.

Figure 3C) The amino acid sequence of 58P1D12 v.3 is shown in Figure 3C; it has 236 amino acids.

As used herein, a reference to 58P1D12 includes all variants thereof, including those shown in Figures 2,3,10,11, and 12 unless the context clearly indicates otherwise.

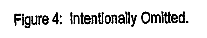

Figure 4. Intentionally Omitted.

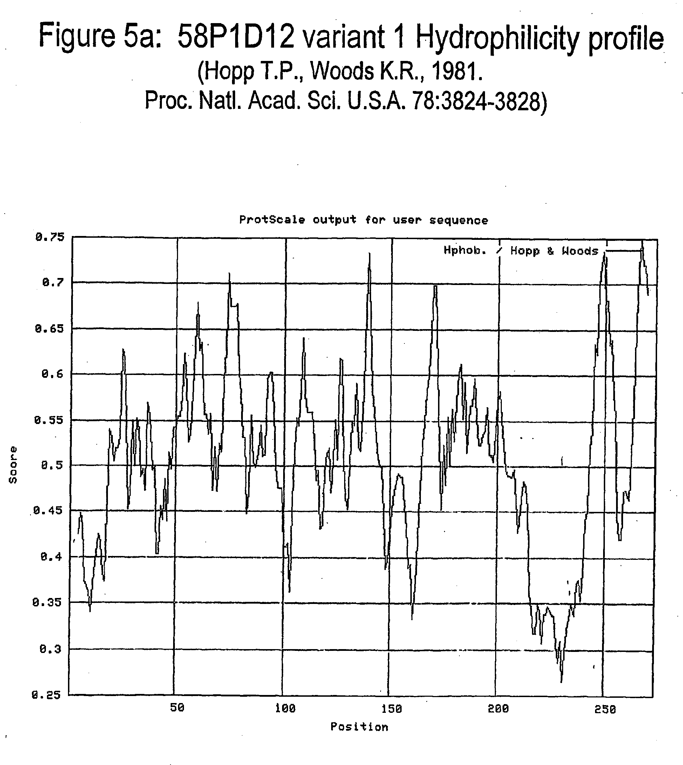

Figure 5(a) - (c). Hydrophilicity amino acid profile of 58P1D12 v.1-v.3 determined by computer algorithm sequence analysis using the method of Hopp and Woods (Hopp T.P., Woods K.R., 1981. Proc. Natl. Acad. Sci. U.SA 78:3824-3828) accessed on the Protscale website located on the World Wide Web at (expasy.ch/cgi-bin/protscale.pl) through the ExPasy molecular biology server.

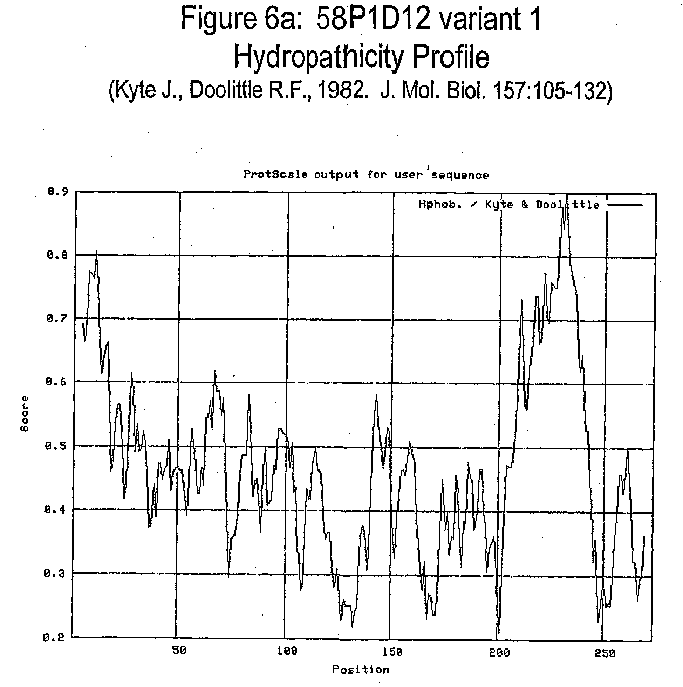

Figure 6(a) - (c). Hydropathicity amino acid profile of 58P1D12 v.1-v.9 determined by computer algorithm sequence analysis using the method of Kyte and Doolittle (Kyte J., Doolittle R.F., 1982. J. Mol. Biol. 157:105-132) accessed on the ProtScale website located on the World Wide Web at (.expasy.ch/cgi-bin/protscale.pl) through the ExPasy molecular biology server.

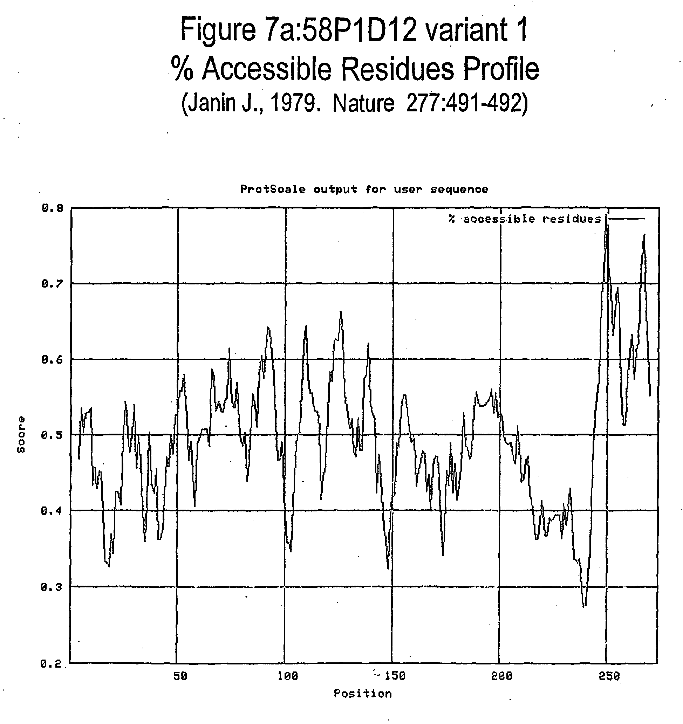

Figure 7(a) - (c). Percent accessible residues amino acid profile of 58P1D12 v.1-v.9 determined by computer algorithm sequence analysis using the method of Janin (Janin J., 1979 Nature 277:491-492) accessed on the ProtScale website located on the World Wide Web at (.expasy.ch/cgi-bin/protscale.pl) through the ExPasy molecular biology server.

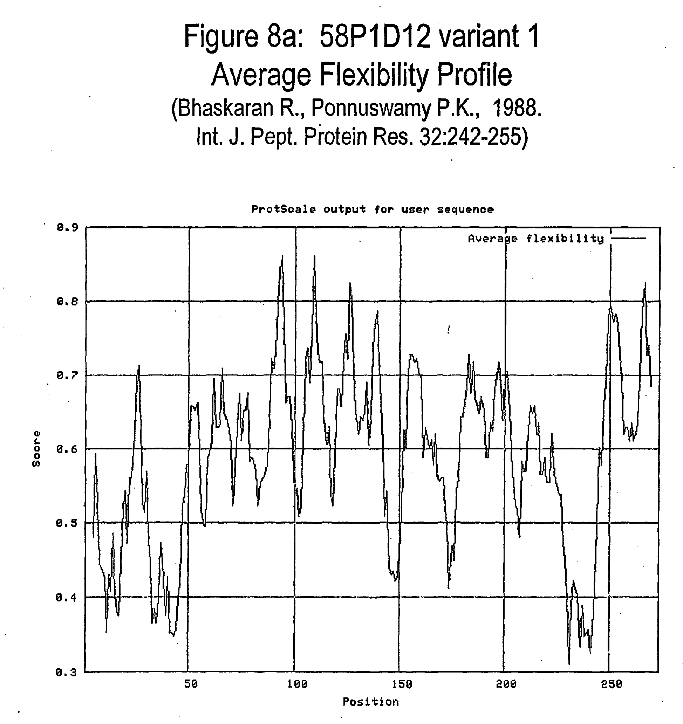

Figure 8(a) - (c). Average flexibility amino acid profile of 58P1D12 v.1-v.9 determined by computer algorithm sequence analysis using the method of Bhaskaran and Ponnuswamy (Bhaskaran R., and Ponnuswamy P.K., 1988. Int. J. Pept. Protein Res. 32:242-255) accessed on the ProtScale website located on the World Wide Web at (.expasy.ch/cgi-bin/protscale.pl) through the ExPasy molecular biology server.

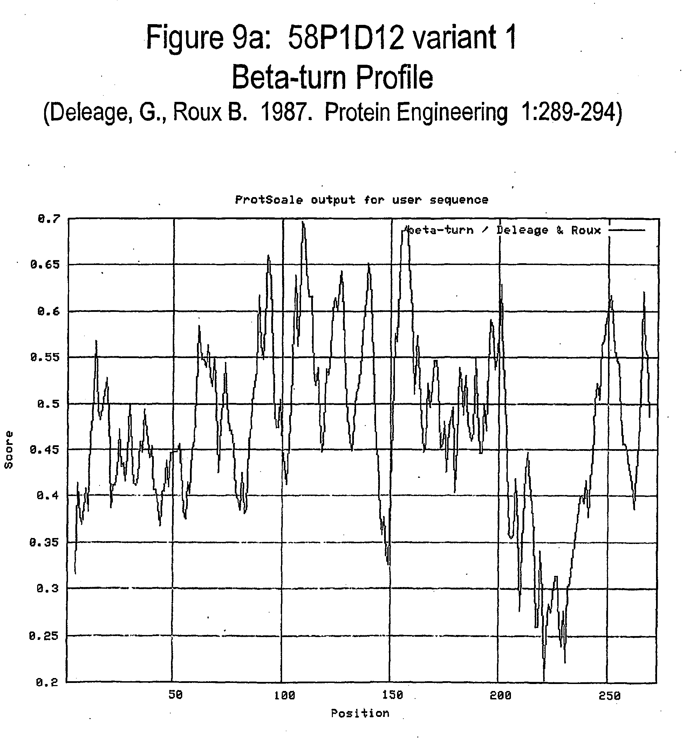

Figure 9(a) - (c). Beta-turn amino acid profile of 58P1 D12 v.1-v.9 determined by computer algorithm sequence analysis using the method of Deleage and Roux (Deleage, G., Roux B.1987 Protein Engineering 1:289-294) accessed on the ProtScale website located on the World Wide Web at (.expasy.ch/cgi-bin/protscale.pl) through the ExPasy molecular biology server.

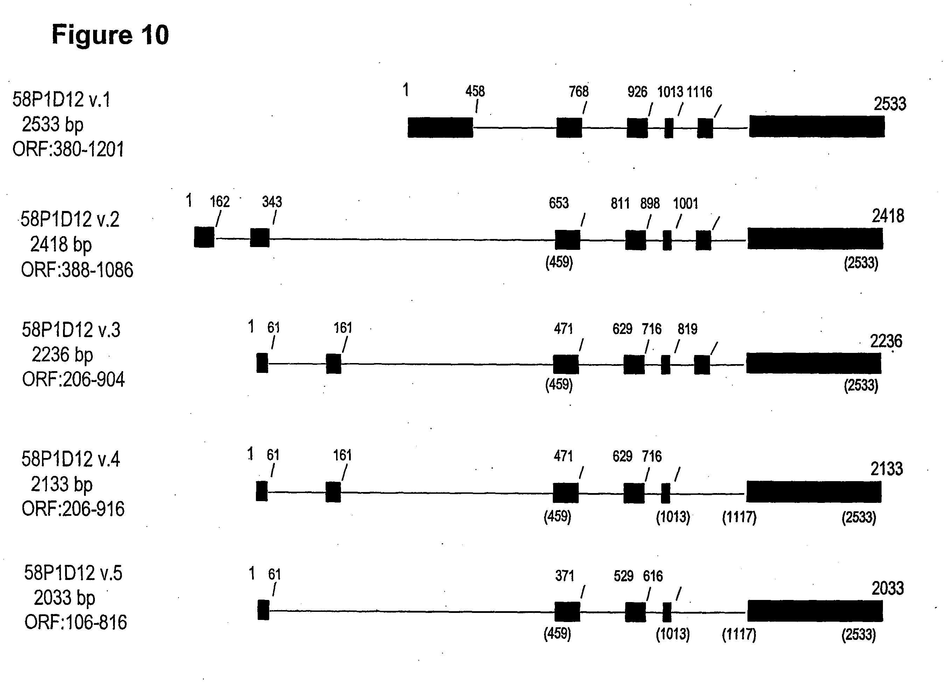

Figure 10. **Structures of transcript variants of 58P1D12**. Variant 58P1D12 v.2 through v.5 were transcript variants of 58P1D12 v.1. Variants 58P1D12 v.2 and v.3, each had two different exons in the 5' end, shared five exons in the 3' end with v.1. Variants v.4 and v.5 were similar to v.3, with deletions of one or two exons from v.3. All the exons in different transcript variants were aligned in relative order as on the genome. Poly A tails and SNP are not shown here. Numbers in "()" underneath the boxes correspond to those of 58P1D12 v.1. Lengths of introns and exons are not proportional.

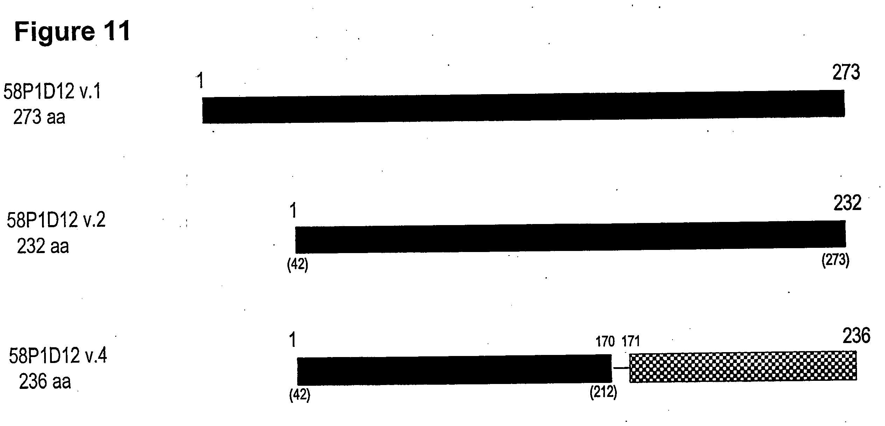

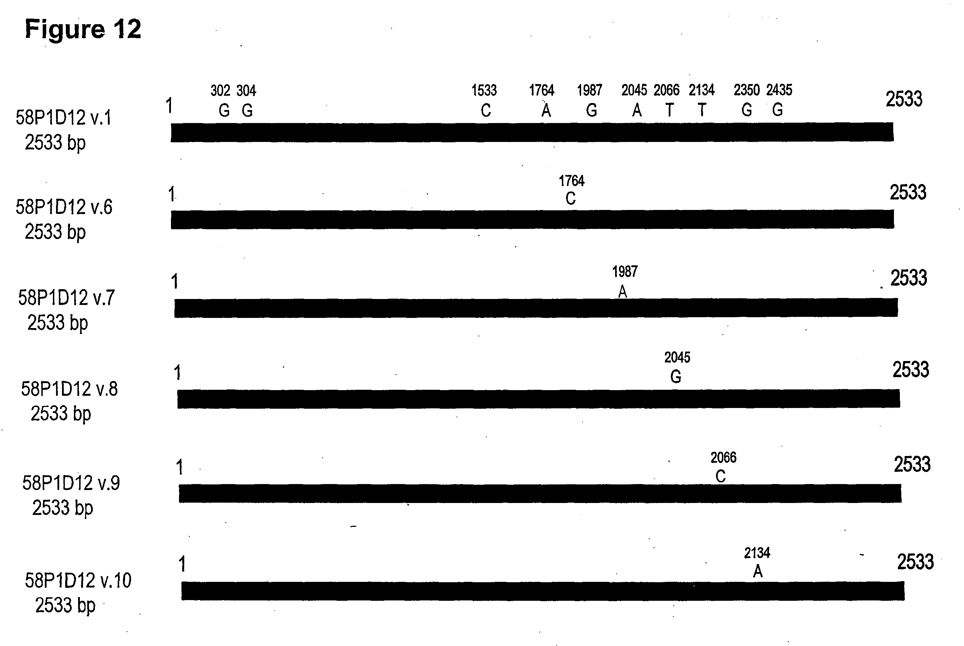

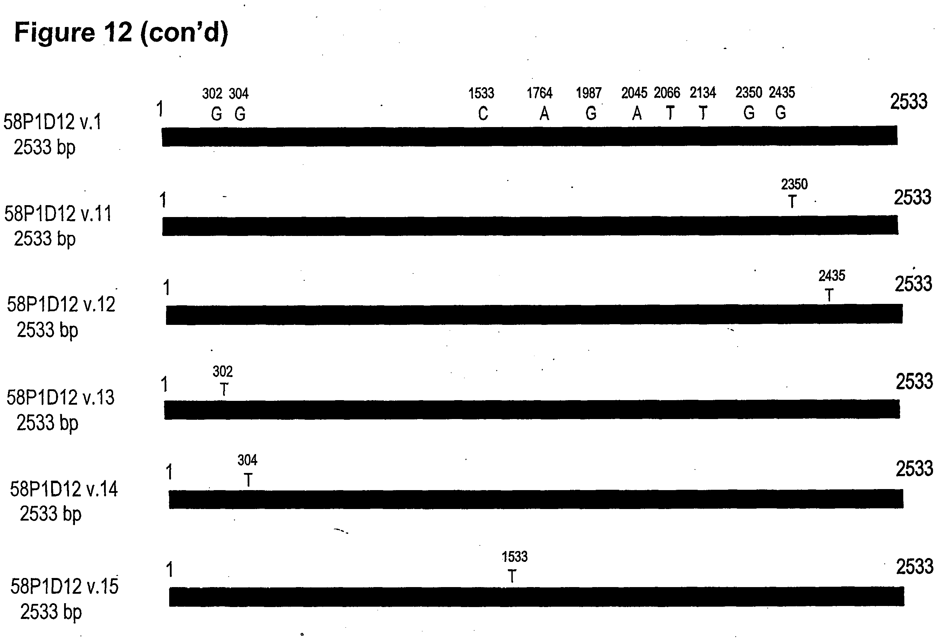

Figure 11. **Schematic alignment of protein variants of 58P1D12**. Protein variants corresponded to nucleotide variants. Nucleotide variants 58P1D12 v.3 coded for the same protein as variant v.2, and variant v.5 coded for the same protein as v.4. SNP variants v.6 through v.15 coded for the same protein as v.1. Nucleotide variants 58P1D12 v.6 through v.15 each had one alternative allele of a SNP as compared with variant v.1, as shown in Figure 12. Proteins of v.2 and v.3 were portion of v.1 protein, while variants v.4 and v.5 had a portion that was different from any port of v.1. Numbers underneath the box correspond to 58P1D12 v.1. Black boxes represent the same sequence as 58P1D12 v.1.

Figure 12. **Schematic alignment of SNP variants of 58P1D12**. Variants 58P1 D12 v.6 through v.12 were variants with single nucleotide differences as compared to variant v.1. Though these SNP variants were shown separately, they could also occur in any combinations (called haplotype), and in any transcript variants that contained the base pairs, such as v.2 shown in Fig.10. Numbers correspond to those of 58P1D12 v.1. Black box shows the same sequence as 58P1D12 v.1. SNPs are indicated above the box.

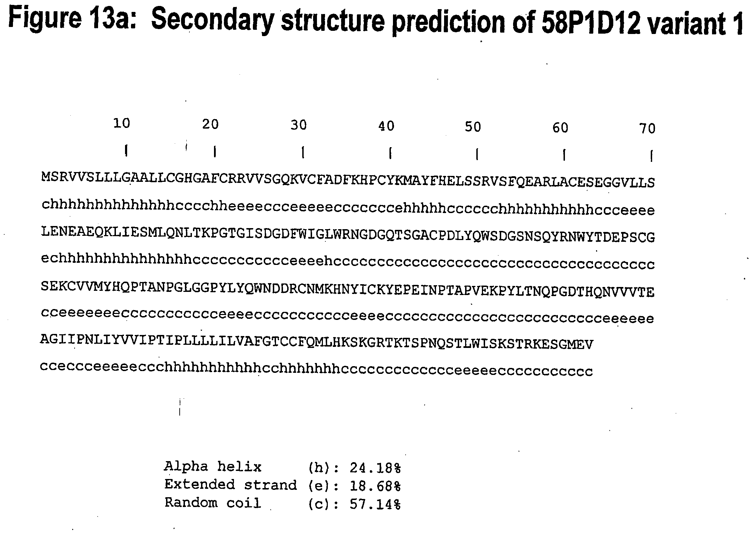

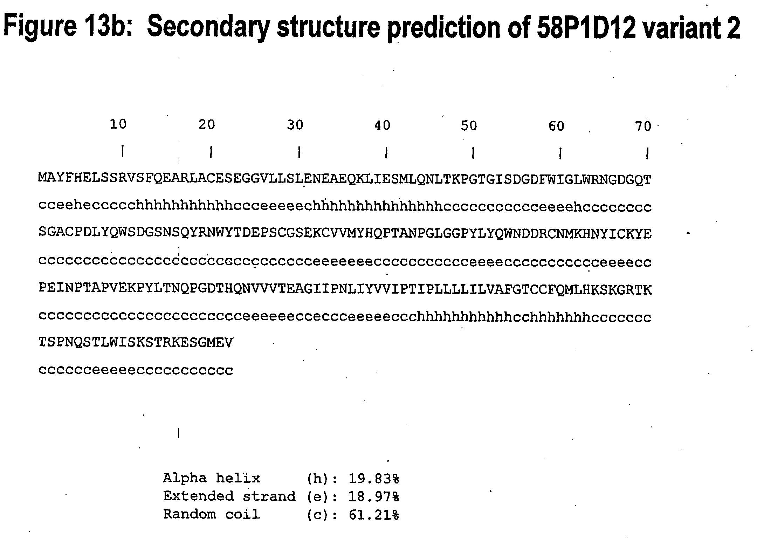

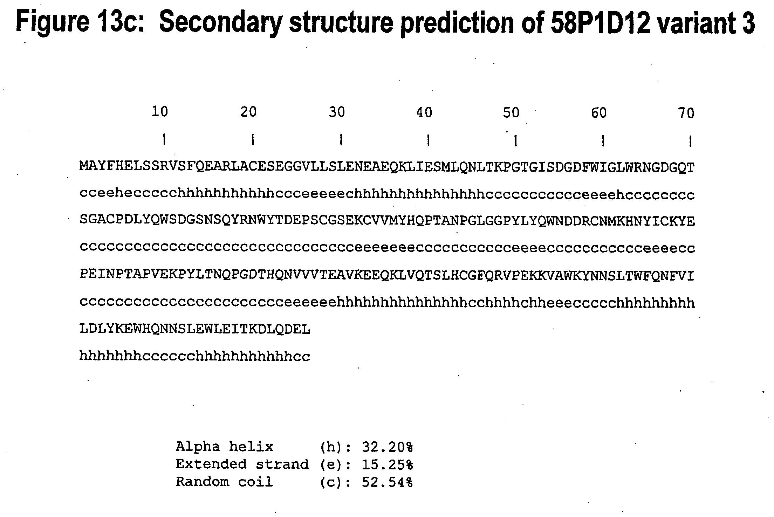

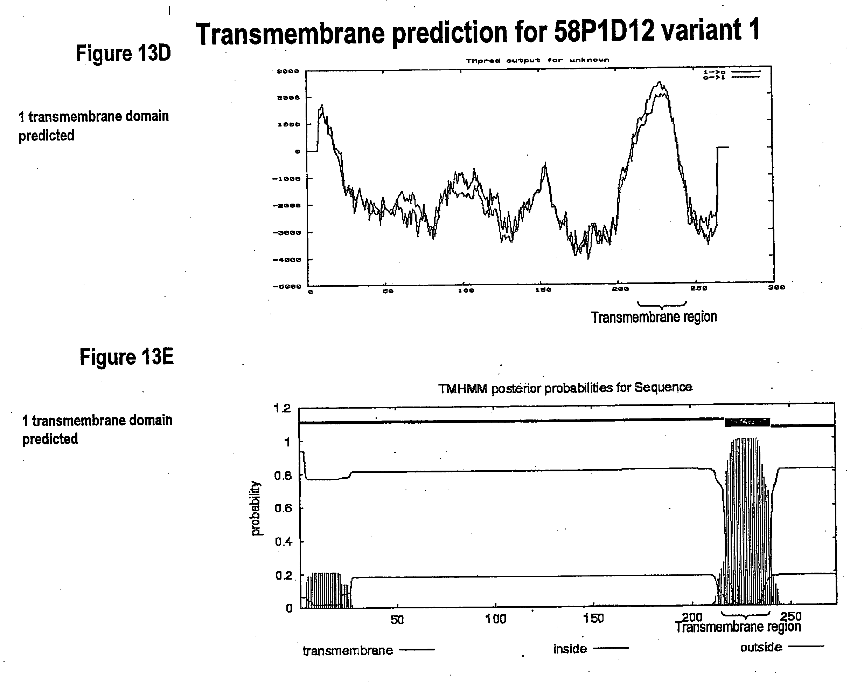

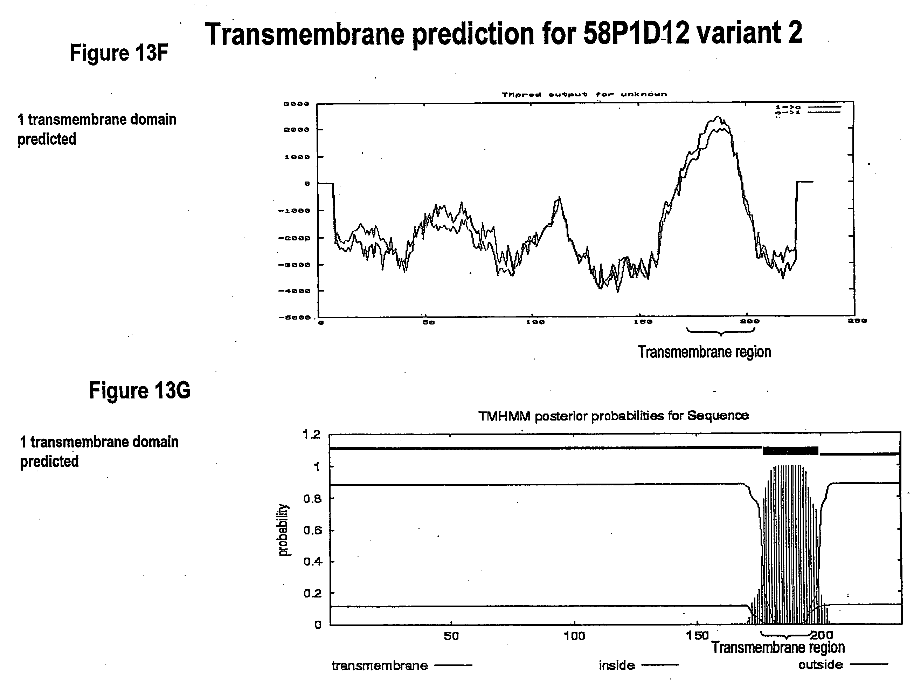

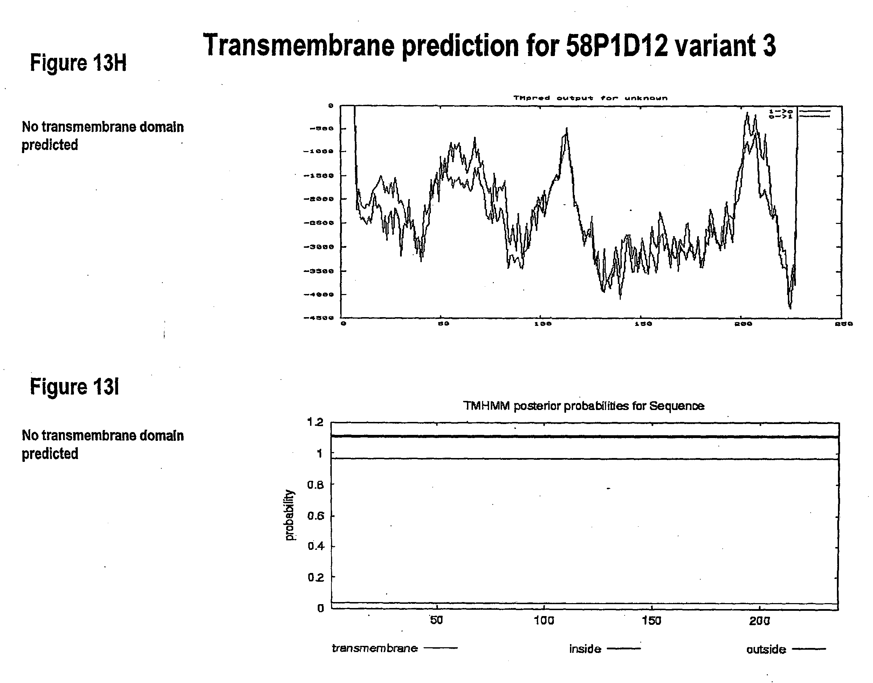

Figure 13. Secondary structure and transmembrane domains prediction for 58P1D12 protein variants.

Figure 13A-13C: The secondary structures of 58P1D12 protein variants 1 (SEQ ID NO: 15), variants 2 (SEQ ID NO: 16), variant 3 (SEQ ID NO: 17), respectively, were predicted using the HNN - Hierarchical Neural Network method (NPS@: Network Protein Sequence Analysis TIBS 2000 March Vol. 25, No 3 [291]:147-150 Combet C., Blanchet C., Geourjon C. and Deléage G., http://pbil.ibcp.fr/cgi-bin/npsa_automat.pl?page=npsa_nn.html), accessed from the ExPasy molecular biology server located on the World Wide Web at (.expasy.ch/tools/). This method predicts the presence and location of alpha helices, extended strands, and random coils from the primary protein sequence. The percent of the protein variant in a given secondary structure is also listed.

Figure 13D, Figure 13F, Figure 13H: Schematic representation of the probability of existence of transmembrane regions of 58P1D12 protein variants 1, 2, 3, respectively, based on the TMpred algorithm of Hofmann and Stoffel which utilizes TMBASE (K. Hofmann, W. Stoffel. TMBASE - A database of membrane spanning protein segments Biol. Chem. Hoppe-Seyler 374:166,1993).

Figure 13E, Figure 13G, Figure 13I: Schematic representation of the probability of the existence of transmembrane regions of 58P1D12 variants 1, 2, 3, respectively, based on the TMHMM algorithm of Sonnhammer, von Heijne, and Krogh (Erik L.L. Sonnhammer, Gunnar von Heijne, and Anders Krogh: A hidden Markov model for predicting transmembrane helices in protein sequences. In Proc. of Sixth Int. Conf. on Intelligent Systems for Molecular Biology, p 175-182 Ed J. Glasgow, T. Littlejohn, F. Major, R, Lathrop, D. Sankoff, and C. Sensen Menlo Park, CA: AAAI Press, 1998). The TMpred and TMHMM algorithms are accessed from the ExPasy molecular biology server located on the World Wide Web at (.expasy.ch/tools/).

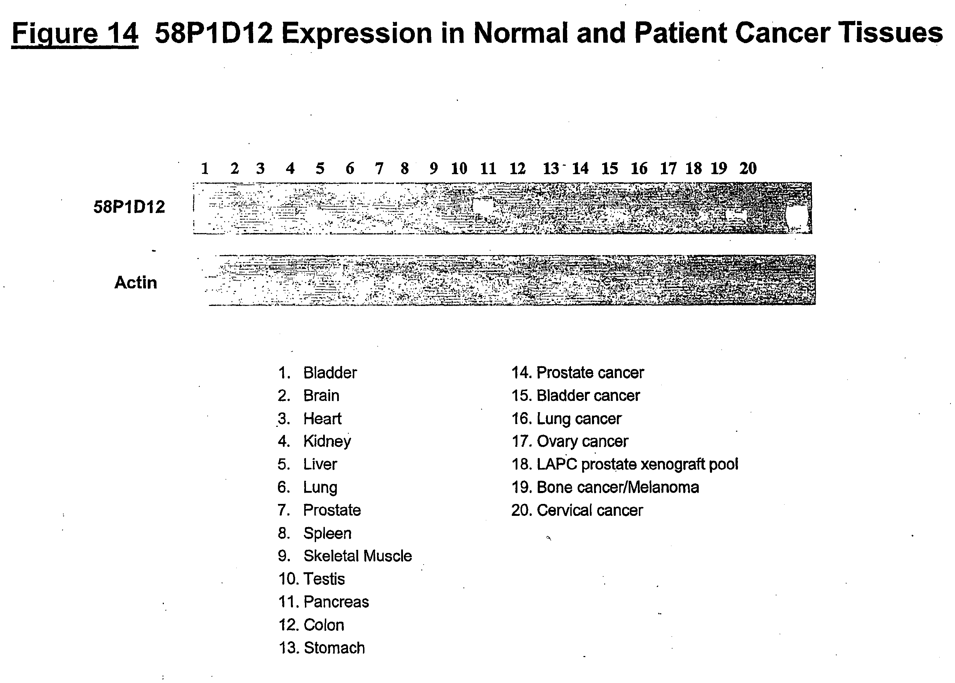

Figure 14. **58P1D12 Expression in Normal and Patient Cancer Tissues.** First strand cDNA was prepared from normal tissues (bladder, brain, heart, kidney, liver, lung, prostate, spleen, skeletal muscle, testis, pancreas, colon and stomach), and from pools of patient cancer specimens (prostate cancer pool, bladder cancer pool, lung cancer pool, ovary cancer pool, prostate cancer xenograft pool, bone and melanoma cancer pool, and cervical cancer pool). Normalization was performed by PCR using primers to actin. Semi-quantitative PCR, using primers to 58P1D12, was performed at 30 cycles of amplification. Results expression of 58P1D12 predominantly in testis amongst the normal tissues tested. 58P1D12 is also expressed in prostate cance, bladder cancer, lung cancer, ovary cancer, prostate cancer xenograft, bone/melanoma cancer pool, and cervical cancer.

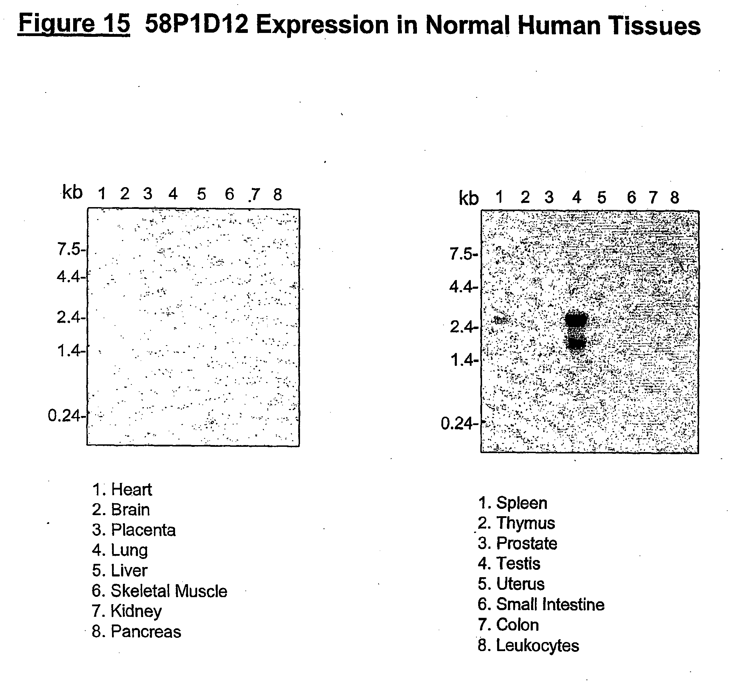

Figure 15. **Expression of 58P1D12 in normal tissues.** Two multiple tissue northern blots (Clontech) both with 2 ug of mRNA/lane were probed with the 58P1D12 sequence. Size standards in kilobases (kb) are indicated on the side. Results show predominant expression of 58P1D12 transcript in normal testis. A significantly weaker signal is detected in spleen, lung, kidney and pancreas.

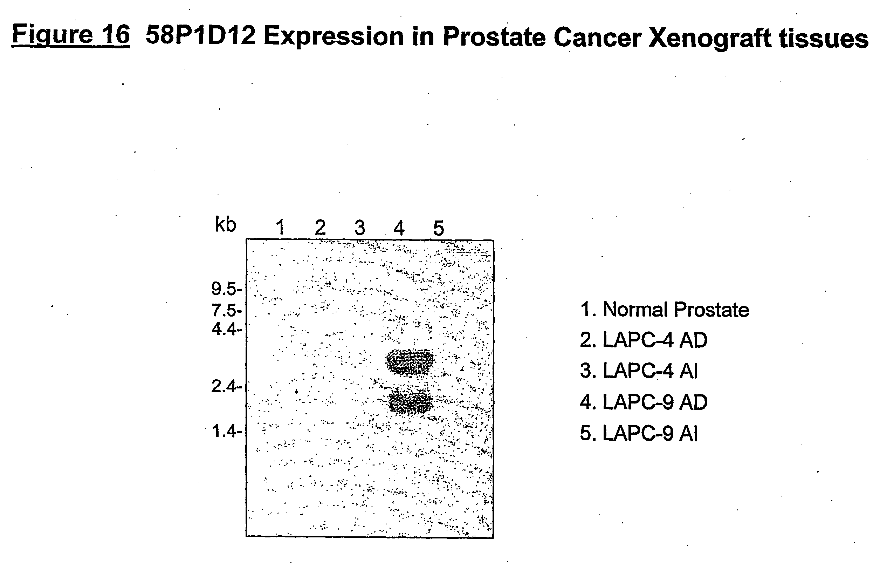

Figure 16. **58P1 D12 Expression in Prostate Cancer Xenograft tissues**. RNA was extracted from LAPC prostate cancer xenografts, LAPC-4AD, LAPC-4Al, LAPC-9AD and LAPC-9Al, as well as from normal prostate. Northern blots with 10 ug of total RNA were probed with the 58P1D12 sequence. Size standards in kilobases are on the side. Results show expression of 58P1D12 in LAPC-9AD but not in the other tissues tested.

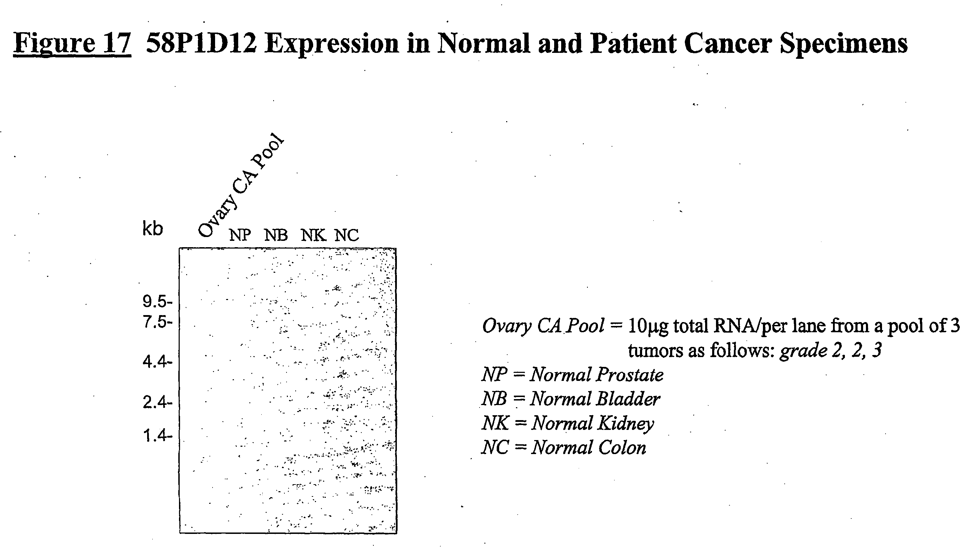

Figure 17. **58P1D12 Expression in Normal and Patient Cancer Specimens.** RNA was extracted from a pool of 3 ovary tumor patient specimens, normal prostate (NP), normal bladder (NB), normal kidney (NK), and normal colon (NC). Northern blots with 10 ug of total RNA were probed with the 58P1D12 sequence. Size standards in kilobases are on the side. Results show expression of 58P1D12 in ovary tumor patient specimens but not in the normal tissues tested.

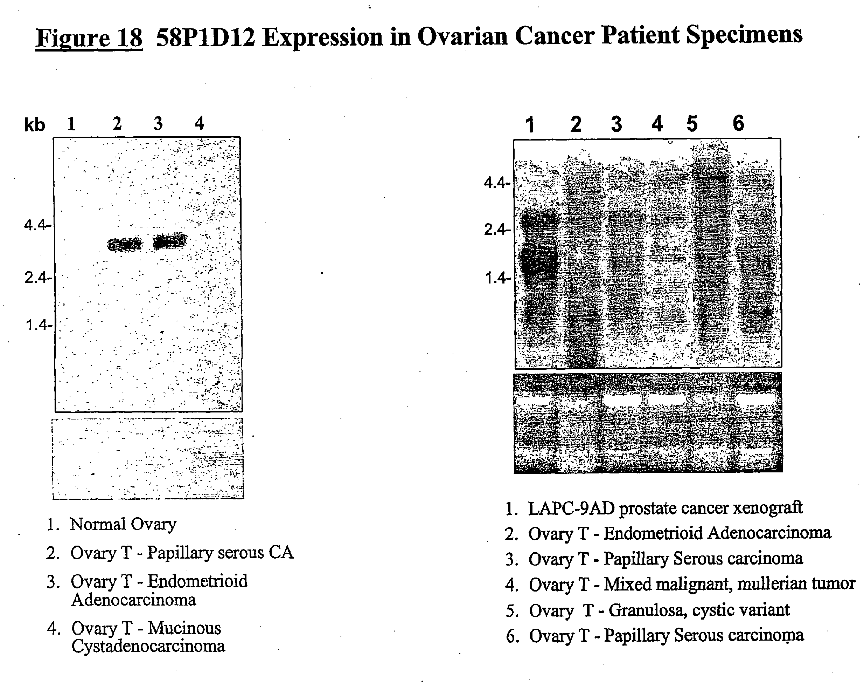

Figure 18. **58P1D12 Expression in Ovarian Cancer Patient Specimens**. RNA was extracted from normal ovary, LAPC-9AD prostate cncer xenograft, and a panel of ovary tumor patient specimens (Ovary T). Northern blots with 10 ug of total RNA were probed with the 58P1D12 sequence. Size standards in kilobases are on the side. Results show expression of 58P1D12 in most of the patient specimens tested as well as in the prostate xenograft.

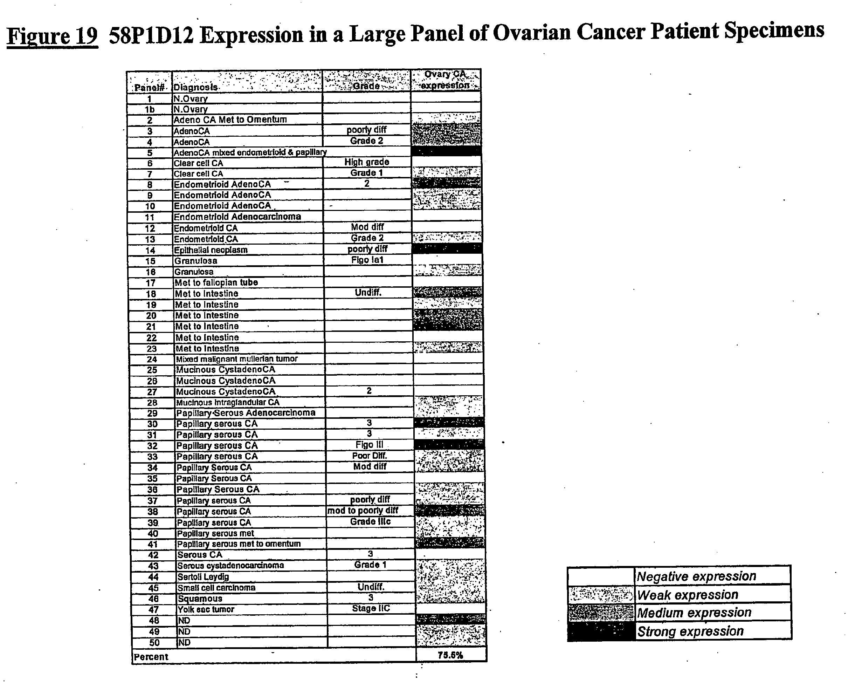

Figure 19. **58P1D12 Expression in a Large Panel of Ovarian Cancer Patient Specimens.** First strand cDNA was prepared from a panel of ovary patient cancer specimens as well as two different normal ovary tissues. Normalization was performed by PCR using primers to actin. Semi-quantitative PCR, using primers to 58P1D12, was performed at 26 and 30 cycles of amplification. Samples were run on an agarose gel, and PCR products were quantitated using the Alphalmager software. Expression was recorded as negative, weak, medium or strong. Results show expression of 58P1D12 in the majority of patient cancer specimens tested (75%), but not in normal ovary.

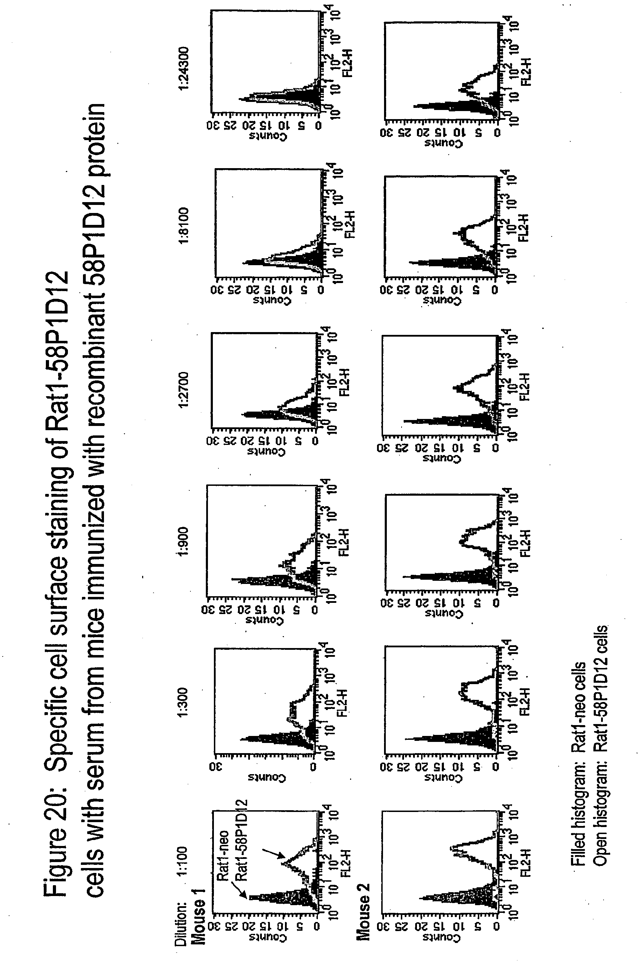

Figure 20. Specific cell surface staining of Rat1-58P1D12 cells with serum from mice immunized with recombinant 58P1D12 protein. Rat1-58P1D12 and control Rat1-neo cells (-100,000 cells) were incubated with various dilutions of serum obtained from 2 different mice immunized with recombinant Tag5-58P1D12 protein. Cells were washed and then incubated with goat anti-mouse IgG-PE secondary Ab (1:100 dilution in PBS + 2% FBS) on ice for 1 hour. Cells were washed again, fixed in 2% paraformaldehyde in PBS, and subjected to FACS analysis. The Rat1-58P1D12 cells, but not the Rat1-neo cells, show a shift in fluorescent intensity over a large dilution range demonstrating specific binding of anti-58P1D12 Abs present in the serum from the 2 mice.

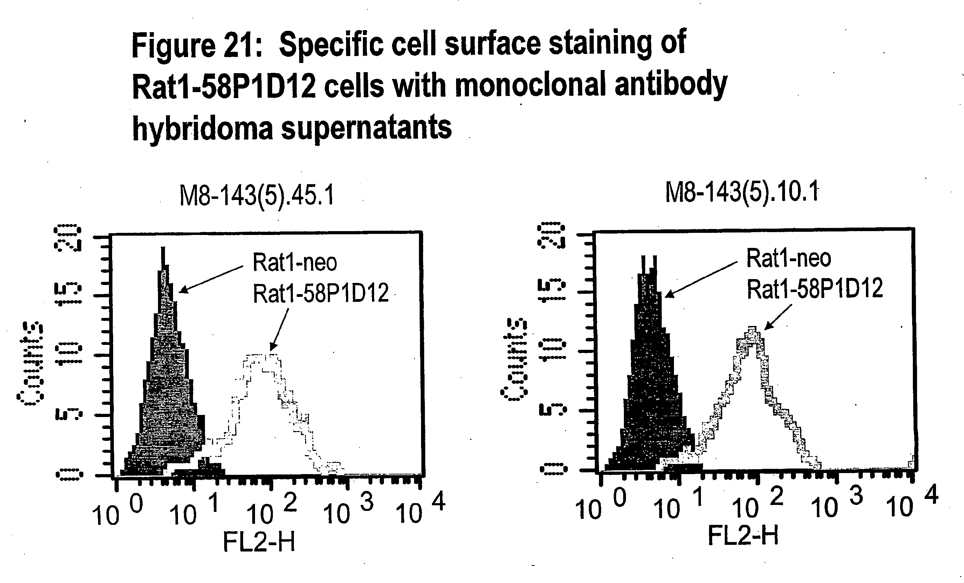

Figure 21. Specific cell surface staining of Rat1-58P1D12 cells with monoclonal antibody hybridoma supernatants. Rat1-58P1D12 and control Rat1-neo cells (-100,000 cells) were incubated with supernatants derived from hybridomas M8-143(5).10.1 and M8-143(5).45.1. on ice for 1 hour. Cells were washed and then incubated with goat anti-mouse IgG-PE secondary Ab (1:100 dilution in PBS + 2% FBS) on ice for 1 hour. Cells were washed again, fixed in 2% paraformaldehyde in PBS, and subjected to FACS analysis. The Rat1-58P1D12 cells, but not the Rat1-neo cells, show a shift in fluorescent intensity demonstrating specific binding of the MAb to 58P1D12 protein on the cell surface.

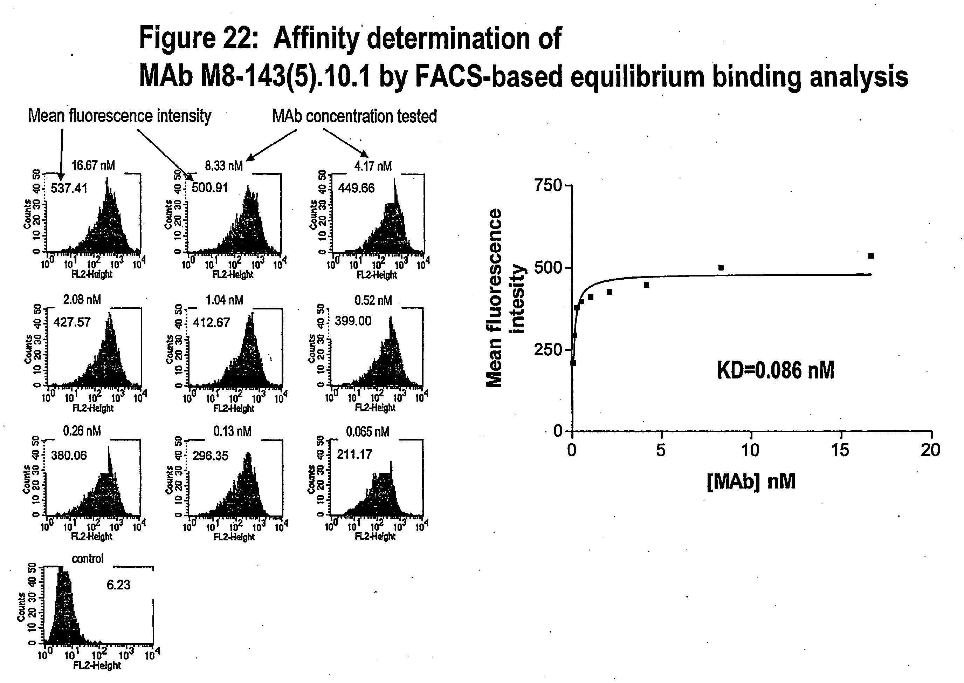

Figure 22. Affinity determination of MAb M8-143(5).10.1 by FACS-based equilibrium binding analysis. 3T3-58P1012 cells were incubated at 4C for 2 hours with various concentrations of MAb M8-143(5).10.1 in Pubs+2% FBS,+0.04% NaN3. Cells were washed, and then incubated at 4C for 1 hour with a 1:100 dilution of goat anti-mouse IgG-PE secondary Ab. After washing, cells were fixed in 2% paraformaldehyde in PBS and subjected to FACS analysis. Shown on the left are the histograms of cells stained with various concentrations of MAb. On the right is a graphical representation of the data plotting MAb concentration versus the mean fluorescent intensity of the stained populations. Non-linear regression of the binding data was carried out with the GraphPad Prism software (Version 3.00 for Windows, GraphPad Software, San Diego California USA) to determine the dissociation constant (KD). The analysis shows that MAb M8-143(5).10.1 is a high affinity MAb with an apparent KD of 0.086 nM on 3T3-58P1D12 cells.

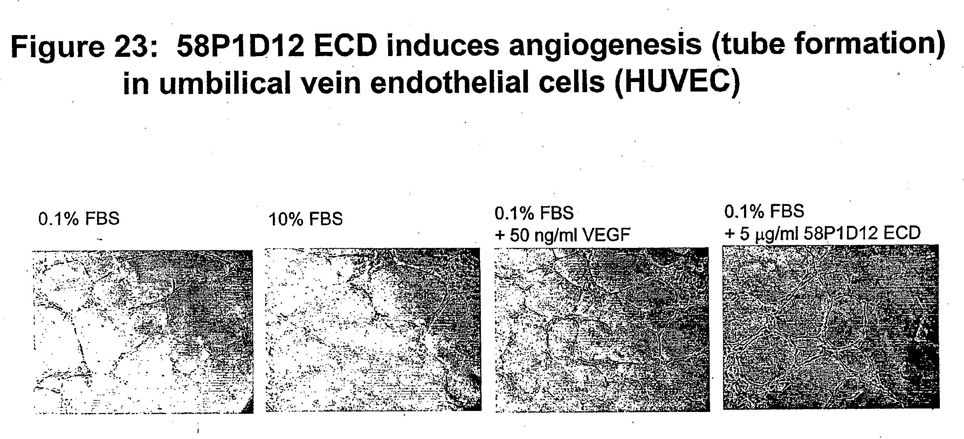

Figure 23. 58P1D12 induces angiogenesis (tube formation) in umbilical vein endothelial cells (HUVEC). Primary umbilical vein endothelial cells were cultured in 10% FBS, then removed with EDTA and mixed with media containing either 10% FBS, 0.1 % FBS, 0.1% FBS + 50 ng/ml VEGF or 0.1 % FBS + 5 µg/ml 58P1D12 ECD protein. The mixtures were plated onto a 200 µl layer of Matrigel® and then incubated at 37°C for 16 hours. The plates were then analyzed for the formation of tubes by microscopy. Images of representative fields are shown. The data show that HUVEC cells form tubes in 10% FBS but not in 0.1% FBS, thereby allowing the assessment of 58P1D12-induced angiogenesis in low serum conditions. At 5 µg/ml, 58P1D12 ECD exhibited the capacity to induce the formation of endothelial tubes similarly as the positive control VEGF.

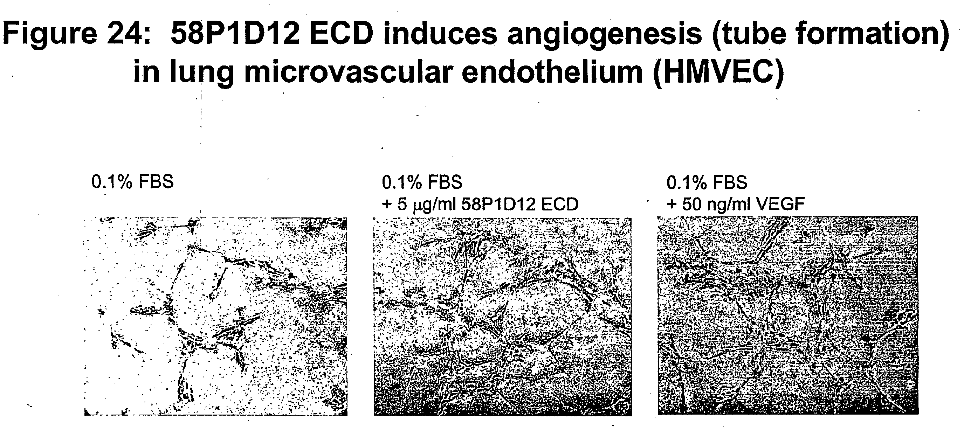

Figure 24. 58P1D12 induces angiogenesis (tube formation) in lung microvascular endothelial cells (HMVEC). Primary lung endothelial cells were cultured in 10% FBS, then removed with EDTA and mixed with media containing either 0.1% FBS, 0.1 % FBS + 50 ng/ml VEGF or 0.1% FBS + 5 µg/ml 58P1D12 ECD protein. The mixtures were plated onto a 200 µl layer of Matrigel® and then incubated at 37oC for 16 hours. The plates were then analyzed for the formation of tubes by microscopy. Images of representative fields are shown. The data show that HMVEC cells do not form tubes in 0.1 % FBS, thereby allowing the assessment of 58P1D12-induced angiogenesis in low serum conditions. At 5 µg/ml, 58P1D12 ECD exhibited the capacity to induce the formation of endothelial tubes similarly as the positive control VEGF.

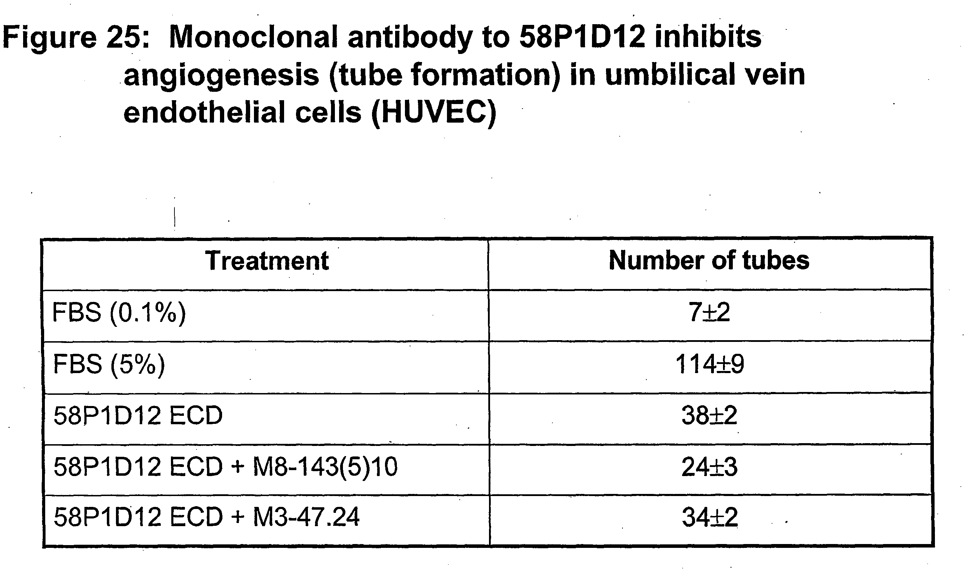

Figure 25. Monoclonal antibody to 58P1D12 inhibits angiogenesis (tube formation) in umbilical vein endothelial cells (HUVEC). Primary umbilical vein endothelial cells were cultured in 10% FBS, then removed with EDTA and mixed with media containing either 0.1% FBS, 5% FBS, 0.1% FBS + 3 µg/ml 58P1D12 ECD protein, 0.1%FBS + 3 µg/ml 58P1D12 ECD protein + 30 µg/ml MAb M8-143(5)10 or 0.1%FBS + 3 µg/ml 58P1D12 ECD protein + 30 µg/ml MAb M3-47.24. The mixtures were plated onto a 200 µl layer of Matrigel® and then incubated at 37°C for 16 hours. The plates were then analyzed for the formation of tubes by microscopy. The total tube counts are shown. The data show that HUVEC cells form tubes in 5% FBS but not in 0.1 % FBS, thereby allowing the assessment of 58P1D12-induced angiogenesis in low serum conditions. At 3 µg/ml, 58P1D12 ECD exhibited the capacity to induce the formation of endothelial tubes, and that incubation with MAb M8-143(5)10, but not MAb M3-47.24, inhibited the tube formation by 45%.

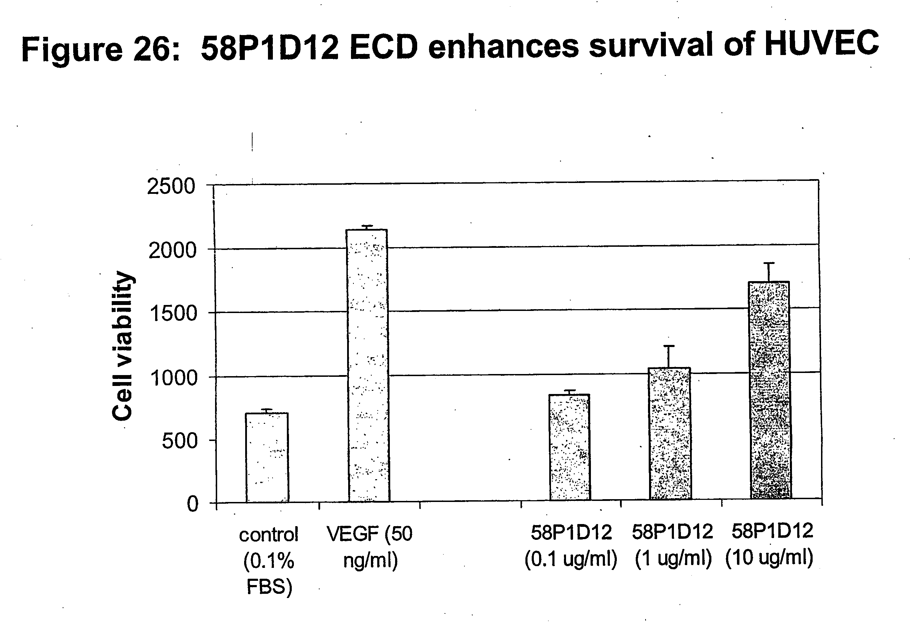

Figure 26. 58P1D12 ECD enhances survival of HUVEC. Primary umbilical vein endothelial cells were cultured in 10% FBS. Cells were trypsinized and 3000 cells per well were plated onto 96-well dishes for 72 hours. Cell viability was assayed by Alamar blue staining for 3 hours, followed by fluorescence measurement. The assay was performed in triplicate with standard deviations represented. In low serum conditions (0.1 % FBS), cell viability was low, but was enhanced by increasing concentrations of 58P1D12 ECD. For positive control, the cells were treated with 50 ng/ml VEGF. Cell viability at 10 µg/ml 58P1D12 ECD was similar to that observed using 50 ng/ml VEGF (a known survival signal for endothelium).

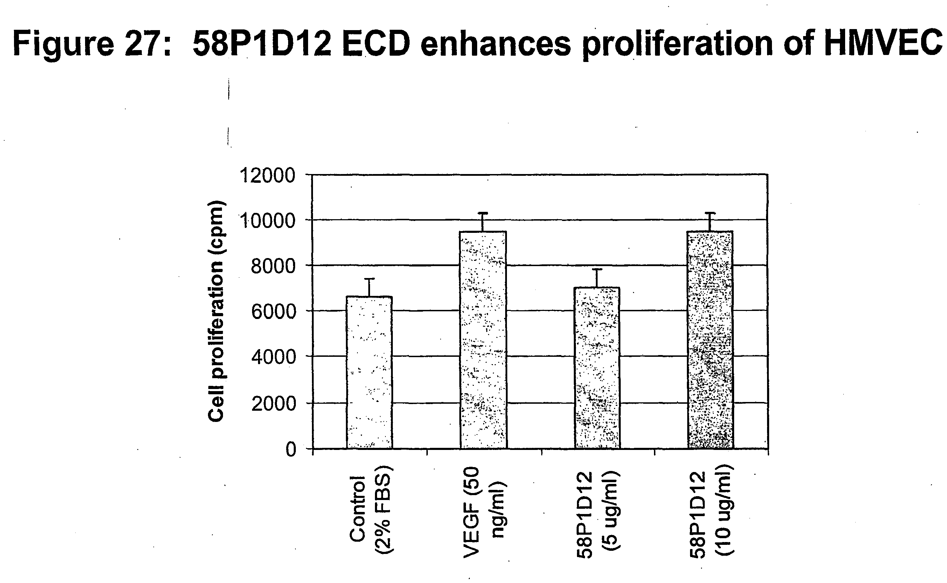

Figure 27. 58P1D12 ECD enhances proliferation of HMVEC. Primary lung microvascular endothelial cells (HMVEC) were cultured in 10% FBS. Cells were trypsinized and 3000 cells per well were plated onto 96-well dishes in 2% FBS. The cells were either left untreated, or were incubated with 50 ng/ml VEGF, 5 µg/ml 58P1D12 ECD or 10 µg/ml 58P1D12 ECD. After 48 hours, the cells were incubated with 3H-thymidine and allowed to grow overnight. Incorporation of 3H-thymidine into cell DNA was assayed by standard protocols. The assay was performed in triplicate with standard deviations represented. The data indicate that basal proliferation could be measured in 2% FBS, where increased proliferation was observed with 58P1D12ECD. Similar levels of enhanced proliferation were seen with 10 µg/ml 58P1D12 ECD compared with the VEGF positive control.

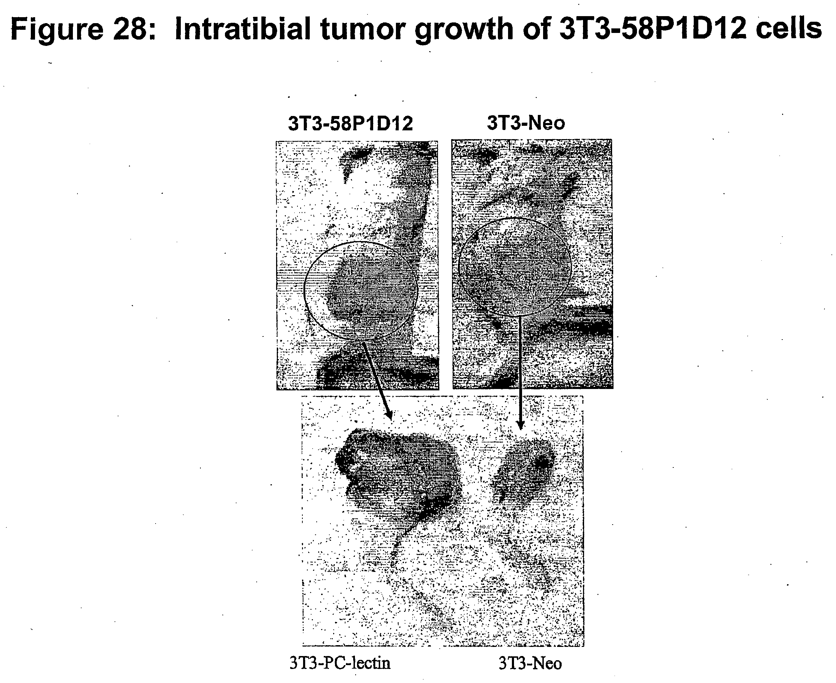

Figure 28. Intratibial tumor growth of 3T3-58P1D12 cells. 3T3 fibroblasts were infected with empty vector control amphotropic virus (Neo) or virus containing the 58P1D12 gene. Stable transfectants were selected in G-418 (geneticin) and then grown in 10% FBS. For each cell type, 3T3-Neo and 3T3-58P1D12,1 x 10⁶ cells were mixed with Matrigel® and injected into the flanks of 7 male SCID mice. After 31 days, the mice were sacrified, and the tumors were extracted with the hind quarters. The data are representative of the 7 mice treated with the two cell types. The data indicate that the expression of 58P1D12 in the non-tumor forming 3T3 line has increased tumor growth potential in vivo.

Detailed Description of the invention

### I.) Definitions:

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art, such as, for example, the widely utilized molecular cloning methodologies described in Sambrook et al., Molecular Cloning: A Laboratory Manual 2nd. edition (1989) Cold Spring Harbor Laboratory Press, Cold Spring Harbor, N.Y. As appropriate, procedures involving the use of commercially available kits and reagents are generally carried out in accordance with manufacturer defined protocols and/or parameters unless otherwise noted.

The terms "advanced prostate cancer", "locally advanced prostate cancer", "advanced disease" and locally advanced disease" mean prostate cancers that have extended through the prostate capsule, and are meant to include stage C disease under the American Urological Association (AUA) system, stage C1- C2 disease under the Whitmore-Jewett system, and stage T3 - T4 and N+ disease under the TNM (tumor, node, metastasis) system. In general, surgery is not recommended for patients with locally advanced disease, and these patients have substantially less favorable outcomes compared to patients having clinically localized (organ-confined) prostate cancer. Locally advanced disease is clinically identified by palpable evidence of induration beyond the lateral border of the prostate, or asymmetry or induration above the prostate base. Locally advanced prostate cancer is presently diagnosed pathologically following radical prostatectomy if the tumor invades or penetrates the prostatic capsule, extends into the surgical margin, or invades the seminal vesicles.

"Altering the native glycosylation pattern" is intended for purposes herein to mean deleting one or more carbohydrate moieties found in native sequence 58P1D12 (either by removing the underlying glycosylation site or by deleting the glycosylation by chemical and/or enzymatic means), and/or adding one or more glycosylation sites that are not present in the native sequence 58P1D12. In addition, the phrase includes qualitative changes in the glycosylation of the native proteins, involving a change in the nature and proportions of the various carbohydrate moieties present.

The term "analog" refers to a molecule which is structurally similar or shares similar or corresponding attributes with another molecule (e.g. a 58P1D12-related protein). For example, an analog of a 58P1D12 protein can be specifically bound by an antibody or T cell that specifically binds to 58P1D12.

The term "antibody" is used in the broadest sense. Therefore, an "antibody" can be naturally occurring or man-made such as monoclonal antibodies produced by conventional hybridoma technology. Anti-58P1D12 antibodies comprise monoclonal and polyclonal antibodies as well as fragments containing the antigen-binding domain and/or one or more complementarity determining regions of these antibodies.

An "antibody fragment" is defined as at least a portion of the variable region of the immunoglobulin molecule that binds to its target, i.e., the antigen-binding region. In one embodiment it specifically covers single anti-58P1D12 antibodies and clones thereof (including agonist, antagonist and neutralizing antibodies) and anti-58P1D12 antibody compositions with polyepitopic specificity.

The term "codon optimized sequences" refers to nucleotide sequences that have been optimized for a particular host species by replacing any codons having a usage frequency of less than about 20%. Nucleotide sequences that have been optimized for expression in a given host species by elimination of spurious polyadenylation sequences, elimination of exon/intron splicing signals, elimination of transposon-like repeats and/or optimization of GC content in addition to codon optimization are referred to herein as an "expression enhanced sequences."

A "combinatorial library" is a collection of diverse chemical compounds generated by either chemical synthesis or biological synthesis by combining a number of chemical "building blocks" such as reagents. For example, a linear combinatorial chemical library, such as a polypeptide (e.g., mutein) library, is formed by combining a set of chemical building blocks called amino acids in every possible way for a given compound length (i.e., the number of amino acids in a polypeptide compound). Numerous chemical compounds are synthesized through such combinatorial mixing of chemical building blocks (Gallop et al., J. Med. Chem. 37(9):1233-1251 (1994)).

Preparation and screening of combinatorial libraries is well known to those of skill in the art. Such combinatorial chemical libraries include, but are not limited to, peptide libraries (see, e.g., U.S. Patent No. 5,010,175, Furka, Pept. Prot. Res. 37:487-493 (1991), Houghton et al., Nature, 354:84-88 (1991)), peptoids (PCT Publication No WO 91/19735), encoded peptides (PCT Publication WO 93/20242), random bio- oligomers (PCT Publication WO 92/00091), benzodiazepines (U.S. Pat. No. 5,288,514), diversomers such as hydantoins, benzodiazepines and dipeptides (Hobbs et al., Proc. Nat. Acad. Sd. USA 90:6909-6913 (1993)), vinylogous polypeptides (Hagihara et al., J. Amer. Chem. Soc. 114:6568 (1992)), nonpeptidal peptidomimetics with a Beta-D-Glucose scaffolding (Hirschmann et al., J. Amer. Chem. Soc. 114:9217-9218 (1992)), analogous organic syntheses of small compound libraries (Chen et al., J. Amer. Chem. Soc. 116:2661 (1994)), oligocarbamates (Cho, et al., Science 261:1303 (1993)), and/or peptidyl phosphonates (Campbell et al., J. Org. Chem. 59:658 (1994)). See, generally, Gordon et al., J. Med. Chem. 37:1385 (1994), nucleic acid libraries (see, e.g., Stratagene, Corp.), peptide nucleic acid libraries (see, e.g., U.S. Patent 5,539,083), antibody libraries (see, e.g., Vaughn et al., Nature Biotechnology 14(3): 309-314 (1996), and PCT/US96/10287), carbohydrate libraries (see, e.g., Liang et al., Science 274:1520-1522 (1996), and U.S. Patent No. 5,593,853), and small organic molecule libraries (see, e.g., benzodiazepines, Baum, C&EN, Jan 18, page 33 (1993); isoprenoids, U.S. Patent No. 5,569,588; thiazolidinones and metathiazanones, U.S. Patent No. 5,549,974; pyrrolidines, U.S. Patent Nos. 5,525,735 and 5,519,134; morpholino compounds, U.S. Patent No. 5,506, 337; benzodiazepines, U.S. Patent No. 5,288,514; and the like).

Devices for the preparation of combinatorial libraries are commercially available (see, e.g., 357 NIPS, 390 NIPS, Advanced Chem Tech, Louisville KY; Symphony, Rainin, Wobum, MA; 433A, Applied Biosystems, Foster City, CA; 9050, Plus, Millipore, Bedford, NIA). A number of well-known robotic systems have also been developed for solution phase chemistries. These systems include automated workstations such as the automated synthesis apparatus developed by Takeda Chemical Industries, LTD. (Osaka, Japan) and many robotic systems utilizing robotic arms (Zymate H, Zymark Corporation, Hopkinton, Mass.; Orca, Hewlett-Packard, Palo Alto, Calif.), which mimic the manual synthetic operations performed by a chemist. Any of the above devices are suitable for use with the present invention. The nature and implementation of modifications to these devices (if any) so that they can operate as discussed herein will be apparent to persons skilled in the relevant art. In addition, numerous combinatorial libraries are themselves commercially available (see, e.g., ComGenex, Princeton, NJ; Asinex, Moscow, RU; Tripos, Inc., St. Louis, MO; ChemStar, Ltd, Moscow, RU; 3D Pharmaceuticals, Exton, PA; Martek Biosciences, Columbia, MD; etc.).

The term "cytotoxic agent" refers to a substance that inhibits or prevents the expression activity of cells, function of cells and/or causes destruction of cells. The term is intended to include radioactive isotopes chemotherapeutic agents, and toxins such as small molecule toxins or enzymatically active toxins of bacterial, fungal, plant or animal origin, including fragments and/or variants thereof. Examples of cytotoxic agents include, but are not limited to auristatins, auromycins, maytansinoids, yttrium, bismuth, ricin, ricin A-chain, combrestatin, duocarmycins, dolostatins, doxorubicin, daunorubicin, taxol, cisplatin, cc1065, ethidium bromide, mitomycin, etoposide, tenoposide, vincristine, vinblastine, colchicine, dihydroxy anthracin dione, actinomycin, diphtheria toxin, Pseudomonas exotoxin (PE) A, PE40, abrin, abrin A chain, modeccin A chain, alpha-sarcin, gelonin, mitogellin, retstrictocin, phenomycin, enomycin, curicin, crotin, calicheamicin, Sapaonaria officinalis inhibitor, and glucocorticoid and other chemotherapeutic agents, as well as radioisotopes such as At211, I131, I125, Y90, Re186, Re188, Sm153, Bi212 or 213, P32 and radioactive isotopes of Lu including Lu177. Antibodies may also be conjugated to an anti-cancer pro-drug activating enzyme capable of converting the pro-drug to its active form.

The "gene product" is sometimes referred to herein as a protein or mRNA. For example, a "gene product of the invention" is sometimes referred to herein as a "cancer amino acid sequence", "cancer protein", "protein of a cancer listed in Table I", a "cancer mRNA", "mRNA of a cancer listed in Table I", etc. In one embodiment, the cancer protein is encoded by a nucleic acid of Figure 2. The cancer protein can be a fragment, or alternatively, be the full-length protein to the fragment encoded by the nucleic acids of Figure 2. In one embodiment, a cancer amino acid sequence is used to determine sequence identity or similarity. In another embodiment, the sequences are naturally occurring allelic variants of a protein encoded by a nucleic acid of Figure 2. In another embodiment, the sequences are sequence variants as further described herein.

"High throughput screening" assays for the presence, absence, quantification, or other properties of particular nucleic acids or protein products are well known to those of skill in the art. Similarly, binding assays and reporter gene assays are similarly well known. Thus, e.g., U.S. Patent No. 5,559,410 discloses high throughput screening methods for proteins; U.S. Patent No. 5,585,639 discloses high throughput screening methods for nucleic acid binding (i.e., in arrays); while U.S. Patent Nos. 5,576,220 and 5,541,061 disclose high throughput methods of screening for ligand/antibody binding.

In addition, high throughput screening systems are commercially available (see, e.g., Amersham Biosciences, Piscataway, NJ; Zymark Corp., Hopkinton, MA; Air Technical Industries, Mentor, OH; Beckman Instruments, Inc. Fullerton, CA; Precision Systems, Inc., Natick, MA; etc.). These systems typically automate entire procedures, including all sample and reagent pipetting, liquid dispensing, timed incubations, and final readings of the microplate in detector(s) appropriate for the assay. These configurable systems provide high throughput and rapid start up as well as a high degree of flexibility and customization. The manufacturers of such systems provide detailed protocols for various high throughput systems. Thus, e.g., Zymark Corp. provides technical bulletins describing screening systems for detecting the modulation of gene transcription, ligand binding, and the like.

The term "homolog" refers to a molecule which exhibits homology to another molecule, by for example, having sequences of chemical residues that are the same or similar at corresponding positions.

"Human Leukocyte Antigen" or "HLA" is a human class I or class II Major Histocompatibility Complex (MHC) protein (see, e.g., Stites, et al., Immunology, 8th Ed., Lange Publishing, Los Altos, CA (1994).

The terms "hybridize", "hybridizing", "hybridizes" and the like, used in the context of polynucleotides, are meant to refer to conventional hybridization conditions, preferably such as hybridization in 50% formamide/6XSSC/0.1% SDS/100 µg/ml ssDNA, in which temperatures for hybridization are above 37 degrees C and temperatures for washing in 0.1XSSC/0.1% SDS are above 55 degrees C.

The phrases "isolated" or "biologically pure" refer to material which is substantially or essentially free from components which normally accompany the material as it is found in its native state. Thus, isolated peptides in accordance with the invention preferably do not contain materials normally associated with the peptides in their in situ environment For example, a polynucleotide is said to be "isolated" when it is substantially separated from contaminant polynucleotides that correspond or are complementary to genes other than the 58P1D12 genes or that encode polypeptides other than 58P1D12 gene product or fragments thereof. A skilled artisan can readily employ nucleic acid isolation procedures to obtain an isolated 58P1D12 polynucleotide. A protein is said to be "isolated," for example, when physical, mechanical or chemical methods are employed to remove the 58P1D12 proteins from cellular constituents that are normally associated with the protein. A skilled artisan can readily employ standard purification methods to obtain an isolated 58P1D12 protein. Alternatively, an isolated protein can be prepared by chemical means.

The term "mammal" refers to any organism classified as a mammal, including mice, rats, rabbits, dogs, cats, cows, horses and humans. In one embodiment of the invention, the mammal is a mouse. In another embodiment of the invention, the mammal is a human.

The terms "metastatic prostate cancer" and "metastatic disease" mean prostate cancers that have spread to regional lymph nodes or to distant sites, and are meant to include stage D disease under the AUA system and stage TxNxM+ under the TNM system. As is the case with locally advanced prostate cancer, surgery is generally not indicated for patients with metastatic disease, and hormonal (androgen ablation) therapy is a preferred treatment modality. Patients with metastatic prostate cancer eventually develop an androgen-refractory state within 12 to 18 months of treatment initiation. Approximately half of these androgen-refractory patients die within 6 months after developing that status. The most common site for prostate cancer metastasis is bone. Prostate cancer bone metastases are often osteoblastic rather than osteolytic (i.e., resulting in net bone formation). Bone metastases are found most frequently in the spine, followed by the femur, pelvis, rib cage, skull and humerus. Other common sites for metastasis include lymph nodes, lung, liver and brain. Metastatic prostate cancer is typically diagnosed by open or laparoscopic pelvic lymphadenectomy, whole body radionuclide scans, skeletal radiography, and/or bone lesion biopsy.

The term "modulator" or "test compound" or "drug candidate" or grammatical equivalents as used herein describe any molecule, e.g., protein, oligopeptide, small organic molecule, polysaccharide, polynucleotide, etc., to be tested for the capacity to directly or indirectly alter the cancer phenotype or the expression of a cancer sequence, e.g., a nucleic acid or protein sequences, or effects of cancer sequences (e.g., signaling, gene expression, protein interaction, etc.) In one aspect, a modulator will neutralize the effect of a cancer protein of the invention. By "neutralize" is meant that an activity of a protein is inhibited or blocked, along with the consequent effect on the cell. In another aspect, a modulator will neutralize the effect of a gene, and its corresponding protein, of the invention by normalizing levels of said protein. In preferred embodiments, modulators alter expression profiles, or expression profile nucleic acids or proteins provided herein, or downstream effector pathways. In one embodiment, the modulator suppresses a cancer phenotype, e.g. to a normal tissue fingerprint. In another embodiment, a modulator induced a cancer phenotype. Generally, a plurality of assay mixtures is run in parallel with different agent concentrations to obtain a differential response to the various concentrations. Typically, one of these concentrations serves as a negative control, i.e., at zero concentration or below the level of detection.

Modulators, drug candidates or test compounds encompass numerous chemical classes, though typically they are organic molecules, preferably small organic compounds having a molecular weight of more than 100 and less than about 2,500 Daltons. Preferred small molecules are less than 2000, or less than 1500 or less than 1000 or less than 500 D. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Modulators also comprise biomolecules such as peptides, saccharides, fatty acids, steroids, purines, pyrimidines, derivatives, structural analogs or combinations thereof. Particularly preferred are peptides. One class of modulators are peptides, for example of from about five to about 35 amino acids, with from about five to about 20 amino acids being preferred, and from about 7 to about 15 being particularly preferred. Preferably, the cancer modulatory protein is soluble, includes a non-transmembrane region, and/or, has an N-terminal Cys to aid in solubility. In one embodiment, the C-terminus of the fragment is kept as a free acid and the N-terminus is a free amine to aid in coupling, i.e., to cysteine. In one embodiment, a cancer protein of the invention is conjugated to an immunogenic agent as discussed herein. In one embodiment, the cancer protein is conjugated to BSA. The peptides of the invention, e.g., of preferred lengths, can be linked to each other or to other amino acids to create a longer peptide/protein. The modulatory peptides can be digests of naturally occurring proteins as is outlined above, random peptides, or "biased" random peptides. In a preferred embodiment, peptide/protein-based modulators are antibodies, and fragments thereof, as defined herein.

Modulators of cancer can also be nucleic acids. Nucleic acid modulating agents can be naturally occurring nucleic acids, random nucleic acids, or "biased" random nucleic acids. For example, digests of prokaryotic or eukaryotic genomes can be used in an approach analogous to that outlined above for proteins.

The term "monoclonal antibody" refers to an antibody obtained from a population of substantially homogeneous antibodies, i.e., the antibodies comprising the population are identical except for possible naturally occurring mutations that are present in minor amounts.

A "motif", as in biological motif of a 58P1D12-related protein, refers to any pattern of amino acids forming part of the primary sequence of a protein, that is associated with a particular function (e.g. protein-protein interaction, protein-DNA interaction, etc) or modification (e.g. that is phosphorylated, glycosylated or amidated), or localization (e.g. secretory sequence, nuclear localization sequence, etc.) or a sequence that is correlated with being immunogenic, either humorally or cellularly. A motif can be either contiguous or capable of being aligned to certain positions that are generally correlated with a certain function or property. In the context of HLA motifs, "motif" refers to the pattern of residues in a peptide of defined length, usually a peptide of from about 8 to about 13 amino acids for a class I HLA motif and from about 6 to about 25 amino acids for a class II HLA motif, which is recognized by a particular HLA molecule. Peptide motifs for HLA binding are typically different for each protein encoded by each human HLA allele and differ in the pattern of the primary and secondary anchor residues.

A "pharmaceutical excipient" comprises a material such as an adjuvant, a carrier, pH-adjusting and buffering agents, tonicity adjusting agents, wetting agents, preservative, and the like.

"Pharmaceutically acceptable" refers to a non-toxic, inert, and/or composition that is physiologically compatible with humans or other mammals.

The term "polynucleotide" means a polymeric form of nucleotides of at least 10 bases or base pairs in length, either ribonucleotides or deoxynucleotides or a modified form of either type of nucleotide, and is meant to include single and double stranded forms of DNA and/or RNA. In the art, this term if often used interchangeably with "oligonucleotide". A polynucleotide can comprise a nucleotide sequence disclosed herein wherein thymidine (T), as shown for example in Figure 2, can also be uracil (U); this definition pertains to the differences between the chemical structures of DNA and RNA, in particular the observation that one of the four major bases in RNA is uracil (U) instead of thymidine (T).

The term "polypeptide" means a polymer of at least about 4, 5, 6, 7, or 8 amino acids. Throughout the specification, standard three letter or single letter designations for amino acids are used. In the art, this term is often used interchangeably with "peptide" or "protein".

An HLA "primary anchor residue" is an amino acid at a specific position along a peptide sequence which is understood to provide a contact point between the immunogenic peptide and the HLA molecule. One to three, usually two, primary anchor residues within a peptide of defined length generally defines a "motif" for an immunogenic peptide. These residues are understood to fit in close contact with peptide binding groove of an HLA molecule, with their side chains buried in specific pockets of the binding groove. In one embodiment, for example, the primary anchor residues for an HLA class I molecule are located at position 2 (from the amino terminal position) and at the carboxyl terminal position of a 8, 9, 10, 11, or 12 residue peptide epitope in accordance with the invention. Alternatively, in another embodiment, the primary anchor residues of a peptide binds an HLA class II molecule are spaced relative to each other, rather than to the termini of a peptide, where the peptide is generally of at least 9 amino acids in length. The primary anchor positions for each motif and supermotif are set forth in Table IV. For example, analog peptides can be created by altering the presence or absence of particular residues in the primary and/or secondary anchor positions shown in Table IV. Such analogs are used to modulate the binding affinity and/or population coverage of a peptide comprising a particular HLA motif or supermotif.

"Radioisotopes" include, but are not limited to the following (non-limiting exemplary uses are also set forth):

### Examples of Medical Isotopes:

| Isotope | Description of use |
|---|---|
| Actinium-225 (AC-225) | See Thorium-229 (Th-229) |
| Actinium-227 (AC-227) | Parent of Radium-223 (Ra-223) which is an alpha emitter used to treat metastases in the skeleton resulting from cancer (i.e., breast and prostate cancers), and cancer radioimmunotherapy |
| Bismuth-212 (Bi-212) | See Thorium-228 (Th-228) |
| Bismuth-213 (Bi-213) | See Thorium-229 (Th-229) |
| Cadmium-109 (Cd-109) | Cancer detection |
| Cobalt-60 (Co-60) | Radiation source for radiotherapy of cancer, for food irradiators, and for sterilization of medical supplies |
| Copper-64 (Cu-64) | A positron emitter used for cancer therapy and SPECT imaging |
| Copper-67 (Cu-67) | Beta/gamma emitter used in cancer radioimmunotherapy and diagnostic studies (i.e., breast and colon cancers, and lymphoma) |
| Dysprosium-166 (Dy-166) | Cancer radioimmunotherapy |
| Erbium-169 (Er-169) | Rheumatoid arthritis treatment, particularly for the small joints associated with fingers and toes |
| Europium-152 (Eu-152) | Radiation source for food irradiation and for sterilization of medical supplies |
| Europium-154 (Eu-154) | Radiation source for food irradiation and for sterilization of medical supplies |
| Gadolinium-153 (Gd-153) | Osteoporosis detection and nuclear medical quality assurance devices |
| Gold-198 (Au-198) | Implant and intracavity therapy of ovarian, prostate, and brain cancers |
| Holmium-166 (Ho-166) | Multiple myeloma treatment in targeted skeletal therapy, cancer radioimmunotherapy, bone marrow ablation, and rheumatoid arthritis treatment |
| Iodine-125 (I-125) | Osteoporosis detection, diagnostic imaging, tracer drugs, brain cancer treatment, radiolabeling, tumor imaging, mapping of receptors in the brain, interstitial radiation therapy, brachytherapy for treatment of prostate cancer, determination of glomerular filtration rate (GFR), determination of plasma volume, detection of deep vein thrombosis of the legs |
| Iodine-131 (I-131) | Thyroid function evaluation, thyroid disease detection, treatment of thyroid cancer as well as other non-malignant thyroid diseases (i.e., Graves disease, goiters, and hyperthyroidism), treatment of leukemia, lymphoma, and other forms of cancer (e.g., breast cancer) using radioimmunotherapy |
| Iridium-192 (Ir-192) | Brachytherapy, brain and spinal cord tumor treatment, treatment of blocked arteries (i.e., arteriosclerosis and restenosis), and implants for breast and prostate tumors |
| Lutetium-177 (Lu-177) | Cancer radioimmunotherapy and treatment of blocked arteries (i.e., arteriosclerosis and restenosis) |
| Molybdenum-99 (Mo-99) | Parent of Technetium-99m (Tc-99m) which is used for imaging the brain, liver, lungs, heart, and other organs. Currently, Tc-99m is the most widely used radioisotope used for diagnostic imaging of various cancers and diseases involving the brain, heart, liver, lungs; also used in detection of deep vein thrombosis of the legs |
| Osmium-194 (Os-194) | Cancer radioimmunotherapy |
| Palladium-103 (Pd-103) | Prostate cancer treatment |
| Platinum-195m (Pt-195m) | Studies on biodistribution and metabolism of cisplatin, a chemotherapeutic drug |
| Phosphorus-32 (P-32) | Polycythemia rubra vera (blood cell disease) and leukemia treatment, bone cancer diagnosis/treatment; colon, pancreatic, and liver cancer treatment; radiolabeling nucleic acids for in vitro research, diagnosis of superficial tumors, treatment of blocked arteries (i.e., arteriosclerosis and restenosis), and intracavity therapy |
| Phosphorus-33 (P-33) | Leukemia treatment, bone disease diagnosis/treatment, radiolabeling, and treatment of blocked arteries (i.e., arteriosclerosis and restenosis) |
| Radium-223 (Ra-223) | See Actinium-227 (Ac-227) |
| Rhenium-186 (Re-186) | Bone cancer pain relief, rheumatoid arthritis treatment, and diagnosis and treatment of lymphoma and bone, breast, colon, and liver cancers using radioimmunotherapy |
| Rhenium-188 (Re-188) | Cancer diagnosis and treatment using radioimmunotherapy, bone cancer pain relief, treatment of rheumatoid arthritis, and treatment of prostate cancer |
| Rhodium-105 (Rh-105) | Cancer radioimmunotherapy |
| Samarium-145 (Sm-145) | Ocular cancer treatment |
| Samarium-153 (Sm-153) | Cancer radioimmunotherapy and bone cancer pain relief |
| Scandium-47 (Sc-47) | Cancer radioimmunotherapy and bone cancer pain relief |
| Selenium-75 (Se-75) | Radiotracer used in brain studies, imaging of adrenal cortex by gamma-scintigraphy, lateral locations of steroid secreting tumors, pancreatic scanning, detection of hyperactive parathyroid glands, measure rate of bile acid loss from the endogenous pool |
| Strontium-85 (Sr-85) | Bone cancer detection and brain scans |
| Strontium-89 (Sr-89) | Bone cancer pain relief, multiple myeloma treatment, and osteoblastic therapy |
| Technetium-99m (Tc-99m) | See Molybdenum-99 (Mo-99) |
| Thorium-228 (Th-228) | Parent of Bismuth-212 (Bi-212) which is an alpha emitter used in cancer radioimmunotherapy |
| Thorium-229 (Th-229) | Parent of Actinium-225 (Ac-225) and grandparent of Bismuth-213 (Bi-213) which are alpha emitters used in cancer radioimmunotherapy |
| Thulium-170 (Tm-170) | Gamma source for blood irradiators, energy source for implanted medical devices |
| Tin-117m (Sn-117m) | Cancer immunotherapy and bone cancer pain relief |
| Tungsten-188 (W-188) | Parent for Rhenium-188 (Re-188) which is used for cancer diagnostics/treatment, bone cancer pain relief, rheumatoid arthritis treatment, and treatment of blocked arteries (i.e., arteriosclerosis and restenosis) |
| Xenon-127 (Xe-127) | Neuroimaging of brain disorders, high resolution SPECT studies, pulmonary function tests, and cerebral blood flow studies |
| Ytterbium-175 (Yb-175) | Cancer radioimmunotherapy |
| Yttrium-90 (Y-90) | Microseeds obtained from irradiating Yttrium-89 (Y-89) for liver cancer treatment |
| Yttrium-91 (Y-91) | A gamma-emitting label for Yttrium-90 (Y-90) which is used for cancer radioimmunotherapy (i.e., lymphoma, breast, colon, kidney, lung, ovarian, prostate, pancreatic, and inoperable liver cancers) |

By "randomized" or grammatical equivalents as herein applied to nucleic adds and proteins is meant that each nucleic add and peptide consists of essentially random nucleotides and amino acids, respectively. These random peptides (or nucleic adds, discussed herein) can incorporate any nucleotide or amino acid at any position. The synthetic process can be designed to generate randomized proteins or nucleic acids, to allow the formation of all or most of the possible combinations over the length of the sequence, thus forming a library of randomized candidate bioactive proteinaceous agents.

In one embodiment, a library is "fully randomized," with no sequence preferences or constants at any position. In another embodiment, the library is a "biased random" library. That is, some positions within the sequence either are held constant, or are selected from a limited number of possibilities. For example, the nucleotides or amino acid residues are randomized within a defined class, e.g., of hydrophobic amino acids, hydrophilic residues, sterically biased (either small or large) residues, towards the creation of nucleic acid binding domains, the creation of cysteines, for cross-linking, prolines for SH-3 domains, serines, threonines, tyrosines or histidines for phosphorylation sites, etc., or to purines, etc.

A "recombinant" DNA or RNA molecule as a DNA or RNA molecule that has been subjected to molecular manipulation in vitro.

Non-limiting examples of small molecules include compounds that bind or interact with 58P1D12, ligands including hormones, neuropeptides, chemokines, odorants, phospholipids, and functional equivalents thereof that bind and preferably inhibit 58P1D12 protein function. Such non-limiting small molecules preferably have a molecular weight of less than about 10 kDa, more preferably below about 9, about 8, about 7, about 6, about 5 or about 4 kDa. In certain embodiments, small molecules physically associate with, or bind, 58P1D12 protein; are not found in naturally occurring metabolic pathways; and/or are more soluble in aqueous than non-aqueous solutions

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured nucleic acid sequences to reanneal when complementary strands are present in an environment below their melting temperature. The higher the degree of desired homology between the probe and hybridizable sequence, the higher the relative temperature that can be used. As a result it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, are identified by, but not limited to, those that (1) employ low ionic strength and high temperature for washing, for example 0.015 M sodium chloride/0.0015 M sodium citrate/0.1% sodium dodecyl sulfate at 50oC; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42 oC; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1 % SDS, and 10% dextran sulfate at 42 oC, with washes at 42oC in 0.2 x SSC (sodium chloride/sodium. citrate) and 50% formamide at 55 oC, followed by a high-stringency wash consisting of 0.1 x SSC containing EDTA at 55 oC. "Moderately stringent conditions" are described by, but not limited to, those in Sambrook et al., Molecular Cloning: A Laboratory Manual, New York: Cold Spring Harbor Press, 1989, and include the use of washing solution and hybridization conditions (e.g., temperature, ionic strength and %SDS) less stringent than those described above. An example of moderately stringent conditions is overnight incubation at 37oC in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/mL denatured sheared salmon sperm DNA, followed by washing the filters in 1 x SSC at about 37-50oC. The skilled artisan will recognize how to adjust the temperature, ionic strength, etc. as necessary to accommodate factors such as probe length and the like.

An HLA "supermotif" is a peptide binding specificity shared by HLA molecules encoded by two or more HLA alleles. Overall phenotypic frequencies of HLA-supertypes in different ethnic populations are set forth In Table IV (F). The non-limiting constituents of various supetypes are as follows:
A2: A*0201, A*0202, A*0203, A*0204, A* 0205, A*0206, A*6802, A*6901, A*0207
A3: A3; A11, A31, A*3301, A*6801, A*0301, A*1101, A*3101
B7: B7, B*3501-03, B*51, B*5301, B*5401, B*5501, B*5502, B*5601, B*6701, B*7801, B*0702, B*5101, B*5602
B44: B*3701, B*4402, B*4403, B*60 (B*4001), B61 (B*4006)
A1: A*0102, A*2604, A*3601, A*4301, A*8001
A24: A*24, A*30, A*2403, A*2404, A*3002, A*3003
B27: B*1401-02, B*1503, B*1509, B*1510, B*1518, B*3801-02, B*3901, B*3902, B*3903-04, B*4801-02, B*7301, B*2701-08
B58: B*1516, B*1517, B*5701, B*5702, B58
B62: B*4601, B52, B*1501 (B62), B*1502 (B75), B*1513 (B77)

Calculated population coverage afforded by different HLA-supertype combinations are set forth in Table IV (G).

As used herein "to treat" or "therapeutic" and grammatical related terms, refer to any improvement of any consequence of disease, such as prolonged survival, less morbidity, and/or a lessening of side effects which are the byproducts of an alternative therapeutic modality; full eradication of disease is not required:

A "transgenic animal" (e.g., a mouse or rat) is an animal having cells that contain a transgene, which transgene was introduced into the animal or an ancestor of the animal at a prenatal, e.g., an embryonic stage. A "transgene" is a DNA that is integrated into the genome of a cell from which a transgenic animal develops.

As used herein, an HLA or cellular immune response "vaccine" is a composition that contains or encodes one or more peptides of the invention. There are numerous embodiments of such vaccines, such as a cocktail of one or more individual peptides; one or more peptides of the invention comprised by a polyepitopic peptide; or nucleic acids that encode such individual peptides or polypeptides, e.g., a minigene that encodes a polyepitopic peptide. The "one or more peptides" can include any whole unit integer from 1-150 or more, e.g., at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 105, 110, 115, 120, 125, 130, 135, 140, 145, or 150 or more peptides of the invention. The peptides or polypeptides can optionally be modified, such as by lipidation, addition of targeting or other sequences. HLA class I peptides of the invention can be admixed with, or linked to, HLA class II peptides, to facilitate activation of both cytotoxic T lymphocytes and helper T lymphocytes. HLA vaccines can also comprise peptide-pulsed antigen presenting cells, e.g., dendritic cells.

The term "variant" refers to a molecule that exhibits a variation from a described type or norm, such as a protein that has one or more different amino acid residues in the corresponding position(s) of a specifically described protein (e.g. the 58P1D12 protein shown in Figure 2 or Figure 3. An analog is an example of a variant protein. Splice isoforms and single nucleotides polymorphisms (SNPs) are further examples of variants.

The "58P1D12-related proteins" include those specifically identified herein, as well as allelic variants, conservative substitution variants, analogs and homologs that can be isolated/generated and characterized without undue experimentation following the methods outlined herein or readily available in the art. Fusion proteins that combine parts of different 58P1D12 proteins or fragments thereof, as well as fusion proteins of a 58P1D12 protein and a heterologous polypeptide are also included. Such 58P1D12 proteins are collectively referred to as the 58P1D12-related proteins, the proteins of the invention, or 58P1 D12. The term "58P1D12-related protein" refers to a polypeptide fragment or a 58P1D12 protein sequence of 4, 5, 6, 7, 8, 9, 10, 11, 12, 13, 14, 15, 16, 17, 18, 19, 20, 21, 22, 23, 24, 25, or more than 25 amino acids; or, at least 30, 35, 40, 45, 50, 55, 60, 65, 70, 80, 85, 90, 95,100, 105, 110, 115, 120, 125, 130, 135, 140, 145, 150, 155, 160, 165, 170, 175, 180, 185, 190, 195, 200, 225, 250, 275, 300, 325, 350, 375, 400, 425, 450, 475, 500, 525, 550, 575, or 576 or more amino acids.

### VII.) Methods for the Detection of 58P1D12

Another aspect of the present invention relates to methods for detecting 58P1D12 protein consisting of the sequence of SEQ ID NO:13 or SEQ ID NO:14 or SEQ ID NO:14 or a 58P1D12 transcription variant polynucleotide encoding a protein consisting of the sequence of SEQ ID NO:13 or SEQ ID NO:14 and 58P1D12-related proteins, as well as methods for identifying a cell that expresses 58P1D12 gene products. "58P1D12 gene products" refers to proteins consisting of the sequence of SEQ ID NO:13 or SEQ ID NO:14 or a 58P1D12 transcription variant RNA encoding a protein consisting of the sequence of SEQ ID NO:13 or SEQ ID NO:14. The expression profile of 58P1D12 makes it a diagnostic marker for metastasized disease. Accordingly, the status of 58P1D12 gene products provides information useful for predicting a variety of factors including susceptibility to advanced stage disease, rate of progression, and/or tumor aggressiveness. As discussed in detail herein, the status of 58P1D12 gene products in patient samples can be analyzed by a variety protocols that are well known in the art including immunohistochemical analysis, the variety of Northern blotting techniques including in situ hybridization, RT-PCR analysis (for example on laser capture micro-dissected samples), Western blot analysis and tissue array analysis.

More particularly, the invention provides assays for the detection of 58P1D12 polynucleotides in a biological sample, such as serum, bone, prostate, and other tissues, urine, semen, cell preparations, and the like. Detectable 58P1D12 polynucleotides include, for example, a 58P1D12 gene or fragment thereof, 58P1D12 mRNA, alternative splice variant 58P1D12 mRNAs, and recombinant DNA or RNA molecules that contain a 58P1D12 polynucleotide. A number of methods for amplifying and/or detecting the presence of 58P1D12 polynucleotides are well known in the art and can be employed in the practice of this aspect of the invention.

In one embodiment, a method for detecting a 58P1D12 mRNA in a biological sample comprises producing cDNA from the sample by reverse transcription using at least one primer; amplifying the cDNA so produced using a 58P1D12 polynucleotides as sense and antisense primers to amplify 58P1D12 cDNAs therein; and detecting the presence of the amplified 58P1D12 cDNA. Optionally, the sequence of the amplified 58P1D12 cDNA can be determined.

In another embodiment, a method of detecting a 58P1D12 gene in a biological sample comprises first isolating genomic DNA from the sample; amplifying the isolated genomic DNA using 58P1D12 polynucleotides as sense and antisense primers; and detecting the presence of the amplified 58P1D12 gene. Any number of appropriate sense and antisense probe combinations can be designed from a 58P1D12 nucleotide sequence (see, e.g., Figure 2) and used for this purpose.

The invention also provides assays for detecting the presence of the 58P1D12 protein in a tissue or other biological sample such as serum, semen, bone, prostate, urine, cell preparations, and the like. Methods for detecting the 58P1D12-related protein are also well known and include, for example, immunoprecipitation, immunohistochemical analysis, Western blot analysis, molecular binding assays, ELISA, ELIFA and the like. For example, a method of detecting the presence of the 58P1D12-related protein in a biological sample comprises first contacting the sample with a 58P1D12 antibody, a 58P1D12-reactive fragment thereof, or a recombinant protein containing an antigen-binding region of a 58P1D12 antibody; and then detecting the binding of 58P1D12-related protein in the sample.

Methods for identifying a cell that expresses 58P1D12 gene products are also within the scope of the invention. In one embodiment, an assay for identifying a cell that expresses a 58P1D12 gene comprises detecting the presence of 58P1D12 mRNA in the cell. Methods for the detection of particular mRNAs in cells are well known and include, for example, hybridization assays using complementary DNA probes (such as in situ hybridization using labeled 58P1D12 riboprobes, Northern blot and related techniques) and various nucleic acid amplification assays (such as RT-PCR using complementary primers specific for 58P1D12, and other amplification type detection methods, such as, for example, branched DNA, SISBA, TMA and the like). Alternatively, an assay for identifying a cell that expresses the 58P1D12 gene comprises detecting the presence of 58P1D12 protein in the cell or secreted by the cell. Various methods for the detection of proteins are well known in the art and are employed for the detection of the 58P1 D12 proteins and cells that express the 58P1 D12 proteins.

58P1D12 expression analysis is also useful as a tool for identifying and evaluating agents that modulate 58P1D12 gene expression. For example, 58P1D12 expression is significantly upregulated in prostate cancer, and is expressed in cancers of the tissues listed in Table I. Identification of a molecule or biological agent that inhibits 58P1D12 expression or over-expression in cancer cells is of therapeutic value. For example, such an agent can be identified by using a screen that quantifies 58P1D12 expression by RT-PCR, nucleic acid hybridization or antibody binding.

### VIII.) Methods for Monitoring the Status of 58P1D12-related Genes and Their Products

Oncogenesis is known to be a multistep process where cellular growth becomes progressively dysregulated and cells progress from a normal physiological state to precancerous and then cancerous states (see, e.g., Alers et al., Lab Invest. 77(5): 437-438 (1997) and Isaacs et al., Cancer Surv. 23: 19-32 (1995)). In this context, examining a biological sample for evidence of dysregulated cell growth (such as aberrant 58P1D12 expression in cancers) allows for early detection of such aberrant physiology, before a pathologic state such as cancer has progressed to a stage that therapeutic options are more limited and or the prognosis is worse. In such examinations, the status of 58P1D12 in a biological sample of interest can be compared, for example, to the status of 58P1D12 in a corresponding normal sample (e.g. a sample from that individual or alternatively another individual that is not affected by a pathology). An alteration in the status of 58P1D12 in the biological sample (as compared to the normal sample) provides evidence of dysregulated cellular growth. In addition to using a biological sample that is not affected by a pathology as a normal sample, one can also use a predetermined normative value such as a predetermined normal level of mRNA expression (see, e.g., Grever et al., J. Comp. Neurol. 1996 Dec 9; 376(2): 306-14 and U.S. Patent No. 5,837,501) to compare 58P1D12 status in a sample.

The term "status" in this context is used according to its art accepted meaning and refers to the condition or state of a gene and its products. Typically, skilled artisans use a number of parameters to evaluate the condition or state of a gene and its products. These include, but are not limited to the location of expressed gene products (including the location of 58P1 D12 expressing cells) as well as the level, and biological activity of expressed gene products (such as 58P1D12 mRNA, polynucleotides and polypeptides). Typically, an alteration in the status of 58P1D12 comprises a change in the location of 58P1D12 and/or 58P1D12 expressing cells and/or an increase in 58P1D12 mRNA and/or protein expression.

58P1D12 status in a sample can be analyzed by a number of means well known in the art, including without limitation, immunohistochemical analysis, in situ hybridization, RT-PCR analysis on laser capture micro-dissected samples, Western blot analysis, and tissue array analysis. Typical protocols for evaluating the status of a 58P1D12 gene and gene products are found, for example in Ausubel et al. eds., 1995, Current Protocols In Molecular Biology, Units 2 (Northern Blotting), 4 (Southern Blotting), 15 (Immunoblotting) and 18 (PCR Analysis). Thus, the status of 58P1D12 in a biological sample is evaluated by various methods utilized by skilled artisans including, but not limited to genomic Southern analysis (to examine, for example perturbations in a 58P1D12 gene), Northern analysis and/or PCR analysis of 58P1D12 mRNA (to examine, for example alterations in the polynucleotide sequences or expression levels of 58P1D12 mRNAs), and, Western and/or immunohistochemical analysis (to examine, for example alterations in polypeptide sequences, alterations in polypeptide localization within a sample, alterations in expression levels of 58P1D12 proteins and/or associations of 58P1D12 proteins with polypeptide binding partners). Detectable 58P1D12 polynucleotides include, for example, a 58P1D12 gene or fragment thereof, 58P1D12 mRNA, alternative splice variants, 58P1D12 mRNAs, and recombinant DNA or RNA molecules containing a 58P1 D12 polynucleotide.

The expression profile of 58P1 D12 makes it a diagnostic marker for local and/or metastasized disease, and provides information on the growth or oncogenic potential of a biological sample. In particular, the status of 58P1D12 provides information useful for predicting susceptibility to particular disease stages, progression, and/or tumor aggressiveness. The invention provides methods and assays for determining 58P1D12 status and diagnosing cancers that express 58P1D12, such as cancers of the tissues listed in Table I. For example, because 58P1D12 mRNA is so highly expressed in prostate and other cancers relative to normal prostate tissue, assays that evaluate the levels of 58P1D12 mRNA transcripts or proteins in a biological sample can be used to diagnose a disease associated with 58P1D12 dysregulation, and can provide prognostic information useful in defining appropriate therapeutic options.

The expression status of 58P1D12 provides information including the presence, stage and location of dysplastic, precancerous and cancerous cells, predicting susceptibility to various stages of disease, and/or for gauging tumor aggressiveness. Moreover, the expression profile makes it useful as an imaging reagent for metastasized disease. Consequently, an aspect of the invention is directed to the various molecular prognostic and diagnostic methods for examining the status of 58P1D12 in biological samples such as those from individuals suffering from, or suspected of suffering from a pathology characterized by dysregulated cellular growth, such as cancer.

As described above, the status of 58P1D12 in a biological sample can be examined by a number of well-known procedures in the art. For example, the status of 58P1D12 in a biological sample taken from a specific location in the body can be examined by evaluating the sample for the presence or absence of 58P1D12 expressing cells (e.g. those that express 58P1D12 mRNAs or proteins). This examination can provide evidence of dysregulated cellular growth, for example, when 58P1D12-expressing cells are found in a biological sample that does not normally contain such cells (such as a lymph node), because such alterations in the status of 58P1D12 in a biological sample are often associated with dysregulated cellular growth. Specifically, one indicator of dysregulated cellular growth is the metastases of cancer cells from an organ of origin (such as the prostate) to a different area of the body (such as a lymph node). In this context, evidence of dysregulated cellular growth is important for example because occult lymph node metastases can be detected in a substantial proportion of patients with prostate cancer, and such metastases are associated with known predictors of disease progression (see, e.g., Murphy et al., Prostate 42(4): 315-317 (2000);Su et al., Semin. Surg. Oncol. 18(1): 17-28 (2000) and Freeman et al., J Urol 1995 Aug 154(2 Pt 1):474-8).

In one aspect, the invention provides methods for monitoring 58P1D12 gene products by determining the status of 58P1D12 gene products expressed by cells from an individual suspected of having a disease associated with dysregulated cell growth (such as hyperplasia or cancer) and then comparing the status so determined to the status of 58P1D12 gene products in a corresponding normal sample. The presence of aberrant 58P1D12 gene products in the test sample relative to the normal sample provides an indication of the presence of dysregulated cell growth within the cells of the individual.

In another aspect, the invention provides assays useful in determining the presence of cancer in an individual, comprising detecting a significant increase in the 58P1D12 mRNA encoding a protein consisting of the sequence of SEO ID NO:13 or SEQ ID NO:14 or protein consisting of the sequence of SEQ ID NO:13 or SEQ ID NO:14 expression in a test cell or tissue sample relative to expression levels in the corresponding normal cell or tissue. The presence of the 58P1D12 mRNA can, for example, be evaluated in tissues including but not limited to those listed in Table I. The presence of significant expression of 58P1D1 mRNA in any of these tissues is useful to indicate the emergence, presence and/or severity of a cancer, since the corresponding normal tissues do not express 58P1D12 mRNA or express it at lower levels.

In a related embodiment, 58P1D12 status is determined at the protein level rather than at the nucleic acid level. For example, such a method comprises determining the level of the 58P1D12 protein expressed by cells in a test tissue sample and comparing the level so determined to the level of the 58P1D12 protein expressed in a corresponding normal sample. In one embodiment, the presence of the 58P1D12 protein is evaluated, for example, using immunohistochemical methods. 58P1D12 antibodies or binding partners capable of detecting the 58P1D12 protein expression are used in a variety of assay formats well known in the art for this purpose.

In a further embodiment, one can evaluate the status of the 58P1D12 polynucleotide and amino acid sequences in a biological sample in order to identify perturbations in the structure of these molecules. These perturbations can include insertions, deletions, substitutions and the like. Such evaluations are useful because perturbations in the nucleotide and amino acid sequences are observed in a large number of proteins associated with a growth dysregulated phenotype (see, e.g., Marrogi et al., 1999, J. Cutan. Pathol. 26(8):369-378). For example, a mutation in the sequence of 58P1D12 may be indicative of the presence or promotion of a tumor. Such assays therefore have diagnostic and predictive value where a mutation in 58P1D12 indicates a potential loss of function or increase in tumor growth.

A wide variety of assays for observing perturbations in nucleotide and amino acid sequences are well known in the art. For example, the size and structure of nucleic acid or amino acid sequences of the 58P1D12 gene products are observed by the Northern, Southern, Western, PCR and DNA sequencing protocols discussed herein. In addition, other methods for observing perturbations in nucleotide and amino acid sequences such as single strand conformation polymorphism analysis are well known in the art (see, e.g., U.S. Patent Nos. 5,382,510 issued 7 September 1999, and 5,952,170 issued 17 January 1995).

Additionally, one can examine the methylation status of a 58P1D12 gene in a biological sample. Aberrant demethylation and/or hypermethylation of CpG islands in gene 5' regulatory regions frequently occurs in immortalized and transformed cells, and can result in altered expression of various genes. For example, promoter hypermethylation of the pi-class glutathione S-transferase (a protein expressed in normal prostate but not expressed in >90% of prostate carcinomas) appears to permanently silence transcription of this gene and is the most frequently detected genomic alteration in prostate carcinomas (De Marzo et al., Am. J. Pathol. 155(6): 1985-1992 (1999)). In addition, this alteration is present in at least 70% of cases of high-grade prostatic intraepithelial neoplasia (PIN) (Brooks et al., Cancer Epidemiol. Biomarkers Prev., 1998, 7:531-536). In another example, expression of the LAGE-I tumor specific gene (which is not expressed in normal prostate but is expressed in 25-50% of prostate cancers) is induced by deoxy-azacytidine in lymphoblastoid cells, suggesting that tumoral expression is due to demethylation (Lethe et al., Int. J. Cancer 76(6): 903-908 (1998)). A variety of assays for examining methylation status of a gene are well known in the art. For example, one can utilize, in Southern hybridization approaches, methylation-sensitive restriction enzymes that cannot cleave sequences that contain methylated CpG sites to assess the methylation status of CpG islands. In addition, MSP (methylation specific PCR) can rapidly profile the methylation status of all the CpG sites present in a CpG island of a given gene. This procedure involves initial modification of DNA by sodium bisulfite (which will convert all unmethylated cytosines to uracil) followed by amplification using primers specific for methylated versus unmethylated DNA. Protocols involving methylation interference can also be found for example in Current Protocols In Molecular Biology, Unit 12, Frederick M. Ausubel et al. eds., 1995.

Gene amplification is an additional method for assessing the status of 58P1D12. Gene amplification is measured in a sample directly, for example, by conventional Southern blotting or Northern blotting to quantitate the transcription of mRNA (Thomas, 1980, Proc. Natl. Acad. Sci. USA, 77:5201-5205), dot blotting (DNA analysis), or in situ hybridization, using an appropriately labeled probe, based on the sequences provided herein. Alternatively, antibodies are employed that recognize specific duplexes, including DNA duplexes, RNA duplexes, and DNA-RNA hybrid duplexes or DNA-protein duplexes. The antibodies in turn are labeled and the assay carried out where the duplex is bound to a surface, so that upon the formation of duplex on the surface, the presence of antibody bound to the duplex can be detected.

Biopsied tissue or peripheral blood can be conveniently assayed for the presence of cancer cells using for example, Northern, dot blot or RT-PCR analysis to detect 58P1D12 expression. The presence of RT-PCR amplifiable 58P1D12 mRNA provides an indication of the presence of cancer. RT-PCR assays are well known in the art. RT-PCR detection assays for tumor cells in peripheral blood are currently being evaluated for use in the diagnosis and management of a number of human solid tumors. In the prostate cancer field, these include RT-PCR assays for the detection of cells expressing PSA and PSM (Verkaik et al., 1997, Urol. Res. 25:373-384; Ghossein et al., 1995, J. Clin. Oncol. 13:1195-2000; Heston et al., 1995, Clin. Chem. 41:1687-1688).

A further aspect of the invention is an assessment of the susceptibility that an individual has for developing cancer. In one embodiment, a method for predicting susceptibility to cancer comprises detecting 58P1D12 mRNA or 58P1D12 protein in a tissue sample, its presence indicating susceptibility to cancer, wherein the degree of 58P1D12 mRNA expression correlates to the degree of susceptibility. In a specific embodiment, the presence of 58P1D12 in prostate or other tissue is examined, with the presence of 58P1D12 in the sample providing an indication of prostate cancer susceptibility (or the emergence or existence of a prostate tumor). Similarly, one can evaluate the integrity 58P1D12 nucleotide and amino acid sequences in a biological sample, in order to identify perturbations in the structure of these molecules such as insertions, deletions, substitutions and the like. The presence of one or more perturbations in 58P1D12 gene products in the sample is an indication of cancer susceptibility (or the emergence or existence of a tumor).

The invention also comprises methods for gauging tumor aggressiveness. In one embodiment, a method for gauging aggressiveness of a tumor comprises determining the level of 58P1D12 mRNA encoding a protein consisting of the sequence of SEQ ID NO:13 or SEQ ID NO:14 or 58P1D12 protein consisting of the sequence of SEQ ID NO:13 or SEQ ID NO:14 protein expressed by tumor cells, comparing the level so determined to the level of the 58P1D12 mRNA or the 58P1D12 protein expressed in a corresponding normal tissue taken from the same individual or a normal tissue reference sample, wherein the degree of 58P1D12 mRNA or 58P1D12 protein expression in the tumor sample relative to the normal sample indicates the degree of aggressiveness. In a specific embodiment, aggressiveness of a tumor is evaluated by determining the extent to which 58P1D12 is expressed in the tumor cells, with higher expression levels indicating more aggressive tumors. Another embodiment is the evaluation of the integrity of 58P1D12 nucleotide and amino acid sequences in a biological sample, in order to identify perturbations in the structure of these molecules such as insertions, deletions, substitutions and the like. The presence of one or more perturbations indicates more aggressive tumors.

Another embodiment of the invention is directed to methods for observing the progression of a malignancy in an individual over time. In one embodiment, methods for observing the progression of a malignancy in an individual over time comprise determining the level of the 58P1D12 mRNA or the 58P1D12 protein expressed by cells in a sample of the tumor, comparing the level so determined to the level of the 58P1D12 mRNA or the 58P1D12 protein expressed in an equivalent tissue sample taken from the same individual at a different time, wherein the degree of the 58P1D12 mRNA or the 58P1D12 protein expression in the tumor sample over time provides information on the progression of the cancer. In a specific embodiment, the progression of a cancer is evaluated by determining 58P1D12 expression in the tumor cells over time, where increased expression over time indicates a progression of the cancer. Also, one can evaluate the integrity the 58P1D12 nucleotide and amino acid sequences in a biological sample in order to identify perturbations in the structure of these molecules such as insertions, deletions, substitutions and the like, where the presence of one or more perturbations indicates a progression of the cancer.

The above diagnostic approaches can be combined with any one of a wide variety of prognostic and diagnostic protocols known in the art. For example, another embodiment of the invention is directed to methods for observing a coincidence between the expression of 58P1D12 gene and 58P1D12 gene products (or perturbations in 58P1D12 gene and 58P1D12 gene products) and a factor that is associated with malignancy, as a means for diagnosing and prognosticating the status of a tissue sample. A wide variety of factors associated with malignancy can be utilized, such as the expression of genes associated with malignancy (e.g. PSA, PSCA and PSM expression for prostate cancer etc.) as well as gross cytological observations (see, e.g., Bocking et al., 1984, Anal. Quant. Cytol. 6(2):74-88; Epstein, 1995, Hum. Pathol. 26(2):223-9; Thorson et al., 1998, Mod. Pathol. 11(6):543-51; Baisden et al., 1999, Am. J. Surg. Pathol. 23(8):918-24). Methods for observing a coincidence between the expression of 58P1D12 gene and 58P1D12 gene products (or perturbations in 58P1D12 gene and 58P1D12 gene products) and another factor that is associated with malignancy are useful, for example, because the presence of a set of specific factors that coincide with disease provides information crucial for diagnosing and prognosticating the status of a tissue sample.

In one embodiment, methods for observing a coincidence between the expression of the 58P1D12 gene and the 58P1D12 gene products (or perturbations in the 58P1D12 gene and the 58P1D12 gene products) and another factor associated with malignancy entails detecting the overexpression of the 58P1D12 mRNA or protein in a tissue sample, detecting the overexpression of PSA mRNA or protein in a tissue sample (or PSCA or PSM expression), and observing a coincidence of the 58P1D12 mRNA or protein and PSA mRNA or protein overexpression (or PSCA or PSM expression). In a specific embodiment, the expression of the 58P1 D12 and PSA mRNA in prostate tissue is examined, where the coincidence of 58P1 D12 and PSA mRNA overexpression in the sample indicates the existence of prostate cancer, prostate cancer susceptibility or the emergence or status of a prostate tumor.

Methods for detecting and quantifying the expression of 58P1D12 mRNA or protein are described herein, and standard nucleic acid and protein detection and quantification technologies are well known in the art. Standard methods for the detection and quantification of the 58P1D12 mRNA include in situ hybridization using labeled 58P1D12 riboprobes, Northern blot and related techniques using 58P1D12 polynucleotide probes, RT-PCR analysis using primers specific for the 58P1D12 mRNA, and other amplification type detection methods, such as, for example, branched DNA, SISBA, TMA and the like. In a specific embodiment, semi-quantitative RT-PCR is used to detect and quantify 58P1D12 mRNA expression. Any number of primers capable of amplifying 58P1D12 can be used for this purpose, including but not limited to the various primer sets specifically described herein. In a specific embodiment, polyclonal or monoclonal antibodies specifically reactive with the wild-type 58P1D12 protein can be used in an immunohistochemical assay of biopsied tissue.

### XI.) Diagnostic and Prognostic Embodiments of 58P1D12.

As disclosed herein, the 58P1D12 protein consisting of the sequence of SEQ ID NO:13 or SEQ ID NO:14 and SEQ ID NO:14 or a 58P1D12 transcription variant polynucleotide encoding a protein consisting of the sequence of SEQ ID NO:13 or SEQ ID NO:14 are used in well known diagnostic and prognostic and therapeutic assays that examine conditions associated with dysregulated cell growth such as cancer, in particular the cancers listed in Table I (see, e.g., both its specific pattern of tissue expression as well as its overexpression in certain cancers as described for example in the Example entitled "Expression analysis of 58P1D12 in normal tissues, and patient specimens").

58P1D12 can be analogized to a prostate associated antigen PSA, the archetypal marker that has been used by medical practitioners for years to identify and monitor the presence of prostate cancer (see, e.g., Merrill et al., J. Urol. 163(2): 503-5120 (2000); Polascik et al., J. Urol. Aug; 162(2):293-306 (1999) and Fortier et al., J. Nat. Cancer Inst. 91(19): 1635-1640(1999)). A variety of other diagnostic markers are also used in similar contexts including p53 and K-ras (see, e.g., Tulchinsky et al., Int J Mol Med 1999 Jul 4(1):99-102 and Minimoto et al., Cancer Detect Prev 2000;24(1):1-12). Therefore, this disclosure of 58P1D12 polynucleotides and polypeptides (as well as 58P1D12 polynucleotide probes and anti-58P1D12 antibodies used to identify the presence of these molecules) and their properties allows skilled artisans to utilize these molecules in methods that are analogous to those used, for example, in a variety of diagnostic assays directed to examining conditions associated with cancer.

Typical embodiments of diagnostic methods which utilize the 58P1D12 protein and polynucleotides, are analogous to those methods from well-established diagnostic assays, which employ, e.g., PSA protein and polynucleotides, polypeptides, reactive T cells and antibodies. For example, just as PSA polynucleotides are used as probes (for example in Northern analysis, see, e.g., Sharief et al., Biochem. Mol. Biol. Int. 33(3):567-74(1994)) and primers (for example in PCR analysis, see, e.g., Okegawa et al., J. Urol. 163(4): 1189-1190 (2000)) to observe the presence and/or the level of PSA mRNAs in methods of monitoring PSA overexpression or the metastasis of prostate cancers, the 58P1D12 polynucleotides described herein can be utilized in the same way to detect 58P1D12 overexpression or the metastasis of prostate and other cancers expressing this gene. Alternatively, just as PSA polypeptides are used to generate antibodies specific for PSA which can then be used to observe the presence and/or the level of PSA proteins in methods to monitor PSA protein overexpression (see, e.g., Stephan et al., Urology 55(4):560-3 (2000)) or the metastasis of prostate cells (see, e.g., Alanen et al., Pathol. Res. Pract. 192(3):233-7 (1996)), the 58P1D12 polypeptides described herein can be utilized to generate antibodies for use in detecting 58P1D12 overexpression or the metastasis of prostate cells and cells of other cancers expressing this gene.

Specifically, because metastases involves the movement of cancer cells from an organ of origin (such as the lung or prostate gland etc.) to a different area of the body (such as a lymph node), assays which examine a biological sample for the presence of cells expressing 58P1D12 polynucleotides and/or polypeptides can be used to provide evidence of metastasis. For example, when a biological sample from tissue that does not normally contain 58P1D12-expressing cells (lymph node) is found to contain 58P1D12-expressing cells such as the 58P1D12 expression seen in LAPC4 and LAPC9, xenografts isolated from lymph node and bone metastasis, respectively, this finding is indicative of metastasis.

Alternatively 58P1 D12 polynucleotides and/or polypeptides can be used to provide evidence of cancer, for example, when cells in a biological sample that do not normally express 58P1 D12 or express 58P1 D12 at a different level are found to express 58P1 D12 or have an increased expression of 58P1 D12 (see, e.g., the 58P1D1 expression in the cancers listed in Table I and in patient samples etc. shown in the accompanying Figures). In such assays, artisans may further wish to generate supplementary evidence of metastasis by testing the biological sample for the presence of a second tissue restricted marker (in addition to 58P1D12) such as PSA, PSCA etc. (see, e.g., Alanen et al., Pathol. Res. Pract. 192(3): 233-237 (1996)).

The use of immunohistochemistry to identify the presence of a 58P1 D12 polypeptide within a tissue section can indicate an altered state of certain cells within that tissue. It is well understood in the art that the ability of an antibody to localize to a polypeptide that is expressed in cancer cells is a way of diagnosing presence of disease, disease stage, progression and/or tumor aggressiveness. Such an antibody can also detect an altered distribution of the polypeptide within the cancer cells, as compared to corresponding non-malignant tissue.

The 58P1D12 polypeptide and immunogenic compositions are also useful in view of the phenomena of altered subcellular protein localization in disease states. Alteration of cells from normal to diseased state causes changes in cellular morphology and is often associated with changes in subcellular protein localization/distribution. For example, cell membrane proteins that are expressed in a polarized manner in normal cells can be altered in disease, resulting in distribution of the protein in a non-polar manner over the whole cell surface.

The phenomenon of altered subcellular protein localization in a disease state has been demonstrated with MUC1 and Her2 protein expression by use of immunohistochemical means. Normal epithelial cells have a typical apical distribution of MUC1, in addition to some supranuclear localization of the glycoprotein, whereas malignant lesions often demonstrate an apolar staining pattern (Diaz et al, The Breast Journal, 7; 40-45 (2001); Zhang et al, Clinical Cancer Research, 4; 2669-2676 (1998): Cao, et al, The Journal of Histochemistry and Cytochemistry, 45: 1547-1557 (1997)). In addition, normal breast epithelium is either negative for Her2 protein or exhibits only a basolateral distribution whereas malignant cells can express the protein over the whole cell surface (De Potter, et al, International Journal of Cancer, 44; 969-974 (1989): McCormick, et al, 117; 935-943 (2002)). Alternatively, distribution of the protein may be altered from a surface only localization to include diffuse cytoplasmic expression in the diseased state. Such an example can be seen with MUC1 (Diaz, et al, The Breast Journal, 7: 40-45 (2001)).

Alteration in the localization/distribution of a protein in the cell, as detected by immunohistochemical methods, can also provide valuable information concerning the favorability of certain treatment modalities. This last point is illustrated by a situation where a protein may be intracellular in normal tissue, but cell surface in malignant cells; the cell surface location makes the cells favorably amenable to antibody-based diagnostic and treatment regimens. When such an alteration of protein localization occurs for 58P1D12, the 58P1D12 protein and immune responses related thereto are very useful. Accordingly, the ability to determine whether alteration of subcellular protein localization occurred for 24P4C12 make the 58P1D12 protein and immune responses related thereto very useful. Use of the 58P1D12 compositions allows those skilled in the art to make important diagnostic and therapeutic decisions.

Immunohistochemical reagents specific to 58P1D12 are also useful to detect metastases of tumors expressing 58P1D12 when the polypeptide appears in tissues where 58P1D12 is not normally produced.

Thus, 58P1D12 polypeptides and antibodies resulting from immune responses thereto are useful in a variety of important contexts such as diagnostic, prognostic, preventative and/or therapeutic purposes known to those skilled in the art.

Just as PSA polynucleotide fragments and polynucleotide variants are employed by skilled artisans for use in methods of monitoring PSA, 58P1D12 polynucleotide fragments and polynucleotide variants are used in an analogous manner. In particular, typical PSA polynucleotides used in methods of monitoring PSA are probes or primers which consist of fragments of the PSA cDNA sequence. Illustrating this, primers used to PCR amplify a PSA polynucleotide must include less than the whole PSA sequence to function in the polymerase chain reaction. In the context of such PCR reactions, skilled artisans generally create a variety of different polynucleotide fragments that can be used as primers in order to amplify different portions of a polynucleotide of interest or to optimize amplification reactions (see, e.g., Caetano-Anolles, G. Biotechniques 25(3): 472-476, 478-480 (1998); Robertson et al., Methods Mol. Biol. 98:121-154 (1998)). An additional illustration of the use of such fragments is provided in the Example entitled "Expression analysis of 58P1D12 in normal tissues, and patient specimens," where a 58P1D12 polynucleotide fragment is used as a probe to show the expression of 58P1D12 RNAs in cancer cells. In addition, variant polynucleotide sequences are typically used as primers and probes for the corresponding mRNAs in PCR and Northern analyses (see, e.g., Sawai et al., Fetal Diagn. Ther. 1996 Nov-Dec 11(6):407-13 and Current Protocols In Molecular Biology, Volume 2, Unit 2, Frederick M. Ausubel et al. eds., 1995)). Polynucleotide fragments and variants are useful in this context where they are capable of binding to a target polynucleotide sequence (e.g., a 58P1D12 polynucleotide shown in Figure 2 or variant thereof) under conditions of high stringency.

Furthermore, PSA polypeptides which contain an epitope that can be recognized by an antibody or T cell that specifically binds to that epitope are used in methods of monitoring PSA. 58P1D12 polypeptide fragments and polypeptide analogs or variants can also be used in an analogous manner. This practice of using polypeptide fragments or polypeptide variants to generate antibodies (such as anti-PSA antibodies or T cells) is typical in the art with a wide variety of systems such as fusion proteins being used by practitioners (see, e.g., Current Protocols In Molecular Biology, Volume 2, Unit 16, Frederick M. Ausubel et al. eds., 1995). In this context, each epitope(s) functions to provide the architecture with which an antibody or T cell is reactive. Typically, skilled artisans create a variety of different polypeptide fragments that can be used in order to generate immune responses specific for different portions of a polypeptide of interest (see, e.g., U.S. Patent No. 5,840,501 and U.S. Patent No. 5,939,533). For example it may be preferable to utilize a polypeptide comprising one of the 58P1D12 biological motifs discussed herein or a motif-bearing subsequence which is readily identified by one of skill in the art based on motifs available in the art. Polypeptide fragments, variants or analogs are typically useful in this context as long as they comprise an epitope capable of generating an antibody or T cell specific for a target polypeptide sequence (e.g. a 58P1D12 polypeptide shown in Figure 3).

As shown herein, the 58P1D12 polynucleotides and polypeptides (as well as the 58P1D12 polynucleotide probes and anti-58P1 D12 antibodies or T cells used to identify the presence of these molecules) exhibit specific properties that make them useful in diagnosing cancers such as those listed in Table I. Diagnostic assays that measure the presence of 58P1D12 gene products, in order to evaluate the presence or onset of a disease condition described herein, such as prostate cancer, are used to identify patients for preventive measures or further monitoring, as has been done so successfully with PSA. Moreover, these materials satisfy a need in the art for molecules having similar or complementary characteristics to PSA in situations where, for example, a definite diagnosis of metastasis of prostatic origin cannot be made on the basis of a test for PSA alone (see, e.g., Alanen et al., Pathol. Res. Pract. 192(3): 233-237 (1996)), and consequently, materials such as 58P1D12 polynucleotides and polypeptides (as well as the 58P1D12 polynucleotide probes and anti-58P1D12 antibodies used to identify the presence of these molecules) need to be employed to confirm a metastases of prostatic origin.

### Examples:

Various aspects of the invention are further described and illustrated by way of the several examples that follow, none of which is intended to limit the scope of the invention.

### Example 1

### SSH-Generated Isolation of a cDNA Fragment of the 58P1D12 Gene

To isolate genes that are over-expressed in prostate cancer we used the Suppression Subtractive Hybridization (SSH) procedure using cDNA derived from prostate cancer tissues. The 58P1D12 SSH DNA sequence was identified from an SSH experiment consisting of LAPC-9AD cDNA minus LAPC-9AI cDNA.

### Materials and Methods

### Human Tissues:

The patient cancer and normal tissues were purchased from different sources such as the NDRI (Philadelphia, PA).

mRNA for some normal tissues were purchased from different companies such as Clontech, Palo Alto, CA.

### RNA Isolation:

Tissues were homogenized in Trizol reagent (Life Technologies, Gibco BRL) using 10 ml/ g tissue to isolate total RNA. Poly A RNA was purified from total RNA using Qiagen's Oligotex mRNA Mini and Midi kits. Total and mRNA were quantified by spectrophotometric analysis (O.D. 260/280 nm) and analyzed by gel electrophoresis.

### Oligonucleotides:

The following HPLC purified oligonucleotides were used.
DPNCDN (cDNA synthesis primer):
   5'TTTTGATCAAGCTT₃₀3' (SEQ ID NO: 22)
Adaptor 1:
   5'CTAATACGACTCACTATAGGGCTCGAGCGGCCGCCCGGGCAG3' (SEQ ID NO: 23)
   3'GGCCCGTCCTAG5' (SEQ ID NO: 24)
Adaptor 2:
   5'GTAATACGACTCACTATAGGGCAGCGTGGTCGCGGCCGAG3' (SEQ ID NO: 25)
   3'CGGCTCCTAG5' (SEQ ID NO: 26)
PCR primer 1:
   5'CTAATACGACTCACTATAGGGC3' (SEQ ID NO: 27)
Nested primer (NP)1:
   5'TCGAGCGGCGGCCCGGGCAGGA3' (SEQ ID NO: 28)
Nested primer (NP)2:
   5'AGCGTGGTCGCGGCCGAGGA3' (SEQ ID NO: 29)

### Suppression Subtractive Hybridization:

Suppression Subtractive Hybridization (SSH) was used to identify cDNAs corresponding to genes that may be differentially expressed in prostate cancer. The SSH reaction utilized cDNA from LAPC-9AD and LAPC-9AI prostate cancer xenograft tissues.

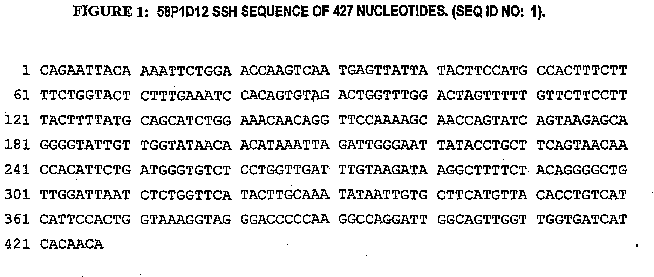

The gene 58P1D12 sequence was derived from the androgen dependent xenograft LAPC-9AD minus the androgen independent xenograft LAPC-9AI cDNA subtraction. The SSH DNA sequence 58P1D12 (Figure 1) was identified.

The cDNA derived from LAPC-9AI was used as the source of the "driver" cDNA, while the cDNA from LAPC-9AD was used as the source of the "tester" cDNA. Double stranded cDNAs corresponding to tester and driver cDNAs were synthesized from 2 µg of poly(A)+ RNA isolated from the relevant xenograft tissue, as described above, using CLONTECH's PCR-Select cDNA Subtraction Kit and 1 ng of oligonucleotide DPNCDN as primer. First- and second-strand synthesis were carried out as described in the Kit's user manual protocol (CLONTECH Protocol No. PT1117-1, Catalog No. K1804-1). The resulting cDNA was digested with Dpn II for 3 hrs at 37°C. Digested cDNA was extracted with phenol/chloroform (1:1) and ethanol precipitated.

Tester cDNA was generated by diluting 1 µl of Dpn II digested cDNA from the relevant tissue source (see above) (400 ng) in 5 µl of water. The diluted cDNA (2 µl, 160 ng) was then ligated to 2 µl of Adaptor 1 and Adaptor 2 (10 µM), in separate ligation reactions, in a total volume of 10 µl at 16°C overnight, using 400 µl of T4 DNA ligase (CLONTECH). Ligation was terminated with 1 µl of 0.2 M EDTA and heating at 72°C for 5 min.

The first hybridization was performed by adding 1.5 µl (600 ng) of driver cDNA to each of two tubes containing 1.5µl (20 ng) Adaptor 1- and Adaptor 2- ligated tester cDNA. In a final volume of 4 µl, the samples were overlaid with mineral oil, denatured in an MJ Research thermal cycler at 98°C for 1.5 minutes, and then were allowed to hybridize for 8 hrs at 68°C. The two hybridizations were then mixed together with an additional 1 µl of fresh denatured driver cDNA and were allowed to hybridize overnight at 68°C. The second hybridization was then diluted in 200 µl of 20 mM Hepes, pH 8.3, 50 mM NaCl, 0.2 mM EDTA, heated at 70°C for 7 min. and stored at -20°C.

PCR Amplification, Cloning and Sequencing of Gene Fragments Generated from SSH:

To amplify gene fragments resulting from SSH reactions, two PCR amplifications were performed. In the primary PCR reaction 1 µl of the diluted final hybridization mix was added to 1 µl of PCR primer 1 (10 µM), 0.5 µl dNTP mix (10 µM), 2.5 µl 10 x reaction buffer (CLONTECH) and 0.5 µl 50 x Advantage cDNA polymerase Mix (CLONTECH) in a final volume of 25 µl. PCR 1 was conducted using the following conditions: 75°C for 5 min., 94°C for 25 sec., then 27 cycles of 94°C for 10 sec, 66°C for 30 sec, 72°C for 1.5 min. Five separate primary PCR reactions were performed for each experiment. The products were pooled and diluted 1:10 with water. For the secondary PCR reaction, 1 µl from the pooled and diluted primary PCR reaction was added to the same reaction mix as used for PCR 1, except that primers NP1 and NP2 (10 µM) were used instead of PCR primer 1. PCR 2 was performed using 10-12 cycles of 94°C for 10 sec, 68°C for 30 sec, and 72°C for 1.5 minutes. The PCR products were analyzed using 2% agarose gel electrophoresis.

The PCR products were inserted into pCR2.1 using the T/A vector cloning kit (Invitrogen). Transformed E. coli were subjected to blue/white and ampicillin selection. White colonies were picked and arrayed into 96 well plates and were grown in liquid culture overnight. To identify inserts, PCR amplification was performed on 1 µl of bacterial culture using the conditions of PCR1 and NP1 and NP2 as primers. PCR products were analyzed using 2% agarose gel electrophoresis.

Bacterial clones were stored in 20% glycerol in a 96 well format. Plasmid DNA was prepared, sequenced, and subjected to nucleic acid homology searches of the GenBank, dBest, and NCI-CGAP databases.

RT-PCR Expression Analysis:

First strand cDNAs can be generated from 1 µg of mRNA with oligo (dT)12-18 priming using the Gibco-BRL Superscript Preamplification system. The manufacturer's protocol was used which included an incubation for 50 min at 42°C with reverse transcriptase followed by RNAse H treatment at 37°C for 20 min. After completing the reaction, the volume can be increased to 200 µl with water prior to normalization. First strand cDNAs from 16 different normal human tissues can be obtained from Clontech.

Normalization of the first strand cDNAs from multiple tissues was performed by using the primers 5'atatcgccgcgctcgtcgtcgacaa3' (SEQ ID NO: 30) and 5'agccacacgcagctcattgtagaagg 3' (SEQ ID NO: 31) to amplify β-actin. First strand cDNA (5 µl) were amplified in a total volume of 50 µl containing 0.4 µM primers, 0.2 µM each dNTPs, 1X PCR buffer (Clontech, 10 mM Tris-HCL, 1.5 mM MgCl₂, 50 mM KCl, pH8.3) and 1X Klentaq DNA polymerase (Clontech). Five µl of the PCR reaction can be removed at 18,20, and 22 cycles and used for agarose gel electrophoresis. PCR was performed using an MJ Research thermal cycler under the following conditions: Initial denaturation can be at 94°C for 15 sec, followed by a 18, 20, and 22 cycles of 94°C for 15, 65°C for 2 min, 72°C for 5 sec. A final extension at 72°C was carried out for 2 min. After agarose gel electrophoresis, the band intensities of the 283 b.p. β-action bands from multiple tissues were compared by visual inspection. Dilution factors for the first strand cDNAs were calculated to result in equal β-actin band intensities in all tissues after 22 cycles of PCR. Three rounds of normalization can be required to achieve equal band intensities in all tissues after 22 cycles of PCR.

To determine expression levels of the 58P1D12 gene, 5 µl of normalized first strand cDNA were analyzed by PCR using 26, and 30 cycles of amplification. Semi-quantitative expression analysis can be achieved by comparing the PCR products at cycle numbers that give light band intensities. The primers used for RT-PCR were designed using the 58P1D12 SSH sequence and are listed below:
[0442] 58P1D12.1
   5'- CCTGCTTCAGTAACAACCACATTCT - 3' (SEQ ID NO: 32)
58P1D12.2
   5'- CTTTACCAGTGGAATGATGACAGG - 3' (SEQ ID NO: 33)

A typical RT-PCR expression analysis is shown in Figure 14.

### Example 2

### Full Length Cloning of 58P1D12

The 58P1D12 SSH sequence of 427 bp (Figure 1) was identified from LAPC xenograft SSH experiment. A full length cDNA clone for 58P1D12 was isolated from an LAPC-9 AD library. The cDNA (clone 2) is 2550 bp in length and encodes a 273 amino acid ORF (Figure 2 and Figure 3). 58P1D12 v.1 exhibited 100% homology to human chondrolectin. Other variants of 58P1D12 were also identified and these are listed in Figure 2 and Figure 3.

### Example 3

### Chromosomal Mapping of 58P1D12

Chromosomal localization can implicate genes in disease pathogenesis. Several chromosome mapping approaches are available including fluorescent *In situ* hybridization (FISH), human/hamster radiation hybrid (RH) panels (Walter et al, 1994; Nature Genetics 7:22; Research Genetics, Huntsville Al), human-rodent somatic cell hybrid panels such as is available from the Coriell Institute (Camden, New Jersey), and genomic viewers utilizing BLAST homologies to sequenced and mapped genomic clones (NCBI, Bethesda, Maryland).

58P1D12 maps to chromosome 21q11.2 using 58P1D12 sequence and the NCBI BLAST tool located on the World Wide Web at: (.ncbi.nlm.nih.gov/genome/seq/page.cgi?F=HsBlast.html&&ORG=Hs).

### Example 4

### Expression Analysis of 58P1D12 in Normal Tissues and Patient Specimens

58P1D12 expression in normal and patient cancer tissues was tested by PCR (Figure 14). First strand cDNA was prepared from normal tissues (bladder, brain, heart, kidney, liver, lung, prostate, spleen, skeletal muscle, testis, pancreas, colon and stomach), and from pools of patient cancer specimens (prostate cancer pool, bladder cancer pool, lung cancer pool, ovary cancer pool, prostate cancer xenograft pool, bone and melanoma cancer pool, and cervical cancer pool). Normalization was performed by PCR using primers to actin. Semi-quantitative PCR, using primers to 58P1D12, was performed at 30 cycles of amplification. Results expression of 58P1D12 predominantly in testis amongst the normal tissues tested. 58P1D12 is also expressed in prostate cancer, bladder cancer, lung cancer, ovary cancer, prostate cancer xenograft, bone/melanoma cancer pool, and cervical cancer.

Figure 15 shows expression of 58P1D12 in normal tissues by Northern blot analysis. Two multiple tissue northern blots (Clontech) both with 2 µg of mRNA/lane were probed with the 58P1D12 sequence. Size standards in kilobases (kb) are indicated on the side. Results show predominant expression of 58P1D12 transcript in normal testis. A significantly weaker signal is detected in spleen, lung, kidney and pancreas.

In order to show expression of 58P1D12 in prostate cancer tissues, RNA was extracted from LAPC prostate cancer xenografts, LAPC-4AD, LAPC-4AI, LAPC-9AD and LAPC-9AI, as well as from normal prostate. Northern blots with 10 µg of total RNA were probed with the 58P1D12 sequence. Size standards in kilobases are on the side. Results show strong expression of 58PID12 in LAPC-9AD, and a weaker signal in LAPC-9Al, but not in the other tissues tested. (Figure 16).

Figure 17 shows 58P1D12 expression in normal and patient cancer specimens. RNA was extracted from a pool of 3 ovary tumor patient specimens, normal prostate (NP), normal bladder (NB), normal kidney (NK), and normal colon (NC). Northern blots with 10 µg of total RNA were probed with the 58P1D12 sequence. Size standards in kilobases are on the side. Results show expression of 58P1D12 in ovary tumor patient specimens but not in the normal tissues tested.

Analysis of 58P1D12 expression in individual ovarian cancer patient specimens is shown in Figure 18. RNA was extracted from normal ovary, LAPC-9AD prostate cancer xenograft, and a panel of ovary tumor patient specimens (Ovary T). Northern blots with 10 µg of total RNA were probed with the 58P1D12 sequence. Size standards in kilobases are on the side. Results show expression of 58P1D12 in most of the patient specimens tested as well as in the prostate xenograft.

58P1D12 expression was tested in a large panel of ovarian cancer patient specimens (Figure 19). First strand cDNA was prepared from a panel of ovary patient cancer specimens as well as two different normal ovary tissues. Normalization was performed by PCR using primers to actin. Semi-quantitative PCR, using primers to 58P1D12, was performed at 26 and 30 cycles of amplification. Samples were run on an agarose gel, and PCR products were quantitated using the Alphalmager software. Expression was recorded as negative, weak, medium or strong. Results show expression of 58P1D12 in the majority of patient cancer specimens tested (75%), but not in normal ovary.

### Example 5

### Transcript Variants of 58P1D12

Transcript variants are variants of mature mRNA from the same gene which arise by alternative transcription or alternative splicing. Alternative transcripts are transcripts from the same gene but start transcription at different points. Splice variants are mRNA variants spliced differently from the same transcript. In eukaryotes, when a multi-exon gene is transcribed from genomic DNA, the initial RNA is spliced to produce functional mRNA, which has only exons and is used for translation into an amino acid sequence. Accordingly, a given gene can have zero to many alternative transcripts and each transcript can have zero to many splice variants. Each transcript variant has a unique exon makeup, and can have different coding and/or noncoding (5' or 3' end) portions, from the original transcript. Transcript variants can code for similar or different proteins with the same or a similar function or can encode proteins with different functions, and can be expressed in the same tissue at the same time, or in different tissues at the same time, or in the same tissue at different times, or in different tissues at different times. Proteins encoded by transcript variants can have similar or different cellular or extracellular localizations, e.g., secreted versus intracellular.

Transcript variants are identified by a variety of art-accepted methods. For example, alternative transcripts and splice variants are identified by full-length cloning experiment, or by use of full-length transcript and EST sequences. First, all human ESTs were grouped into clusters which show direct or indirect identity with each other. Second, ESTs in the same cluster were further grouped into sub-clusters and assembled into a consensus sequence. The original gene sequence is compared to the consensus sequence(s) or other full-length sequences. Each consensus sequence is a potential splice variant for that gene (see, e.g., URL www.doubletwist.com/products/c11_agentsOverview.jhtml). Even when a variant is identified that Is not a full-length done, that portion of the variant is very useful for antigen generation and for further cloning of the full-length splice variant, using techniques known in the art.

Moreover, computer programs are available in the art that identify transcript variants based on genomic sequences. Genomic-based transcript variant identification programs include FgenesH (A. Salamov and V. Solovyev, "Ab initio gene finding in Drosophila genomic DNA," Genome Research. 2000 April;10(4):516-22); Grail (URL compbio.ornl.gov/Grailbin/EmptyGrailForm) and GenScan (URL genes.mit.edu/GENSCAN.html). For a general discussion of splice variant identification protocols see., e.g., Southan, C., A genomic perspective on human proteases, FEBS Lett. 2001 Jun 8; 498(2-3):214-8; de Souza, S.J., et al., Identification of human chromosome 22 transcribed sequences with ORF expressed sequence tags, Proc. Natl Acad Sci U S A. 2000 Nov 7; 97(23):12690-3.

To further confirm the parameters of a transcript variant, a variety of techniques are available in the art, such as full-length cloning, proteomic validation, PCR-based validation, and 5' RACE validation, etc. (see e.g., Proteomic Validation: Brennan, S.O., et al., Albumin banks peninsula: a new termination variant characterized by electrospray mass spectrometry, Biochem Biophys Acta. 1999 Aug 17;1433(1-2):321-6; Ferranti P, et al., Differential splicing of pre-messenger RNA produces multiple forms of mature caprine alpha(s1)-casein, Eur J Biochem. 1997 Oct 1;249(1):1-7. For PCR-based Validation: Wellmann S, et al., Specific reverse transcription-PCR quantification of vascular endothelial growth factor (VEGF) splice variants by LightCycler technology, Clin Chem. 2001 Apr;47(4):654-60; Jia, H.P., et al., Discovery of new human beta-defensins using a genomics-based approach, Gene. 2001 Jan 24; 263(1-2):211-8. For PCR-based and 5' RACE Validation: Brigle, K.E., et al., Organization of the murine reduced folate carrier gene and identification of variant splice forms, Biochem Biophys Acta.1997 Aug 7; 1353(2):191-8).

It is known in the art that genomic regions are modulated in cancers. When the genomic region to which a gene maps is modulated in a particular cancer, the alternative transcripts or splice variants of the gene are modulated as well. Disclosed herein is that 58P1D12 has a particular expression profile related to cancer. Alternative transcripts and splice variants of 58P1D12 may also be involved in cancers in the same or different tissues, thus serving as tumor-associated markers/antigens.

Using the full-length gene and EST sequences, four transcript variants were identified, designated as 58P1D12 v.2, v.3, v.4 and v.5. The boundaries of the exon in the original transcript, 58P1D12 v.1 were shown in Table LI. Exon compositions of the variants were shown in Figure 10. Theoretically, each different combination of exons in spatial order, e.g. exon 1 of v.2 and exons 3, 4, 5 and 6 of v.1, is a potential splice variant.

Tables LII(a)-(d) through LV(a)-(d) are set forth on a variant-by-variant bases. LII(a)-(d) shows nucleotide sequence of the transcript variant. Table LIII(a)-(d) shows the alignment of the transcript variant with nucleic acid sequence of 58P1D12 v.1. LIV(a)-(d) lays out amino acid translation of the transcript variant for the identified reading frame orientation. Table LV(a)-(d) displays alignments of the amino acid sequence encoded by the splice variant with that of 58P1D12 v.1.

### Example 6

### Single Nucleotide Polymorphisms of 58P1D12

A Single Nucleotide Polymorphism (SNP) is a single base pair variation in a nucleotide sequence at a specific location. At any given point of the genome, there are four possible nucleotides base pairs: A/T, C/G, G/C and T/A. Genotype refers to the specific base pair sequence of one or more locations in the genome of an individual. Haplotype refers to the base pair sequence of more than one location on the same DNA molecule (or the same chromosome in higher organisms), often in the context of one gene or in the context of several lightly linked genes. SNP that occurs on a cDNA is called cSNP. This cSNP may change amino acids of the protein encoded by the gene and thus change the functions of the protein. Some SNP cause inherited diseases; others contribute to quantitative variations in phenotype and reactions to environmental factors including diet and drugs among individuals. Therefore, SNP and/or combinations of alleles (called haplotypes) have many applications, induding diagnosis of inherited diseases, determination of drug reactions and dosage, identification of genes responsible for diseases, and analysis of the genetic relationship between individuals (P. Nowotny, J. M. Kwon and A. M. Goate, "SNP analysis to dissect human traits," Curr. Opin. Neurobiol. 2001 Oct; 11 (5):637-641; M. Pirmohamed and B. K Park, "Genetic susceptibility to adverse drug reactions," Trends Pharmacol. Sci. 2001 Jun; 22(6):298-305; J. H. Riley, C. J. Allan, E. Lai and A. Roses, "The use of single nucleotide polymorphisms in the isolation of common disease genes," Pharmacogenomics. 2000 Feb; 1(1):39-47; R. Judson, J. C. Stephens and A. Windemuth, The predictive power of haplotypes in clinical response," Pharmacogenomics. 2000 feb; 1 (1):15-26).

SNP are identified by a variety of art-accepted methods (P. Bean, "The promising voyage of SNP target discovery," Am. Clin. Lab. 2001 Oct-Nov; 20(9):18-20; K. M. Weiss, "In search of human variation," Genome Res. 1998 Jul; 8(7):691-697; M. M. She, "Enabling large-scale pharmacogenetic studies by high-throughput mutation detection and genotyping technologies," Clin. Chem. 2001 Feb; 47(2):164-172). For example, SNP can be identified by sequencing DNA fragments that show polymorphism by gel-based methods such as restriction fragment length polymorphism (RFLP) and denaturing gradient gel electrophoresis (DGGE). They can also be discovered by direct sequencing of DNA samples pooled from different individuals or by comparing sequences from different DNA samples. With the rapid accumulation of sequence data in public and private databases, one can discover SNP by comparing sequences using computer programs (Z. Gu, L. Hillier and P. Y. Kwok, "Single nucleotide polymorphism hunting in cyberspace," Hum. Mutat. 1998; 12(4):221-225). SNP can be verified and genotype or haplotype of an individual can be determined by a variety of methods including direct sequencing and high throughput microarrays (P. Y. Kwok, "Methods for genotyping single nucleotide polymorphisms," Annu. Rev. Genomics Hum. Genet. 2001; 2:235-258; M. Kokoris, K. Dix, K. Moynihan, J. Mathis, B. Erwin, P. Grass, B. Hines and A. Duesterhoeft, "High-throughput SNP genotyping with the Masscode system," Mol. Diagn. 2000 Dec; 5(4):329-340).

Using the methods described above, ten SNP were identified in the original transcript, 58P1D12 v.1, at positions 1764 (A/C), 1987 (G/A), 2045 (A/G), 2066 (T/C), 2134 (T/A), 2350 (G/T), 2435 (G/T), 302 (G/T), 304 (G/T) and 1533 (C/T). The transcripts or proteins with alternative allele were designated as variant 58P1D12 v.6 through v.15, respectively. Figure 12 shows the schematic alignment of the SNP variants. Figure 11 shows the schematic alignment of protein variants, corresponding to nucleotide variants. Nucleotide variants that code for the same amino acid sequence as v.1 are not shown in Figure 11. These alleles of the SNP, though shown separately here, can occur In different combinations (haplotypes) and In any one of the transcript variants (such as 58P1D12 v.2) that contains the site of the SNP.

### Example 7

### Production of Recombinant 58P1D12 in Prokaryotic Systems

To express recombinant 58P1D12 and 58P1D12 variants in prokaryotic cells, the full or partial length 58P1D12 and 58P1D12 variant cDNA sequences are cloned into any one of a variety of expression vectors known in the art. One or more of the following regions of 58P1D12 variants are expressed: the full length sequence presented in Figures 2 and 3, or any 8, 9, 10, 11, 12, 13, 14, 15, 16, 17, 18, 19, 20, 21, 22, 23, 24, 25, 26, 27, 28, 29, 30 or more contiguous amino acids from 58P1D12, variants, or analogs thereof.

A. *In vitro* transcription and translation constructs:

pCRII: To generate 58P1D12 sense and anti-sense RNA probes for RNA *in situ* investigations, pCRII constructs (Invitrogen, Carlsbad CA) are generated encoding either all or fragments of the 58P1D12 cDNA. The pCRII vector has Sp6 and T7 promoters flanking the insert to drive the transcription of 58P1D12 RNA for use as probes in RNA *in situ* hybridization experiments. These probes are used to analyze the cell and tissue expression of 58P1D12 at the RNA level. Transcribed 58P1D12 RNA representing the cDNA amino acid coding region of the 58P1D12 gene is used In *in vitro* translation systems such as the TnT^{™} Coupled Reticulolysate System (Promega, Corp., Madison, WI) to synthesize 58P1D12 protein.

### B. Bacterial Constructs:

pGEX Constructs: To generate recombinant 58P1D12 proteins in bacteria that are fused to the Glutathione S-transferase (GST) protein, all or parts of the 58P1D12 cDNA protein coding sequence are cloned into the pGEX family of GST-fusion vectors (Amersham Pharmacia Biotech, Piscataway, NJ). These constructs allow controlled expression of recombinant 58P1D12 protein sequences with GST fused at the amino-terminus and a six histidine epitope (6X His) at the carboxyl-terminus. The GST and 6X His tags permit purification of the recombinant fusion protein from induced bacteria with the appropriate affinity matrix and allow recognition of the fusion protein with anti-GST and anti-His antibodies. The 6X His tag is generated by adding 6 histidine codons to the cloning primer at the 3' end, e.g., of the open reading frame (ORF). A proteolytic cleavage site, such as the PreScission^{™} recognition site in pGEX-6P-1, may be employed such that it permits cleavage of the GST tag from 58P1D12-related protein. The ampicillin resistance gene and pBR322 origin permits selection and maintenance of the pGEX plasmids in E. *coli.*

pMAL Constructs: To generate, in bacteria, recombinant 58P1D12 proteins that are fused to maltose-binding protein (MBP), all or parts of the 58P1D12 cDNA protein coding sequence are fused to the MBP gene by cloning into the pMAL-c2X and pMAL-p2X vectors (New England Biolabs, Beverly, MA). These constructs allow controlled expression of recombinant 58P1D12 protein sequences with MBP fused at the amino-terminus and a 6X His epitope tag at the carboxyl-terminus. The MBP and 6X His tags permit purification of the recombinant protein from induced bacteria with the appropriate affinity matrix and allow recognition of the fusion protein with anti-MBP and anti-His antibodies. The 6X His epitope tag is generated by adding 6 histidine codons to the 3' cloning primer. A Factor Xa recognition site permits cleavage of the pMAL tag from 58P1D12. The pMAL-c2X and pMAL-p2X vectors are optimized to express the recombinant protein in the cytoplasm or periplasm respectively. Periplasm expression enhances folding of proteins with disulfide bonds.

pET Constructs: To express 58P1D12 in bacterial cells, all or parts of the 58P1D12 cDNA protein coding sequence are cloned Into the pET family of vectors (Novagen, Madison, WI). These vectors allow tightly controlled expression of recombinant 58P1D12 protein in bacteria with and without fusion to proteins that enhance solubility, such as NusA and thioredoxin (Trx), and epitope tags, such as 6X His and S-Tag^{™} that aid purification and detection of the recombinant protein. For example, constructs are made utilizing pET NusA fusion system 43.1 such that regions of the 58P1D12 protein are expressed as amino-terminal fusions to NusA. The cDNA encoding amino acids 22-213 of 58P1D12 was cloned Into the pET-21b vector, expressed, and purified from bacteria. The recombinant protein was used to generate rabbit polyclonal antibodies.

### C. Yeast Constructs:

pESC Constructs: To express 58P1D12 in the yeast species *Saccharomyces cerevisiae* for generation of recombinant protein and functional studies, all or parts of the 58P1D12 cDNA protein coding sequence are cloned into the pESC family of vectors each of which contain 1 of 4 selectable markers, HIS3, TRP1, LEU2, and URA3 (Stratagene, La Jolla, CA). These vectors allow controlled expression from the same plasmid of up to 2 different genes or cloned sequences containing either Flag^{™} or Myc epitope tags in the same yeast cell. This system is useful to confirm protein-protein interactions of 58P1D12. In addition, expression in yeast yields similar post-translational modifications, such as glycosylations and phosphorylations that are found when expressed In eukaryotic cells.

pESP Constructs: To express 58P1D12 in the yeast species *Saccharomyces pombe,* all or parts of the 58P1D12 cDNA protein coding sequence are cloned into the pESP family of vectors. These vectors allow controlled high level of expression of a 58P1D12 protein sequence that is fused at either the amino terminus or at the carboxyl terminus to GST which aids purification of the recombinant protein. A Flag^{™} epitope tag allows detection of the recombinant protein with anti- Flag^{™} antibody.

### Example 8

### Production of Recombinant 58P1D12 in Higher Eukaryotic Systems

### A. Mammalian Constructs:

To express recombinant 58P1D12 in eukaryotic cells, the full or partial length 58P1D12 cDNA sequences were cloned into any one of a variety of expression vectors known in the art. One or more of the following regions of 58P1D12 were expressed in these constructs, amino acids 1 to 273 orany 8, 9, 10, 11, 12, 13, 14, .15,16,17,18,19, 20, 21, 22, 23, 24, 25, 26, 27, 28, 29, 30 or more contiguous amino acids from 58P1D12 v.1; amino acids 1 to 232 of v. 2; amino acids 1 to 236 of v.4; or any 8, 9, 10, 11, 12, 13, 14, 15, 16, 17, 18, 19, 20, 21, 22, 23, 24, 25, 26, 27, 28, 29, 30 or more contiguous amino acids from 58P1D12 variants, or analogs thereof.

The constructs can be transfected into any one of a wide variety of mammalian cells such as 293T cells. Transfected 293T cell lysates can be probed with the anti-58P1D12 polyclonal serum, described herein.

pcDNA4/HisMax Constructs: To express 58P1D12 in mammalian cells, a 58P1D12 ORF, or portions thereof, of 58P1D12 are cloned into pcDNA4/HisMax Version A (Invitrogen, Carlsbad, CA). Protein expression is driven from the cytomegalovirus (CMV) promoter and the SP16 translational enhancer. The recombinant protein has Xpress^{™} and six histidine (6X His) epitopes fused to the amino-terminus. The pcDNA4/HisMax vector also contains the bovine growth hormone (BGH) polyadenylation signal and transcription termination sequence to enhance mRNA stability along with the SV40 origin for episomal replication and simple vector rescue in cell lines expressing the large T antigen. The Zeocin resistance gene allows for selection of mammalian cells expressing the protein and the ampicillin resistance gene and ColE1 origin permits selection and maintenance of the plasmid in *E. coli.*

pcDNA3.1/MycHisConstructs: To express 58P1012 in mammalian cells, a 58P1D12 ORF, or portions thereof, of 58P1D12 with a consensus Kozak translation initiation site was cloned into pcDNA3.1/MycHis Version A (Invitrogen, Carlsbad, CA). Protein expression is driven from the cytomegalovirus (CMV) promoter. The recombinant proteins have the myc epitope and 6X His epitope fused to the carboxyl-terminus. The pcDNA3.1/MycHis vector also contains the bovine growth hormone (BGH) polyadenylation signal and transcription termination sequence to enhance mRNA stability, along with the SV40 origin for episomal replication and simple vector rescue in cell lines expressing the large T antigen. The Neomycin resistance gene can be used, as it allows for selection of mammalian cells expressing the protein and the ampicillin resistance gene and ColE1 origin permits selection and maintenance of the plasmid In *E. coli*.

The complete ORF of 58P1D12 v.1 was cloned into the pcDNA3.1/MycHis construct to generate 58P1D12.pcDNA3.1/MycHis.

pcDNA3.1/CT-GFP-TOPOConstruct: To express 58P1D12 in mammalian cells and to allow detection of the recombinant proteins using fluorescence, a 58P1D12 ORF, or portions thereof, with a consensus Kozak translation initiation site are cloned into pcDNA3.1/CT-GFP-TOPO (Invitrogen, CA). Protein expression is driven from the cytomegalovirus (CMV) promoter. The recombinant proteins have the Green Fluorescent Protein (GFP) fused to the carboxyl-terminus facilitating non-invasive, *in vivo* detection and cell biology studies. The pcDNA3.1 CT-GFP-TOPO vector_also contains the bovine growth hormone (BGH) polyadenylation signal and transcription termination sequence to enhance mRNA stability along with the SV40 origin for episomal replication and simple vector rescue in cell lines expressing the large T antigen. The Neomycin resistance gene allows for selection of mammalian cells that express the protein and the ampicillin resistance gene and ColE1 origin permits selection and maintenance of the plasmid in *E*. *coli.* Additional constructs with an amino-terminal GFP fusion are made in pcDNA3.1/NT-GFP-TOPO spanning the entire length of a 58P1D12 protein.

PAPtag: A 58P1D12 ORF, or portions thereof, were cloned into pAPtag-5 (GenHunter Corp. Nashville, TN). This construct generates an alkaline phosphatase fusion at the carboxyl-terminus of a 58P1 D12 protein while fusing the IgGκ signal sequence to the amino-terminus. Constructs are also generated in which alkaline phosphatase with an amino-terminal IgGκ signal sequence is fused to the amino-terminus of a 58P1012 protein. The resulting recombinant 58P1D12 proteins are optimized for secretion into the media of transfected mammalian cells and can be used to identify proteins such as ligands or receptors that interact with 58P1D12 proteins. Protein expression is driven from the CMV promoter and the recombinant proteins also contain myc and 6X His epitopes fused at the carboxyl-terminus that facilitates detection and purification. The Zeocin resistance gene present in the vector allows for selection of mammalian cells expressing the recombinant protein and the ampicillin resistance gene permits selection of the plasmid in *E*. *coli.*

pTag5: A 58P1D12 ORF, or portions thereof, were cloned into pTag-5. This vector is similar to pAPtag but without the alkaline phosphatase fusion. This construct generated 58P1D12 protein with an amino-terminal IgGκ signal sequence and myc and 6X His epitope tags at the carboxyl-terminus that facilitate detection and affinity purification. The resulting recombinant 58P1D12 protein was optimized for secretion into the media of transfected mammalian cells, and was used as immunogen or ligand to identify proteins such as ligands or receptors that interact with the 58P1 D12 proteins. Protein expression is driven from the CMV promoter. The Zeocin resistance gene present in the vector allows for selection of mammalian cells expressing the protein, and the ampicillin resistance gene permits selection of the plasmid in *E. coli*.

PsecFc: A 58P1D12 ORF, or portions thereof, were cloned into psecFc. The psecFc vector was assembled by cloning the human immunoglobulin G1 (IgG) Fc (hinge, CH2, CH3 regions) into pSecTag2 (Invitrogen, California). This construct generated an IgG1 Fc fusion at the carboxyl-terminus of the 58P1D12 proteins, while fusing the IgGK signal sequence to N-terminus. 58P1D12 fusions utilizing the murine IgG1 Fc region are also used. The resulting recombinant 58P1D12 proteins are optimized for secretion into the media of transfected mammalian cells, and can be used as immunogens or to identify proteins such as ligands or receptors that interact with 58P1D12 protein. Protein expression is driven from the CMV promoter. The hygromycin resistance gene present in the vector allows for selection of mammalian cells that express the recombinant protein, and the ampicillin resistance gene permits selection of the plasmid in *E*. *coli.*

pSRα Constructs: To generate mammalian cell lines that express 58P1 D12 constitutively, 58P1 D12 ORF, or portions thereof, were cloned into pSRα constructs. Amphotropic and ecotropic retroviruses were generated by transfection of pSRα constructs into the 293T-10A1 packaging line or co-transfection of pSRα and a helper plasmid (containing deleted packaging sequences) into the 293 cells, respectively. The retrovirus is used to infect a variety of mammalian cell lines, resulting in the integration of the cloned gene, 58P1D12, into the host cell-lines. Protein expression is driven from a long terminal repeat (LTR). The Neomycin resistance gene present in the vector allows for selection of mammalian cells that express the protein, and the ampicillin resistance gene and ColE1 origin permit selection and maintenance of the plasmid in *E. coli*. The retroviral vectors can thereafter be used for infection and generation of various cell lines using, for example, PC3, NIH 3T3, TsuPr1, 293 or rat-1 cells.

Additional pSRα constructs are made that fuse an epitope tag such as the FLAG^{™} tag to the carboxyl-terminus of 58P1D12 sequences to allow detection using anti-Flag antibodies. For example, the FLAG^{™} sequence 5' gat tac aag gat gac gac gat aag 3' (SEQ ID NO: 34) is added to cloning primer at the 3' end of the ORF. Additional pSRα constructs are made to produce both amino-terminal and carboxyl-terminal GFP and myc/6X His fusion proteins of the full-length 58P1D12 proteins.

Additional Viral Vectors: Additional constructs are made for viral-mediated delivery and expression of 58P1D12. High virus titer leading to high level expression of 58P1D12 is achieved In viral delivery systems such as adenoviral vectors and herpes amplicon vectors. A 58P1D12 coding sequence or fragments thereof are amplified by PCR and subcloned into the AdEasy shuttle vector (Stratagene). Recombination and virus packaging are performed according to the manufacturer's instructions to generate adenoviral vectors. Alternatively, 58P1D12 coding sequences or fragments thereof are cloned into the HSV-1 vector (Imgenex) to generate herpes viral vectors. The viral vectors are thereafter used for infection of various cell lines such as PC3, NIH 3T3, 293 or rat-1 cells.

Regulated Expression Systems: To control expression of 58P1D12 in mammalian cells, coding sequences of 58P1D12, or portions thereof, are cloned into regulated mammalian expression systems such as the T-Rex System (Invitrogen), the GeneSwitch System (Invitrogen) and the tightly-regulated Ecdysone System (Sratagene). These systems allow the study of the temporal and concentration dependent effects of recombinant 58P1D12. These vectors are thereafter used to control expression of 58P1D12 in various cell lines such as PC3, NIH 3T3, 293 or rat-1 cells.

### B. Baculovirus Expression Systems

To generate recombinant 58P1D12 proteins in a baculovirus expression system, 58P1D12 ORF, or portions thereof, are cloned into the baculovirus transfer vector pBlueBac 4.5 (Invitrogen), which provides a His-tag at the N-terminus. Specifically, pBlueBac-58P1D12 is co-transfected with helper plasmid pBac-N-Blue (Invitrogen) into SF9 (*Spodoptera frugiperda*) insect cells to generate recombinant baculovirus (see Invitrogen instruction manual for details). Baculovirus is then collected from cell supernatant and purified by plaque assay.

Recombinant 58P1D12 protein is then generated by infection of HighFive insect cells (Invitrogen) with purified baculovirus. Recombinant 58P1D12 protein can be detected using anti-58P1D12 or anti-His-tag antibody. 58P1D12 protein can be purified and used in various cell-based assays or as immunogen to generate polyclonal and monoclonal antibodies specific for 58P1D12.

### Example 9

### Antigenicity Profiles and Secondary Structure

Figure 5A-C, Figure 6A-C, Figure 7A-C, Figure 8A-C, and Figure 9A-C depict graphically five amino acid profiles of 58P1D12 variant 1, variant 2, and variant 3, A through C respectively, each assessment available by accessing the ProtScale website (URL www.expasy.ch/cgi-bin/protseale.pl) on the ExPasy molecular biology server.

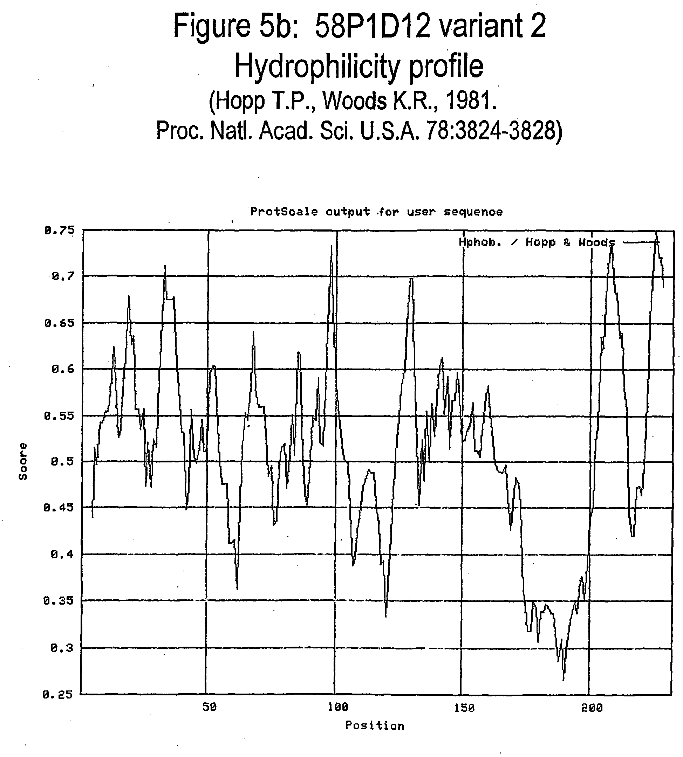

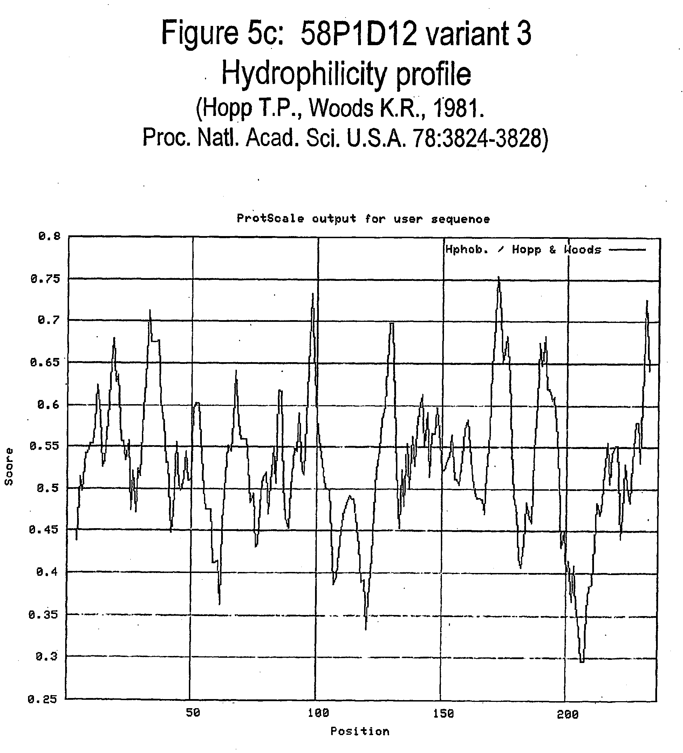

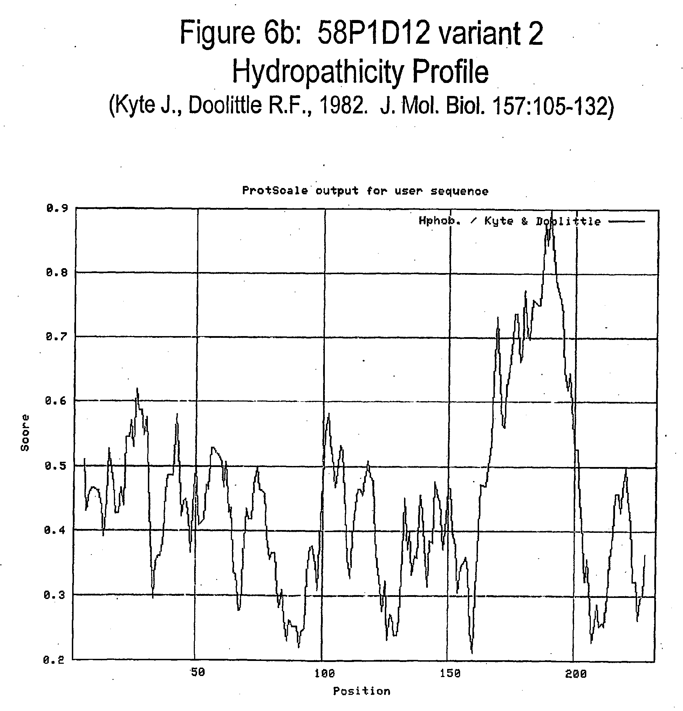

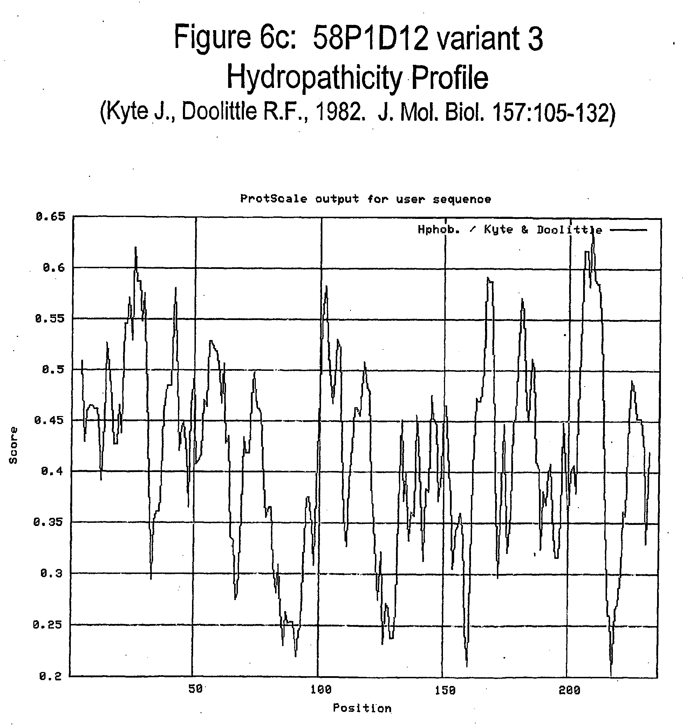

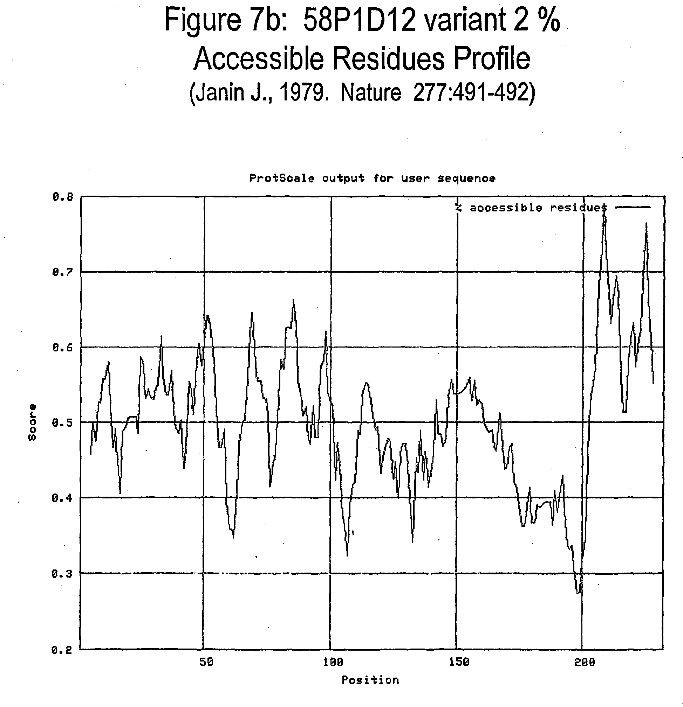

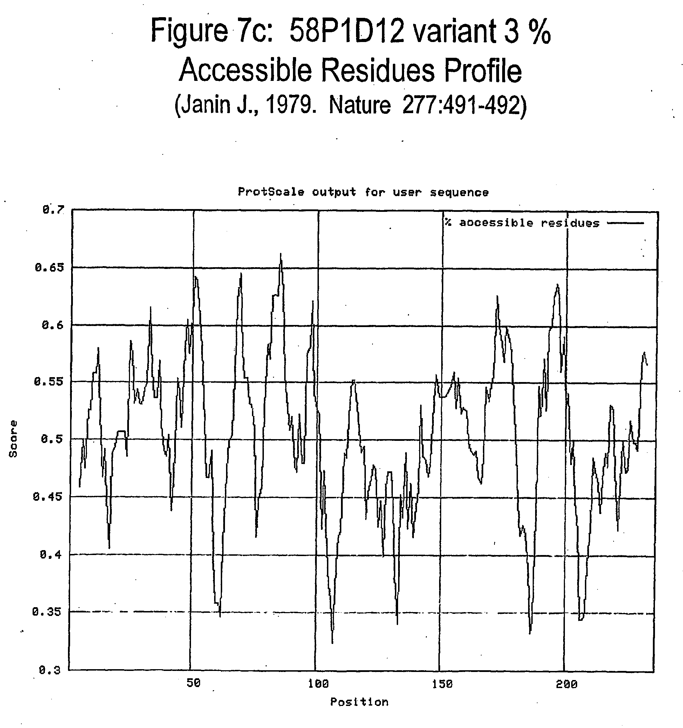

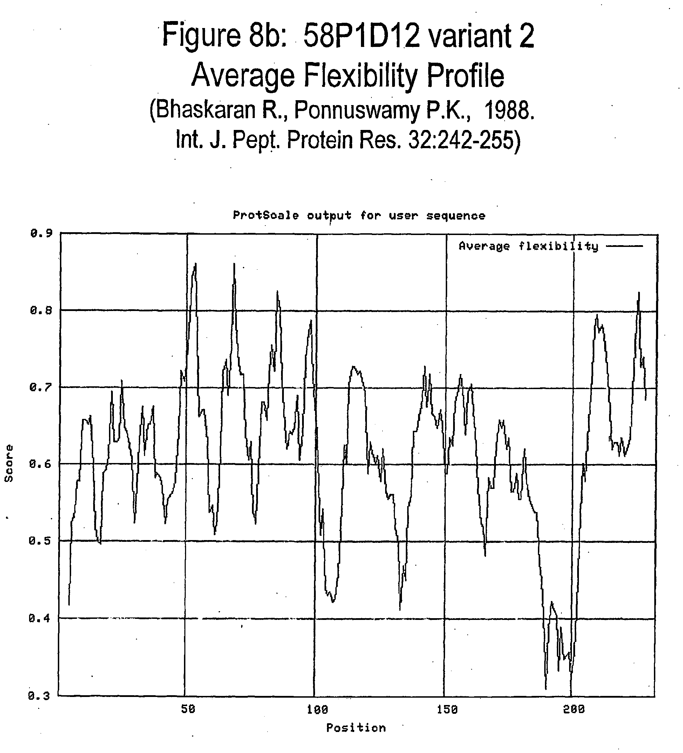

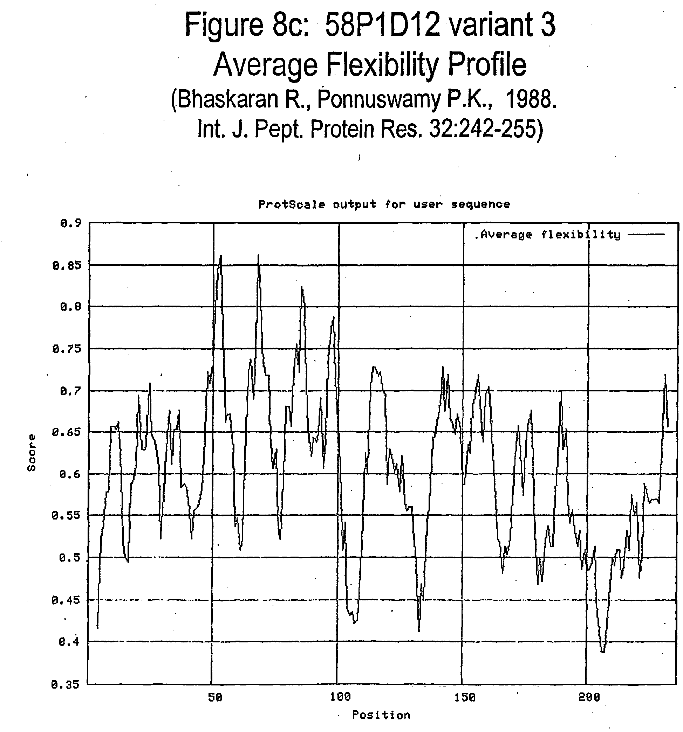

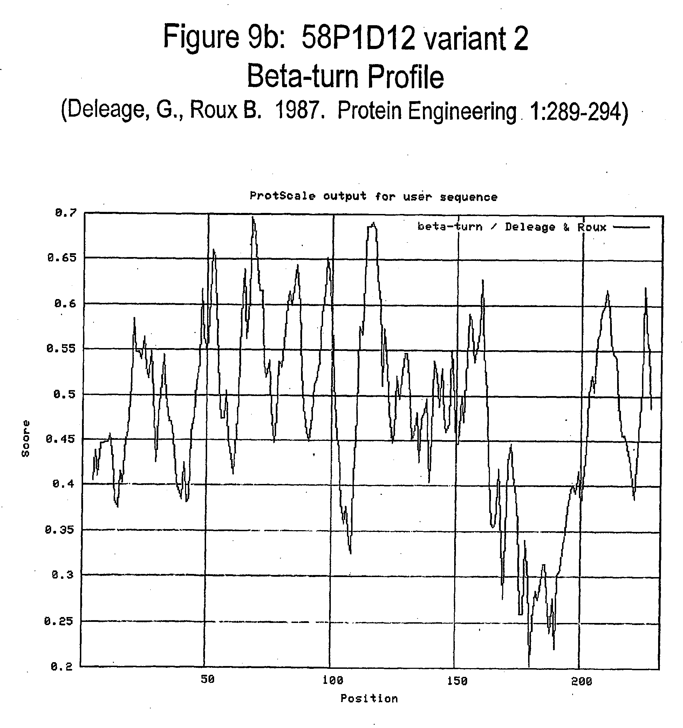

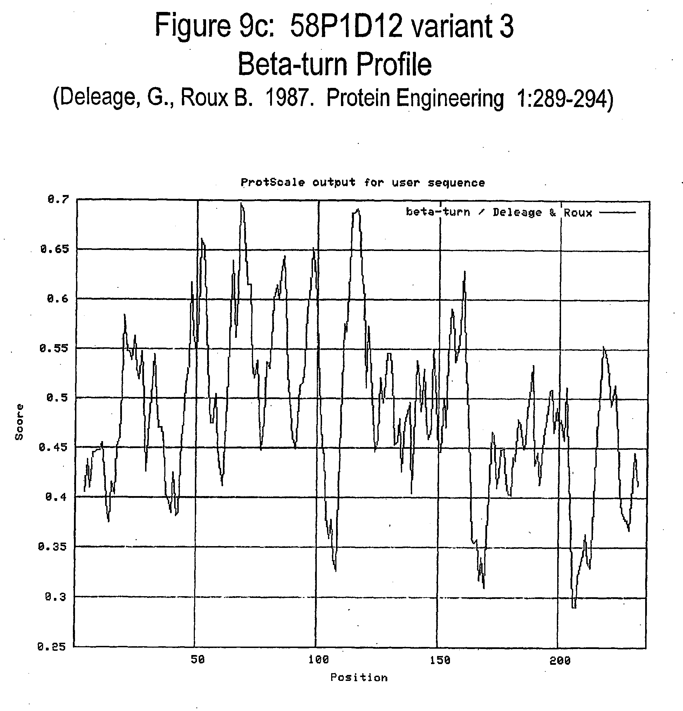

These profiles: Figure 5, Hydrophilicity, (Hopp T.P., Woods K.R., 1981. Proc. Natl. Acad. Sci. U.S.A. 78:3824-3828); Figure 6, Hydropathicity, (Kyte J., Doolittle R.F., 1982. J. Mol. Biol. 157:105-132); Figure 7, Percentage Accessible Residues (Janin J., 1979 Nature 277:491-492); Figure 8, Average Flexibility, (Bhaskaran R., and Ponnuswamy P.K., 1988. Int. J. Pept. Protein Res. 32:242-255); Figure 9, Beta-turn (Deleage, G., Roux B. 1987 Protein Engineering 1:289-294); and optionally others available in the art, such as on the ProtScale website, were used to identify antigenic regions of each of the 58P1D12 variant proteins. Each of the above amino acid profiles of 58P1D12 variants were generated using the following ProtScale parameters for analysis: 1) A window size of 9; 2) 100% weight of the window edges compared to the window center; and, 3) amino acid profile values normalized to lie between 0 and 1.

Hydrophilicity (Figure 5), Hydropathicity (Figure 6) and Percentage Accessible Residues (Figure 7) profiles were used to determine stretches of hydrophilic amino acids (i.e., values greater than 0.5 on the Hydrophilicity and Percentage Accessible Residues profile, and values less than 0.5 on the Hydropathicity profile). Such regions are likely to be exposed to the aqueous environment, be present on the surface of the protein, and thus available for immune recognition, such as by antibodies.

Average Flexibility (Figure 8) and Beta-turn (Figure 9) profiles determine stretches of amino acids. (i.e., values greater than 0.5 on the Beta-turn profile and the Average Flexibility profile) that are not constrained in secondary structures such as beta sheets and alpha helices. Such regions are also more likely to be exposed on the protein and thus accessible to immune recognition, such as by antibodies.

Antigenic sequences of the 58P1D12 variant proteins indicated, e.g., by the profiles set forth in Figure 5, Figure 6, Figure 7, Figure 8, and/or Figure 9 are used to prepare immunogens, either peptides or nucleic acids that encode them, to generate therapeutic and diagnostic anti-58P1D12 antibodies. The immunogen can be any 5, 6, 7, 8, 9,10,11,12,13,14,15, 16, 17, 18, 19, 20, 21, 22, 23, 24, 25, 30, 35, 40, 45, 50 or more than 50 contiguous amino adds, or the corresponding nucleic acids that encode them, from the 58P1D12 protein variants listed in Figures 2 and 3. In particular, peptide immunogens of the invention can comprise, a peptide region of at least 5 amino acids of Figures 2 and 3 in any whole number increment that includes an amino acid position having a value greater than 0.5 in the Hydrophilicity profiles of Figure 5; a peptide region of at least 5 amino acids of Figures 2 and 3 in any whole number increment that includes an amino acid position having a value less than 0.5 in the Hydropathicity profile of Figures 6 ; a peptide region of at least 5 amino acids of Figures 2 and 3 in any whole number increment that includes an amino acid position having a value greater than 0.5 in the Percent Accessible Residues profiles of Figure 7; a peptide region of at least 5 amino acids of Figures 2 and 3 in any whole number increment that includes an amino acid position having a value greater than 0.5 in the Average Flexibility profiles on Figure 8 ; and, a peptide region of at least 5 amino acids of Figures 2 and 3 in any whole number increment that includes an amino acid position having a value greater than 0.5 in the Beta-turn profile of Figures 9. Peptide immunogens of the invention can also comprise nucleic acids that encode any of the forgoing.

All immunogens of the invention, peptide or nucleic acid, can be embodied in human unit dose form, or comprised by a composition that includes a pharmaceutical excipient compatible with human physiology.

The secondary structures of 58P1D12 protein variants 1, 2, and 3, namely the predicted presence and location of alpha helices, extended strands, and random coils, are predicted from their primary amino acid sequences using the HNN - Hierarchical Neural Network method (NPS@: Network Protein Sequence Analysis TIBS 2000 March Vol. 25, No 3 [291]:147-150 Combet C., Blanchet C., Geourjon C. and Deléage G., http://pbil.ibcp.fr/cgi-bin/npsa_automat.pl?page=npsa_nn.html), accessed from the ExPasy molecular biology server (http://www.expasy.ch/tools/). The analysis indicates that 58P1D12 variant 1 is composed of 24.18 % alpha helix, 18.68 % extended strand, and 57.14 % random coil (Figure 13A). 58P1D12 variant 2 is composed of 19.83% alpha helix, 18.97% extended strand, and 61.21% random coil (Figure 13B). 58P1D12 variant 3 is composed of 32.20% alpha helix, 15.25% extended strand, and 52.54% random coil (Figure 13C).

Analysis for the potential presence of transmembrane domains in the 58P1D12 variant proteins 1, 2 and 3, was carried out using a variety of transmembrane prediction algorithms accessed from the ExPasy molecular biology server (http://www.expasy.ch/tools/): Shown graphically in figure 13D is the result of analysis of variant 1 using the TMpred program and in figure 13E results using the TMHMM program, both of which predict the presence of 1 transmembrane domain. Shown graphically in figure 13F is the result of analysis of variant 2 using the TMpred program and in figure 13G results using the TMHMM program, both of which predict the presence of 1 transmembrane domain. Variant 3 is not predicted to contain transmembrane domains (Figure 13H and 13I). Analyses of the variants using other structural prediction programs are summarized in Table VI and Table L.

### Example 10

### Generation of 58P1D12 Polyclonal Antibodies

Polyclonal antibodies can be raised In a mammal, for example, by one or more injections of an immunizing agent and, if desired, an adjuvant Typically, the immunizing agent and/or adjuvant will be injected in the mammal by multiple subcutaneous or intraperitoneal injections. In addition to immunizing with a full length 58P1D12 protein variant, computer algorithms are employed in design of immunogens that based on amino add sequence analysis contain characteristics of being antigenic and available for recognition by the immune system of the immunized host (see the Example entitled "Antigenicity Profiles and Secondary Structure"). Such regions would be predicted to be hydrophilic, flexible, in beta-turn conformations, and be exposed on the surface of the protein (see, e.g., Figure 5, Figure 6, Figure 7, Figure 8, or Figure 9 for amino acid profiles that indicate such regions of 58P1D12 protein variant 1).

For example, recombinant bacterial fusion proteins or peptides containing hydrophilic, flexible, beta-turn regions of 58P1D12 protein variants are used as antigens to generate polyclonal antibodies in New Zealand White rabbits or monoclonal antibodies as described in Example 11. For example, in 58P1D12 variant 1, such regions include, but are not limited to, amino acids 19-30, amino acids 49-66, amino acids 70-82, , amino acids 88-115, amino acids 131-145, amino acids 165-203, amino acids 243-255, and amino acids 262-273. It is useful to conjugate the immunizing agent to a protein known to be immunogenic in the mammal being immunized. Examples of such immunogenic proteins include, but are not limited to, keyhole limpet hemocyanin (KLH), serum albumin, bovine thyroglobulin, and soybean trypsin inhibitor. In one embodiment, a peptide encoding amino acids 102-115 of 58P1D12 variant 1 was conjugated to KLH and used to immunize a rabbit. Alternatively the immunizing agent may include all or portions of the 58P1D12 variant proteins, analogs or fusion proteins thereof. For example, the 58P1D12 variant 1 amino acid sequence can be fused using recombinant DNA techniques to any one of a variety of fusion protein partners that are well known in the art, such as glutathione-S-transferase (GST) and HIS tagged fusion proteins. In another embodiment, amino acids 22-213 of 58P1D12 variant 1 is fused to His using recombinant techniques and the pET21b expression vector. The protein was then expressed, purified, and used to immunize 2 rabbits. Such fusion proteins are purified from incluced bacteria using the appropriate affinity matrix.

Other recombinant bacterial fusion proteins that may be employed include maltose binding protein, LacZ, thioredoxin, NusA, or an immunoglobulin constant region (see the section entitled "Production of 58P1D12 in Prokaryotic Systems" and Current Protocols In Molecular Biology, Volume 2, Unit 16, Frederick M. Ausubul et al. eds., 1995; Linsley, P.S., Brady, W., Urnes, M., Grosmaire, L., Damle, N., and Ledbetter, J.(1991) J.Exp. Med.174; 561-566).

In addition to bacterial derived fusion proteins, mammalian expressed protein antigens are also used. These antigens are expressed from mammalian expression vectors such as the Tag5 and Fc-fusion vectors (see the section entitled "Production of Recombinant 58P1D12 in Eukaryotic Systems"), and retain post-translational modifications such as glycosylations found in native protein. In one embodiment, amino acids 22-213 of 58P1D12 variant 1 was cloned into the Tag5 mammalian secretion vector, and expressed in 293T cells. The recombinant protein was purified by metal chelate chromatography from tissue culture supernatants of 293T cells stably expressing the recombinant vector. The purified Tag5 58P1D12 protein was then used as immunogen.

During the immunization protocol, it is useful to mix or emulsify the antigen in adjuvants that enhance the immune response of the host animal. Examples of adjuvants include, but are not limited to, complete Freund's adjuvant (CFA) and MPL-TDM adjuvant (monophosphoryl Lipid A, synthetic trehalose dicorynomycolate).

In a typical protocol, rabbits are initially immunized subcutaneously with up to 200 µg, typically 100-200 µg, of fusion protein or peptide conjugated to KLH mixed in complete Freund's adjuvant (CFA). Rabbits are then injected subcutaneously every two weeks with up to 200 µg, typically 100-200 µg, of the immunogen in incomplete Freund's adjuvant (IFA). Test bleeds are taken approximately 7-10 days following each immunization and used to monitor the titer of the antiserum by ELISA

To test reactivity and specificity of immune serum, such as the rabbit serum derived from immunization with the His-fusion of 58P1D12 variant 1 protein, the full-length 58P1D12 variant 1 cDNA was cloned into pCDNA 3.1 myc-his expression vector (Invitrogen, see the Example entitled "Production of Recombinant 58P1D12 in Eukaryotic Systems"). After transfection of the constructs into 293T cells, cell lysates are probed with the anti-58P1D12 serum and with anti-His antibody (Santa Cruz Biotechnologies, Santa Cruz, CA) to determine specific reactivity to denatured 58P1D12 protein using the Western blot technique. In addition, the immune serum is tested by fluorescence microscopy, flow cytometry and immunoprecipitation against 293T and other recombinant 58P1D12-expressing cells to determine specific recognition of native protein. Western blot, immunoprecipitation, fluorescent microscopy, and flow cytometric techniques using cells that endogenously express 58P1D12 are also carried out to test reactivity and specificity.

Anti-serum from rabbits immunized with 58P1D12 variant fusion proteins, such as GST and MBP fusion proteins, are purified by depletion of antibodies reactive to the fusion partner sequence by passage over an affinity column containing the fusion partner either alone or in the context of an irrelevant fusion protein. For example, antiserum derived from a GST-58P1D12 variant 1 fusion protein is first purified by passage over a column of GST protein covalently coupled to AffiGel matrix (BioRad, Hercules, Calif.). The antiserum is then affinity purified by passage over a column composed of a MBP-58P1D12 fusion protein covalently coupled to Affigel matrix. The serum is then further purified by protein G affinity chromatography to isolate the IgG fraction. Sera from other His-tagged antigens and peptide immunized rabbits as well as fusion partner depleted sera are affinity purified by passage over a column matrix composed of the original protein immunogen or free peptide.

### Example 11

### Generation of 58P1D12 Monoclonal Antibodies (mAbs)

In one embodiment, therapeutic mAbs to 58P1D12 variants comprise those that react with epitopes specific for each variant protein or specific to sequences in common between the variants that would disrupt or modulate the biological function of the 58P1D12 variants, for example those that would disrupt the interaction with ligands and binding partners. Immunogens for generation of such mAbs include those designed to encode or contain the entire 58P1D12 protein variant sequence, regions predicted to contain functional motifs, and regions of the 58P1D12 protein variants predicted to be antigenic from computer analysis of the amino acid sequence (see, e.g., Figure 5, Figure 6, Figure 7, Figure 8, or Figure 9, and the Example entitled "Antigenicity Profiles and Secondary Structure"). Immunogens include peptides, recombinant bacterial proteins, and mammalian expressed Tag 5 proteins and human and murine IgG FC fusion proteins. In addition, cells engineered to express high levels of a respective 58P1D12 variant, such as Rat1-58P1D12 variant 1 or 300.19-58P1D12 variant 1murine Pre-B cells, were used to immunize mice.

To generate mAbs to a 58P1012 variant, mice are first immunized intraperitoneally (IP) with, typically, 10-50 µg of protein immunogen or 10⁷ 58P1D12-expressing cells mixed in complete Freund's adjuvant. Mice are then subsequently immunized IP every 2-4 weeks with, typically, 10-50 µg of protein immunogen or 10⁷ cells mixed in incomplete Freund's adjuvant. Alternatively, MPL-TDM adjuvant is used in immunizations. In addition to the above protein and cell-based immunization strategies, a DNA-based immunization protocol is employed in which a mammalian expression vector encoding a 58P1D12 variant sequence is used to immunize mice by direct injection of the plasmid DNA. For example, amino acids 22-213 of 58P1D12 of variant 1 is cloned into the Tag5 mammalian secretion vector and the recombinant vector will then be used as immunogen. In another example the same amino acids are cloned into an Fc-fusion secretion vector in which the 58P1D12 variant 1 sequence is fused at the amino-terminus to an IgK leader sequence and at the carboxyl-terminus to the coding sequence of the human or murine IgG Fc region. This recombinant vector is then used as immunogen. The plasmid immunization protocols are used in combination with purified proteins expressed from the same vector and with cells expressing the respective 58P1D12 variant.

During the immunization protocol, test bleeds are taken 7-10 days following an injection to monitor titer and specificity of the immune response. Once appropriate reactivity and specificity is obtained as determined by ELISA, Western blotting, immunoprecipitation, fluorescence microscopy, and flow cytometric analyses, fusion and hybridoma generation is then carried out with established procedures well known in the art (see, e.g., Harlow and Lane, 1988).

In one embodiment for generating 58P1D12 monoclonal antibodies, amino acids 22-213 of 58P1D12 variant 1 was cloned into the pTag5 vector, the protein was then expressed in 293T cells and purified by metal chelate chromatography. Balb C mice were immunized with 10µg of recombinant variant 1 protein mixed in adjuvant. Mice were subsequently immunized over several weeks with the protein. ELISA using the antigen determined the titer of serum from the immunized mice. Reactivity and specificity of the serum to full length 58P1D12 variant 1 protein was monitored by flow cytometry using Rat-58P1D12 cells compared to Rat1-neo control cells (Figure 20). Other recombinant 58P1D12 variant 1-expressing cells or cells endogenously expressing 58P1D12 variant 1 are also used. Mice showing the strongest reactivity were rested and given a final injection of antigen and sacrificed for fusion. The lymph nodes of the sacrificed mice were harvested and fused to SP2/0 myeloma cells using standard procedures (Harlow and Lane, 1988). Supernatants from HAT selected growth wells were analyzed by flow cytometry to identify specific-58P1 D12 surface binding MAbs (Figure 21). Supernatants are also be screened by ELISA, Western blot, immunoprecipitation, and fluorescent microscopy to identify 58P1D12 specific antibody-producing clones.

In another embodiment 58P1D12 variant 3 specific MAbs are generated by employing immunogens that encode amino acid sequences unique to variant 3. For example, a GST-fusion protein is constructed to encode amino acids 171-236, expressed, purified, and used to immunize mice. Hybridomas resulting from fusion of the B-calls from the mice are screened on cells expressing 58P1D12 variant 3 protein and cross-screened on cells expressing variant 1 protein to identify variant 3-specific MAbs.

The binding affinity of 58P1D12 variant specific monoclonal antibodies are determined using standard technologies. Affinity measurements quantify the strength of antibody to epitope binding and are used to help define which 58P1D12 variant monoclonal antibodies preferred for diagnostic or therapeutic use, as appreciated by one of skill in the art. The BIAcore system (Uppsala, Sweden) is a preferred method for determining binding affinity. The BIAcore system uses surface plasmon resonance (SPR, Welford K. 1991, Opt. Quant. Elect. 23:1; Morton and Myszka, 1998, Methods in Enzymology 295: 268) to monitor biomolecular interactions in real time. BIAcore analysis conveniently generates association rate constants, dissociation rate constants, equilibrium dissociation constants, and affinity constants. In addition, equilibrium binding analysis of a dilution series of the MAb is also used to determine affinity defined by the dissociation constant (KD). The KD is determined by non-linear regression of the equilibrium binding data of the concentration series. The KD is defined as the concentration at which half maximal binding of the MAb to the antigen is attained. Such analysis for MAb M8-143(5).10.1 is shown in Figure 22.

### Example 34

### Complementary Polynucleotides

Sequences complementary to the 58P1 D12-encoding sequences, or any parts thereof, are used to detect, decrease, or inhibit expression of naturally occurring 58P1D12. Although use of oligonucleotides comprising from about 15 to 30 base pairs is described, essentially the same procedure is used with smaller or with larger sequence fragments. Appropriate oligonucleotides are designed using, e.g., OLIGO 4.06 software (National Biosciences) and the coding sequence of 58P1D12. To inhibit transcription, a complementary oligonucleotide is designed from the most unique 5' sequence and used to prevent promoter binding to the coding sequence. To inhibit translation, a complementary oligonucleotide is designed to prevent ribosomal binding to a 58P1D12-encoding transcript

### Example 35

### Purification of Naturally-occuring or Recombinant 58P1D12 Using 58P1D12-Specific Antibodies

Naturally occurring or recombinant 58P1D12 is substantially purified by immunoaffinity chromatography using antibodies specific for 58P1D12. An immunoaffinity column is constructed by covalently coupling anti-58P1D12 antibody to an activated chromatographic resin, such as CNBr-activated SEPHAROSE (Amersham Pharmacia Biotech). After the coupling, the resin is blocked and washed according to the manufacturer's instructions.

Media containing 58P1D12 are passed over the immunoaffinity column, and the column is washed under conditions that allow the preferential absorbance of 58P1D12 (e.g., high Ionic strength buffers in the presence of detergent). The column is eluted under conditions that disrupt antibody/58P1D12 binding (e.g., a buffer of pH 2 to pH 3, or a high concentration of a chaotrope, such as urea or thiocyanate ion), and GCR.P is collected.

### Example 36

### Identification of Molecules Which Interact with 58P1D12

58P1D12, or biologically active fragments thereof, are labeled with 121 1 Bolton-Hunter reagent (See, e.g., Bolton et al. (1973) Biochem. J. 133:529.) Candidate molecules previously arrayed in the wells of a multi-well plate are Incubated with the labeled 58P1D12, washed, and any wells with labeled 58P1D12 complex are assayed. Data obtained using different concentrations of 58P1 D12 are used to calculate values for the number, affinity, and association of 58P1D12 with the candidate molecules.

### Example 37

### In Vivo Assay for 58P1D12 Tumor Growth Promotion

### In vivo assay of 3T3 cell growth by recombinant expression of 58P1D12.

To address the potential for 58P1D12 to accelerate the growth of non-tumorigenic cells in an *in vivo* mouse model, non-transformed 3T3 cells were prepared by infection with either a virus containing an empty vector control (Neo gene alone) or with a vector containing the 58P1D12 full-length gene. 3T3 cells were selected for survival in G-418, and expression of 58P1 D12 confirmed by Northern blot analysis. To assess the growth potential of these cells, 1 x 10⁶ 58P1D12 expressing 3T3 cells or 1 x 10⁶ Neo control were mixed with Matrigel®, then injected intratibially in SCID mice and allowed to grow for 31 days. The growth of these cells was assessed on day 31 by visual inspection and by necropsy (Figure 28). Clearly, the 58P1D12 expressing 3T3 cells showed a potent effect in comparison to the 3T3-Neo cells, indicating that the 58P1D12 protein enhanced the growth of the cells in Matrigel®. These data indicate that 58P1D12 promotes the growth of non-tumorigenic cells and provides a growth advantage *in vivo* that mimics the role of this protein in human malignancies.

### Example 39

### Therapeutic and Diagnostic use of Anti-58P1D12 Antibodies in Humans.

Anti-58P1 D12 monoclonal antibodies are safely and effectively used for diagnostic, prophylactic, prognostic and/or therapeutic purposes in humans. Western blot and immunohistochemical analysis of cancer tissues and cancer xenografts with anti-58P1D12 mAb show strong extensive staining in carcinoma but significantly lower or undetectable levels in normal tissues. Detection of 58P1D12 in carcinoma and in metastatic disease demonstrates the usefulness of the mAb as a diagnostic and/or prognostic Indicator. Anti-58P1 D12 antibodies are therefore used In diagnostic applications such as immunohistochemistry of kidney biopsy specimens to detect cancer from suspect patients.

As determined by flow cytometry, anti-58P1D12 mAb specifically binds to carcinoma cells. Thus, anti-58P1D12 antibodies are used in diagnostic whole body imaging applications, such as radioimmunoscintigraphy and radioimmunotherapy, (see, e.g., Potamianos S., et al. Anticancer Res 20(2A):925-948 (2000)) for the detection of localized and metastatic cancers that exhibit expression of 58P1D12. Shedding or release of an extracellular domain of 58P1 D12 into the extracellular milieu, such as that seen for alkaline phosphodiesterase B10 (Meerson, N. R., Hepatology 27:563-568 (1998)), allows diagnostic detection of 58P1D12 by anti-58P1D12 antibodies in serum and/or urine samples from suspect patients.

Anti-58P1D12 antibodies that specifically bind 58P1D12 are used in therapeutic applications for the treatment of cancers that express 58P1D12. Anti-58P1D12 antibodies are used as an unconjugated modality and as conjugated form in which the antibodies are attached to one of various therapeutic or imaging modalities well known in the art, such as a prodrugs, enzymes or radioisotopes. In preclinical studies, unconjugated and conjugated anti-58P1D12 antibodies are tested for efficacy of tumor prevention and growth inhibition in the SCID mouse cancer xenograft models, e.g., kidney cancer models AGS-K3 and AGS-K6, (see, e.g., the Example entitled °58P1D12 Monoclonal Antibody-mediated Inhibition of Bladder and Lung Tumors *in Vivo*"). Either conjugated and unconjugated anti-58P1D12 antibodies are used as a therapeutic modality in human clinical trials either alone or in combination with other treatments as described in following Examples.

### Example 44

### I. Enhanced angiogenesis (tube formation) of human umbilical vein endothelial cells by recombinant extracellular domain (ECD) of 58P1D12.

Angiogenesis is a critical event in the maintenance of tumors for their growth, nutrition and oxygenation. Growth and migration of endothelial cells Is a hallmark of angiogenesis, and the stimulation of such cells requires multiple serum growth factors including vascular endothelial growth factor (VEGF). To address whether 58P1 D12 facilitates angiogenesis of primary endothelium, an assay was established to detect the effect of the extracellular domain (ECD) (amino adds x-y) of 58P1D12 on the formation of endothelial matrices (tubes) when plated on Matrigel®. Human umbilical vein endothelial cells (HUVEC) or human lung microvascular endothelial cells (HMVEC) were grown in 10% FBS In media to 70% confluency (passage 2-passage 6). Cells were detached in 1:1 trypsin:PBS or 10 mM EDTA, washed and resuspended in EBM-0.1 % FBS. Recombinant 58P1D12 ECD (described in Example entitled "Production of Recombinant 58P1D12 in Eukaryotic Systems") was added to the cells and then incubated on 200 µl Matrigel® for 8-16 hours at 37°C. Tube formation was quantitated and captured by photography. **Figure 23** shows that the 58P1D12 ECD induced the formation of endothelial tubes similarly as generated by treatment with purified VEGF protein. **Figure 24** shows that similar results were obtained when treating mature lung-derived microvascular endothelial cells (HMVEC) with 58P1D12 ECD. Together, these results indicate that the 58P1D12 protein is involved in promoting angiogenesis that facilitates tumor growth, activation of endothelium for tumor vascularization or tumor cell invasion. **Figure 25** shows inhibition of endothelial tubes induced by 58P1D12 ECD with monoclonal antibody M8-143(5)10 at 30 µg/ml. An isotype control monoclonal antibody at the same concentration did not inhibit tube formation.

### II. Enhanced survival and proliferation of endothelial cells by recombinant extracellular domain (ECD) of 58P1D12.

Enhanced proliferation, entry into S-phase and survival of endothelial cells is a hallmark of both angiogenesis and the cancer cell phenotype. To address the effect of the 58P1D12 protein on the survival and proliferation of normal endothelial cells, both HUVEC and HMVEC were treated with the 58P1D12 ECD and assayed in low serum conditions. The cells were grown overnight in 0.5% FBS and then compared to cells treated with VEGF or 58P1D12 ECD. The cells were evaluated for survival by the Alamar blue assay. The results in **Figure 26** indicate that HUVEC cell viability was increased by the endothelial growth factor VEGF (positive control) as well as increasing concentrations of recombinant 58P1D12 ECD. Further, the results in **Figure 27** show that proliferation of HMVEC in response to 58P1D12 ECD was enhanced at 72 hr post-treatment measured by a ³H-thymidine incorporation assay. These results confirm those measured by Alamar blue assay, and indicate that cells that are treated with 58P1D12 protein have an enhanced proliferative capacity and survive in low serum conditions. Accordingly, 58P1D12 expressing cells induce increased potential for growth of endothelium in vivo.

Together, these data indicate that the 58P1D12 protein has growth promoting, survival potentiating and angiogenic activities for primary endothelium. Such activities are vital for the establishment and maintenance of tumors by stimulating capillary bed formation by endothelial cells. This increased angiogenesis provides a means of increased nutrition and oxygenation of growing tumors, and affords a therapeutic intervention strategy for targeting of 58P1D12 in malignancies. The 58P1D12 protein also plays a role in cell migration, particularly for cells that express the protein, and also serves as an attractant for endothelial cells and other cells expressing its ligand. Cell migration is important for the metastatic potential of tumor cells, and facilitates their homing to remote tissues. Expression of 58P1D12 protein in bone metastases indicates that the migratory phenotype of 58P1D12-expressing cells imparts a strong potential for such cells to metastasize.

### Example 45

### 58P1D12 RNA Interference (RNAi)

RNA interference (RNAi) technology is implemented to a variety of cell assays relevant to oncology. RNAi is a post-transcriptional gene silencing mechanism activated by double-stranded RNA (dsRNA). RNAi induces specific mRNA degradation leading to changes in protein expression and subsequently in gene function. In mammalian cells, these dsRNAs called short interfering RNA (siRNA) have the correct composition to activate the RNAi pathway targeting for degradation, specifically some mRNAs. *See*, Elbashir S.M., et. al., Duplexes of 21-nucleotide RNAs Mediate RNA interference in Cultured Mammalian Cells, Nature 411(6836):494-8 (2001). Thus, RNAi technology is used successfully in mammalian cells to silence targeted genes.

Loss of cell proliferation control is a hallmark of cancerous cells; thus, assessing the role of 58P1D12 in cell survival/proliferation assays is relevant. Accordingly, RNAi was used to investigate the function of the 58P1D12 antigen. To generate siRNA for 58P1D12, algorithms were used that predict oligonucleotides that exhibit the critical molecular parameters (G:C content, melting temperature, etc.) and have the ability to significantly reduce the expression levels of the 58P1D12 protein when introduced into cells. In accordance with this Example, 58P1D12 siRNA compositions are used that comprise siRNA (double stranded, short interfering RNA) that correspond to the nucleic acid ORF sequence of the 58P1D12 protein or subsequences thereof. Thus, siRNA subsequences are used in this manner are generally 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or more than 35 contiguous RNA nucleotides in length. These siRNA sequences are complementary and non-complementary to at least a portion of the mRNA coding sequence. In a preferred embodiment, the subsequences are 19-25 nucleotides in length, most preferably 21-23 nucleotides in length. In preferred embodiments, these siRNA achieve knockdown of 58P1D12 antigen in cells expressing the protein and have functional effects as described below.

**Mammalian siRNA transfections**: The day before siRNA transfection, the different cell lines are plated in media (RPMI 1640 with 10% FBS w/o antibiotics) at 2x10³ cells/well in 80 µ (96 well plate format) for the proliferation assay. In parallel with the 58P1D12 specific siRNA oligo, the following sequences are included in every experiment as controls: a) Mock transfected cells with Lipofectamine 2000 (Invitrogen, Carlsbad, CA) and annealing buffer (no siRNA); b) Luciferase-4 specific siRNA (targeted sequence: 5'-AAGGGACGAAGACGAACACUUCTT-3') (SEQ ID NO: 35); and, c) Eg5 specific siRNA (targeted sequence: 5'-AACTGAAGACCTGAAGACAATAA-3') (SEQ ID NO: 36). SiRNAs are used at 10nM and µg/ml Lipofectamine 2000 final concentration.

The procedure is as follows: The siRNAs are first diluted in OPTIMEM (serum-free transfection media, Invitrogen) at 0.1 µM (10-fold concentrated) and incubated 5-10 min RT. Lipofectamine 2000 was diluted at 10 µg/ml (10-fold concentrated) for the total number transfections and incubated 5-10 minutes at room temperature (RT). Appropriate amounts of diluted 10-fold concentrated Lipofectamine 2000 were mixed 1:1 with diluted 10-fold concentrated siRNA and Incubated at RT for 20-30" (5-fold concentrated transfection solution). 20 µls of the 5-fold concentrated transfection solutions were added to the respective samples and incubated at 37°C for 96 hours before analysis.

**³****H-Thyrmidine Incorporation assay:** The proliferation assay is a ³H-thymidine incorporation method for determining the proliferation of viable cells by uptake and incorporation of label into DNA.

The procedure was as follows: Cells growing in log phase are trypsinized, washed, counted and plated in 96-well plates at 1000-4000 cells/well in 10% FBS. After 4-8 hrs, the media is replaced. The cells are incubated for 24-72 hrs, pulsed with ³H-Thy at 1.5 µCi/ml for 14 hrs, harvested onto a filtermat and counted in scintillation cocktail on a Microbeta trilux or other counter.

These data indicate that 58P1D12 plays an important role in the proliferation of cancer cells and that the lack of 58P1D12 clearly decreases the survival potential of these cells. It is to be noted that 58P1D12 is constitutively expressed in many tumor cell lines. 58P1D12 serves a role in malignancy; its expression is a primary indicator of disease, where such disease is often characterized by high rates of uncontrolled cell proliferation and diminished apoptosis. Correlating cellular phenotype with gene knockdown following RNAi treatments is important, and allows one to draw valid conclusions and rule out toxicity or other non-specific effects of these reagents. To this end, assays to measure the levels of expression of both protein and mRNA for the target after RNAi treatments are important, including Western blotting, FACS staining with antibody, immunoprecipitation, Northern blotting or RT-PCR (Taqman or standard methods). Any phenotypic effect of the siRNAs in these assays should be correlated with the protein and/or mRNA knockdown levels in the same cell lines. 58P1D12 protein is reduced after treatment with siRNA oligos described above (e.g., 58P1D12.a, etc.).

A method to analyze 58P1D12 related cell proliferation is the measurement of DNA synthesis as a marker for proliferation. Labeled DNA precursors (i.e. ³H-Thymidine) are used and their incorporation to DNA is quantified. Incorporation of the labeled precursor into DNA is directly proportional to the amount of cell division occurring in the culture. Another method used to measure cell proliferation is performing clonogenic assays. In these assays, a defined number of cells are plated onto the appropriate matrix and the number of colonies formed after a period of growth following siRNA treatment is counted.

In 58P1D12 cancer target validation, complementing the cell survival/proliferation analysis with apoptosis and cell cycle profiling studies are considered. The biochemical hallmark of the apoptotic process is genomic DNA fragmentation, an irreversible event that commits the cell to die. A method to observe fragmented DNA in cells is the immunological detection of histone-complexed DNA fragments by an immunoassay (i.e. cell death detection ELISA) which measures the enrichment of histone-complexed DNA fragments (mono- and oligo-nucleosomes) in the cytoplasm of apoptotic cells. This assay does not require pre-labeling of the cells and can detect DNA degradation in cells that do not proliferate in vitro (i.e. freshly isolated tumor cells).

The most important effector molecules for triggering apoptotic cell death are caspases. Caspases are proteases that when activated cleave numerous substrates at the carboxy-terminal site of an aspartate residue mediating very early stages of apoptosis upon activation. All caspases are synthesized as pro-enzymes and activation involves cleavage at aspartate residues. In particular, caspase 3 seems to play a central role in the initiation of cellular events of apoptosis. Assays for determination of caspase 3 activation detect early events of apoptosis. Following RNAi treatments, Western blot detection of active caspase 3 presence or proteolytic cleavage of products (i.e. PARP) found in apoptotic cells further support an active induction of apoptosis. Because the cellular mechanisms that result in apoptosis are complex, each has its advantages and limitations. Consideration of other criteria/endpoints such as cellular morphology, chromatin condensation, membrane blebbing, apoptotic bodies help to further support cell death as apoptotic. Since not all the gene targets that regulate cell growth are anti-apoptotic, the DNA content of permeabilized cells is measured to obtain the profile of DNA content or cell cycle profile. Nuclei of apoptotic cells contain less DNA due to the leaking out to the cytoplasm (sub-G1 population). In addition, the use of DNA stains (i.e., propidium iodide) also differentiate between the different phases of the cell cycle in the cell population due to the presence of different quantities of DNA in G0/G1, S and G2/M. In these studies the subpopulations can be quantified.

For the 58P1D12 gene, RNAi studies facilitate the understanding of the contribution of the gene product in cancer pathways. Such active RNAi molecules have use in identifying assays to screen for mAbs that are active anti-tumor therapeutics. Further, siRNA are administered as therapeutics to cancer patients for reducing the malignant growth of several cancer types, including those listed in Table I. When 58P1D12 plays a role in cell survival, cell proliferation, tumorigenesis, or apoptosis, it is used as a target for diagnostic, prognostic, preventative and/or therapeutic purposes.

### Example 46

### Homology Comparison of 58P1D12 to known Sequences

The 58P1D12 protein has 273 amino acids with a calculated molecular weight of 30.47 kDa and a pl of 6.38. 58P1D12 is predicted to be a plasma membrane protein (PSORT http://psort.nibb.ac.jp/form.html). 58P1D12 contains a single transmembrane region from amino acids 218-240 with high probability that the amino-terminus resides outside, consistent with the topology of a Type 1 transmembrane protein (http://www.cbs.dtu.dk/services/TMHMM). Also visualized is a short hydrophobic stretch from amino acids 1-21, consistent with the existence of an amino-terminal signal peptide (http://www.cbs.dtu.dk/services/SignalP). Based on the TMpred algorithm of Hofmann and Stoffel which utilizes TMBASE (K. Hofmann, W. Stoffel, TMBASE - A database of membrane spanning protein segments Biol. Chem. Hoppe-Seyler 374:166, 1993), 58P1D12 contains a primary transmembrane region from amino acids 220-243 and a secondary transmembrane region from amino acids 1-21 (contiguous amino acids with values greater than 0 on the plot have high probability of being transmembrane regions) with an orientation in which the amino terminus resides inside and the carboxyl terminus outside. An alternative model is also predicted that 58P1D12 is a Type 1 transmembrane protein in which the amino-terminus resides outside and the protein contains a secondary transmembrane domain signal peptide from amino adds 1-21 and a primary transmembrane domain from aa214-aa246. The transmembrane prediction algorithms are accessed through the ExPasy molecular biology server (http://www.expasy.ch/tools/).

The C-type lectins are a family of both transmembrane and secreted proteins belonging to the lectin superfamily (8 different types of lectins) of carbohydrate recognition/adhesion molecules. They have related carbohydrate recognition domains (CRDs) in their extracellular domains, but for the transmembrane anchored proteins, they contain unique cytoplasmic domains. There are 14 identified families of C-type lectins, and they are all believed to have carbohydrate recognition properties, and have roles in various cell functions including cell adhesion, glycoprotein clearance and innate immunity. The adhesion function of C-type lectins is well-established for the selectin family. Additional functions of C-type lectins include signal transduction, and may provide a platform for protein associations. Some C-type lectins have been shown to facilitate glycoprotein clearance, elimination of foreign pathogens during innate immune responses and migration of cells. In mammals, C-type lectins are widely expressed, yet play roles in other species.

58P1D12 belongs to the type VIII family of Type lectins, which includes one other member, layilin. Layilin has ∼45% homology with 58P1D12, and interacts with the carbohydrate ligand hyaluronate. Hyaluronate, an extracellular matrix component consisting of repeating disaccharide units, is a ligand for several C-type lectins and is a putative ligand for 58P1D12, but was not detected in a binding reaction with 58P1D12 using the cetylpyridinium precipitation technique (Weng, L. et al, 2002, Genomics 80:62-70). Layilin localizes in membrane ruffles during cell migration, and interacts with the cytoskeletal protein talin (Borowsky, M.L and Hynes, R. O., 1998, J. Cell Biol. 143:429-442). 58P1D12 also is observed in membrane ruffles and associates with talin through its cytoplasmic domain.

58P1D12 also interacts with other cytoplasmic signaling molecules, including serine/threonine and dual kinases that phosphorylate its intracellular serine and threonine residues. Such phosphorylation leads to the interaction of 58P1012 with signaling molecules that are regulated by their interaction with the plasma membrane through association with 58P1D12. Such molecules induce signals that are critical to the function of 58P1D12 protein, which mediate the growth pathways that are stimulated in tumor cells expressing 58P1D12. Further, the adhesion properties of the 58P1D12 protein expressed on tumor cells enhances their ability to migrate during metastases, facilitates their homing to distal sites (lymph nodes), and promotes interactions with, and-activation of, other cells such endothelia during the formation of tumor masses. The adhesive and growth signals of 58P1D12 protein significantly promote unregulated growth of cancer cells, contributing to their growth advantage in vivo.

Similar to other C-type lectins, 58P1D12 is membrane associated. Functional analysis shows that a recombinant soluble form of 58P1D12 has both angiogenic and proliferative enhancing properties for endothelial cells (see Example 44). The membrane-assodated form of the 58P1 D12 protein or a secreted portion promotes establishment and maintenance of the malignant state of tumors expressing the 58P1D12 antigen. The formation of capillary beds surrounding tumors through the angiogenic function of 58P1D12 protein as well as the proliferative signals that are imparted by the protein promote survival of tumors which require nutrition and oxygenation.

## Claims

1. A method for detecting expression levels of a 58P1D12 protein consisting of the sequence of SEQ ID NO:13 or SEQ ID NO:14 or of a 58P1D12 transcription variant polynucleotide encoding a protein consisting of the sequence of SEQ ID NO:13 or SEQ ID NO:14 in a biological sample and a corresponding normal sample, comprising:
determining expression levels of the 58P1D12 protein or the 58P1D12 transcription variant polynucleotide in the biological sample and the corresponding normal sample; and
comparing the expression levels of the 58P1D12 protein or the 58P1D12 transcription variant polynucleotide detected in the biological sample and the corresponding normal sample,
wherein the biological sample is from a patient who has or is suspected of having a cancer selected from the group consisting of prostate cancer, bladder cancer, lung cancer, ovarian cancer, bone cancer, and cervical cancer.

2. The method of claim 1, wherein the 58P1D12 transcription variant polynucleotide has the sequence of SEQ ID NO: 4 or SEQ ID NO: 8.

3. The method of claim 1 or 2, wherein the biological sample is from serum, blood or urine, or tissue selected from the group consisting of prostate, bladder, lung, ovarian, bone and cervix.

4. The method of any one of claims 1 to 3, wherein the presence of elevated 58P1D12 protein or transcription variant polynucleotide in the biological sample relative to the corresponding normal sample provides an indication of the presence of cancer.

## Patentansprüche

1. Verfahren zum Nachweis von Expressionsgraden eines 58P1D12-Proteins, das aus einer Sequenz gemäß SEQ ID Nr.: 13 oder SEQ ID Nr.: 14 oder aus einem 58P1D12-Transkriptionsvarianten-Polynucleotid besteht, das für ein aus der Sequenz gemäß SEQ ID Nr.: 13 oder SEQ ID Nr.: 14 bestehendes Protein codiert, in einer biologischen Probe und einer entsprechenden normalen Probe, wobei das Verfahren aufweist:
Bestimmung von Expressionsgraden des 58P1D12-Proteins oder des 58P1D12-Transkriptionsvarianten-Polynucleotids in der biologischen Probe und der entsprechenden normalen Probe; und
Vergleich der in der biologischen Probe und der entsprechenden normalen Probe nachgewiesenen Expressionsgrade des 58P1D12-Proteins oder des 58P1D12-Transkriptionsvarianten-Polynucleotids,
wobei die biologische Probe von einem Patienten herrührt, der an einem Krebs leidet oder bei dem Verdacht auf einen Krebs besteht, der aus der Gruppe ausgewählt ist, die aus Prostatakrebs, Blasenkrebs, Lungenkrebs, Eierstockkrebs, Knochenkrebs und Gebärmutterhalskrebs besteht.

2. Verfahren nach Anspruch 1, wobei das 58P1D12-Transkriptionsvarianten-Polynucleotid die Sequenz gemäß SEQ ID Nr.: 4 oder SEQ ID Nr.: 8 aufweist.

3. Verfahren nach Anspruch 1 oder 2, wobei die biologische Probe aus Serum, Blut oder Urin oder Gewebe entnommen wird, das aus der Gruppe ausgewählt ist, die aus Prostata, Blase, Lunge, Eierstock, Knochen und Gebärmutterhals besteht.

4. Verfahren nach einem der Ansprüche 1 bis 3, wobei die Anwesenheit einer erhöhten Konzentration von 58P1D12-Protein oder -Transkriptionsvarianten-Polynucleotid in der biologischen Probe im Vergleich zur entsprechenden normalen Probe einen Hinweis auf die Anwesenheit von Krebs liefert.

## Revendications

1. Procédé de détection des niveaux d'expression d'une protéine 58P1D12 consistant en la séquence de la SEQ ID NO:13 ou de la SEQ ID NO:14 ou d'un polynucléotide 58P1D12 variant de transcription codant une protéine consistant en la séquence de la SEQ ID NO:13 ou de la SEQ ID NO:14 dans un échantillon biologique et dans un échantillon normal correspondant, comprenant:
déterminer les niveaux d'expression de la protéine 58P1D12 ou du polynucléotide 58P1D12 variant de transcription dans l'échantillon biologique et dans l'échantillon normal correspondant; et
comparer les niveaux d'expression de la protéine 58PID12 ou du polynucléotide 58P1D12 variant de transcription dans l'échantillon biologique et dans l'échantillon normal correspondant,
dans lequel l'échantillon biologique vient d'un patient qui souffre ou qui est soupçonné souffrir d'un cancer sélectionné parmi le groupe consistant en cancer de la prostate, cancer de la vessie, cancer du poumon, cancer des ovaires, cancer des os et cancer du col de l'utérus.

2. Procédé selon la revendication 1, dans lequel le polynucléotide 58P1D12 variant de transcription a la séquence de la SEQ ID NO:4 ou de la SEQ ID NO: 8.

3. Procédé selon la revendication 1 ou 2, dans lequel l'échantillon biologique vient du sérum, du sang ou de l'urine ou d'un tissu sélectionné parmi le groupe consistant en prostate, vessie, poumon, ovaire, os et col de l'utérus.

4. Procédé selon l'une quelconque des revendications 1 à 3, dans lequel la présence de protéine ou de polynucléotide 58P1D12 variant de transcription élevé dans l'échantillon biologique par rapport à l'échantillon normal correspondant, fournit une indication de la présence de cancer.
